# EUROPEAN PATENT SPECIFICATION

(11) **EP 3 145 424 B1**
(45) Date of publication and mention of the grant of the patent: **27.06.2018**
(21) Application number: 15796711.8
(22) Date of filing: 18.05.2015
(51) Int. Cl.: A61B 17/221, A61B 90/00

(54) **CLOT RETRIEVAL SYSTEM**
GERINNSELBESEITIGUNGSSYSTEM
SYSTÈME D'EXTRACTION DE CAILLOT

(30) Priority: 18.05.2014 US 201461994919 P; 18.05.2014 US 201461994934 P; 02.12.2014 US 201414558712; 02.12.2014 US 201414558705; 05.01.2015 WO PCT/US2015/010178
(43) Date of publication of application: 29.03.2017
(60) Divisional application: 18172665.4
(73) Proprietor: Legacy Ventures LLC, Nashville, Tennessee 37212 (US)
(72) Inventor: ULM III, Arthur John, Nashville, Tennessee 37215 (US)
(74) Representative: Dehns
(86) International application number: PCT/US2015/031447
(87) International publication number: WO 2015/179324

(56) References cited:
- WO-A1-2012/110619
- WO-A1-2014/169266
- US-A1- 2004 068 314
- US-A1- 2006 015 136
- US-A1- 2010 049 240
- US-A1- 2010 268 265
- US-A1- 2012 116 440
- US-A1- 2013 345 739
- US-A1- 2013 345 739
- US-A1- 2014 371 779

## Description

### BACKGROUND

### TECHNICAL FIELD

The present invention relates to a deployable system for removing a blood clot or other object from a lumen of an animal.

### BACKGROUND OF THE INVENTION

Acute ischemic strokes develop when a blood clot (thrombus) blocks an artery supplying blood to the brain. Needless to say, when a blood clot creates such a blockage, time in removing the clot is critical.

The removal of intracranial obstructions is limited by several factors, such as the distance of the intracranial obstruction from the femoral access site, the tortuosity (twists and turns in the artery as it enters the base of the skull) of the cervical and proximal intracranial vasculature, the small size of the vessels and the extremely thin walls of intracranial vessels, which lack a significant muscular layer. These limitations require a device to be small and flexible enough to navigate through tortuous vessels within a guide catheter and microcatheter, expand after delivery at the site of occlusion and be retrievable into the microcatheter and yet be strong enough to dislodge strongly adherent thrombus from the vessel wall. In addition, the device should distally entrap or encase the thrombus to prevent embolization to other vessels and to completely remove the occlusion. The device should be retrievable without the need for proximal occlusion of the vessel, which carries risk of further ischemia and risk of vessel injury. The device should be simple to use and be capable of multi-use within the same patient treatment. The device should not be abrasive and should not have sharp corners exposed to the endothelial layer of the vessel wall.

Currently available intravascular thrombus and foreign body removal devices lack several of these features. Currently available devices include the MERCI™ RETRIEVER clot retriever device marketed by Concentric Medical, Inc. (Mountainview, CA), the PENUMBRA™ system marketed by Penumbra Inc. (Alameda, CA) to retrieve clots, and the newer stent retrieval devices TREVO™ (Stryker, Kalamazoo, MI) and SOLITAIRE™ (eV3 Endovascular Inc., Plymouth, MA, which is a subsidiary of Covidien). All the devices are ineffectual at removing organized hard thrombus that embolize to the brain from the heart and from atherosclerotic proximal vessels. These "hard" thrombi constitute the majority of strokes which are refractory to medical treatment and are therefore referred for removal by mechanical means through an endovascular approach. The MERCI retrieval system is comprised of coiled spring-like metal and associated suture material. The method of use is deployment distal to the thrombus and by withdrawing the device through the thrombus, the thrombus becomes entangled in the coil and mesh and then is retrieved. The MERCI system requires occlusion of the proximal vessel with a balloon catheter and simultaneous aspiration of blood while the thrombus is being removed. Most of the time, the device fails to dislodge the thrombus from the wall of the vessel and often, even when successfully dislodging the thrombus, the thrombus embolizes into another or the same vessel due to the open ended nature of the device.

The next attempt at a thrombus removal system was the PENUMBRA. The PENUMBRA is a suction catheter with a separator that macerates the thrombus which is then removed by suction. The device is ineffective at removing hard, organized thrombus which has embolized from the heart, cholesterol plaque from proximal feeding arteries and other foreign bodies.

The SOLITAIRE and TREVO systems are self-expanding non-detachable stents. The devices are delivered across the thrombus which is then supposed to become entwined in the mesh of the stent and which is then removed in a manner similar to the MERCI system. Again, these devices are ineffectual at treating hard thrombus. In fact, the thrombus is often compressed against the vessel wall by the stent which temporarily opens the vessel by outwardly pressing the clot against the vessel wall. Upon retrieval of the devices, the clot remains or is broken up into several pieces which embolize to vessels further along the vessel.

US 2004/068314 A1 discloses a system for removing objects from an interior of lumen of an animal as recited in the preamble to claim 1. US 2010/268265 A1 discloses an apparatus from retrieving material within a body lumen of a patient.

Thus, there is a need for new, easy-to-use, easy-to-manufacture, safe surgical devices for removing obstructions, such as blood clots, from internal lumens of humans and other animals in a timely manner.

### BRIEF SUMMARY

The invention is defined by claim 1 while preferred embodiments are defined by the dependent claims. The present disclosure provides several systems for removing obstructions and other objects within a blood vessel or other lumen of an animal. The system may be deployed in the lumen from a distal end of a catheter and, in some embodiments, includes a pull wire having a proximal end and a distal end; a distal body attached to the pull wire, the distal body comprising an interior, an exterior, a proximal end, a distal end, a plurality of proximal memory metal strips located at the proximal end, a proximal hub located in the distal body interior, and a distal hub located distal relative to the proximal hub. The distal body has a relaxed state wherein the distal body has a first height and width and a collapsed state wherein the distal body has a second height and width, the second height less than said first height, the second width less than the first width. The system further includes a catheter having an interior, a proximal end leading to the interior and a distal end leading to the interior, the catheter comprised of a biocompatible material and configured to envelope the distal body when the distal body is in the collapsed state. Each of the proximal memory metal strips has a proximal end and a distal end and preferably, in the relaxed state, each of the proximal ends of the proximal memory metal strips is located proximal relative to the proximal hub. Preferably, in the relaxed state, the proximal ends of the proximal memory metal strips are configured to move towards each other and towards the pull wire when an operator moves the proximal hub distally and closer to the stationary distal hub (i.e., when the operator decreases the distance between the hubs). Preferably, in the relaxed state, the proximal ends of the proximal memory metal strips are configured to move away from each other and away from the pull wire by moving the proximal hub proximally away from the stationary distal hub (i.e., when the operator increases the distance between the hubs).

Optionally, the system further includes a plurality of memory metal connector strips, the plurality of memory metal connector strips each having a proximal end attached to a proximal memory metal strip and a distal end attached to the proximal hub. Optionally, the connector strips are integral with the proximal hub (i.e., optionally, the connector strips and the proximal hub are formed from the same piece of memory metal). Optionally, the proximal hub is a tube having an aperture and the pull wire passes through the aperture. Optionally, in the relaxed state, the proximal hub is slideable along the pull wire (i.e., at least a segment of the pull wire). Optionally, in the relaxed state, the proximal memory metal strips are distributed substantially evenly about a perimeter of the distal body. Optionally, the distal hub is a tube having an aperture. Optionally, the distal hub is attached to the pull wire such that the distal hub is not slideable along the pull wire. Optionally, the distal body further comprises a lead wire extending distally from the distal hub. Optionally, the distal body comprises a basket comprised of a plurality of memory metal strips distal relative to the proximal memory metal strips. Optionally, the distal hub, the proximal hub, and the distal basket are comprised of a nitinol having the same material composition. Optionally, the distal body further comprises an x-ray marker. Optionally, the proximal memory metal strips form a claw, the claw having a closeable proximal end formed by the proximal ends of the proximal memory metal strips. Optionally, between 2 and 4 proximal memory metal strips form the claw. Optionally, the distal body, in the relaxed state, has a tapered shape in which the distal body height and width decrease from the proximal end to the distal end. Optionally, the distal body, in the relaxed state, has a bullet shape. Optionally, the proximal hub and the distal hub are generally cylindrical in shape and each has an outer diameter and an inner diameter that forms the apertures of the proximal and distal hubs, the outer diameters of the proximal and distal hubs are substantially the same size, and the inner diameters of the proximal and distal hubs are substantially the same size. Optionally, the outer diameters of the proximal and distal hubs are from about 0.28 mm (0.011 inches) to about 1.37 mm (0.054 inches), and the inner diameters of the proximal and distal hubs are from about 0.20 mm (0.008 inches) to about 1.30 mm (0.051 inches). Optionally, the pull wire is generally cylindrical and the diameter of the pull wire is between about 0.20 mm (0.008 inches) and about 1.30 mm (0.051 inches). Optionally, the proximal memory metal strips have a length of between about 10 and about 60 millimeters. Optionally, the first height and first width of the distal body are between about 2 millimeters (mm) and about 6 millimeters. Optionally, the proximal memory metal strips are configured to a separate a clot from a blood vessel wall.

The system may be used in an exemplary method to remove an object from an interior lumen of an animal, the lumen having an interior wall forming the lumen. The exemplary method may include:
a) providing a system comprising: i) a pull wire having a proximal end and a distal end; ii) a distal body attached to the pull wire, the distal body comprising a proximal end, a distal end, and a claw, the claw comprised of a plurality of memory metal strips, the distal body having a relaxed state wherein the distal body has a first height and width and a collapsed state wherein the distal body has a second height and width, the second height less than said first height, the second width less than said first width; and iii) a catheter having an interior, a proximal end leading to the interior and a distal end leading to the interior, the catheter comprised of a biocompatible material and configured to envelope the distal body when said distal body is in said collapsed state;
b) positioning the system in the lumen;
c) deploying the distal body from the distal end of the catheter;
d) allowing the height and width of said distal body to increase; and
e) moving the memory metal strips towards each other and the pull wire so as to capture the obstruction. Optionally, the claw and the memory metal strips are located at the proximal end of said distal body and the distal body is deployed distal to said object. Optionally, the proximal memory metal strips have a proximal end forming the proximal end of the claw and a distal end, and the exemplary method includes moving the proximal ends of the memory metal strips towards each other and the pull wire so as to capture the obstruction. Optionally, the distal body further comprises a proximal hub located in the distal body interior, and a distal hub located distal relative to the proximal hub, each of the memory metal strips has a proximal end and a distal end, each of the proximal ends of the memory metal strips is located proximal relative to the proximal hub, and the proximal ends of the memory metal strips are configured to move towards each other and towards the pull wire by moving the proximal hub distally and closer to the distal hub, and the proximal ends of the memory metal strips are configured to move away from each other and away from the pull wire by moving the proximal hub proximally and away from the distal hub, and the exemplary method further comprises moving the proximal hub distally and closer to the distal hub so as to capture the obstruction in the claw. Optionally, the interior lumen is an intracranial artery and the obstruction is a blood clot. Optionally, the exemplary method further comprises using the clot to move the proximal hub toward the distal hub and exert tension on the proximal memory metal strips. Optionally, the exemplary method further comprises using a tube to move the proximal hub toward the distal hub and exert tension on the proximal memory metal strips.

The present disclosure also describes an exemplary method of manufacturing a system for removing objects within an interior lumen of an animal. In some examples, the exemplary method includes: a) providing a single tube comprised of a memory metal, the single tube having an exterior, a hollow interior, a wall separating the exterior from the hollow interior, a proximal portion comprising an aperture leading to the hollow interior, a distal portion comprising an aperture leading to the hollow interior, and a middle portion between the proximal portion and the distal portion;
b) cutting the wall of the middle portion with a laser;
c) removing the pieces of the middle portion cut by the laser to form a proximal tube, a middle portion comprising a plurality of memory metal strips attached to the proximal tube and a distal tube;
d) altering the shape of the middle portion;
e) allowing the middle portion to expand relative to the distal tube and the proximal tube;
f) cutting the memory metal strips to form a first segment comprising the proximal tube and a proximal segment of the memory metal strips, and a second segment comprising the distal tube and a distal segment of the memory metal strips; and
g) joining the proximal segments to the distal segments such that the distal segments form the proximal end of a distal body, such that the proximal tube is located inside an interior of said distal body, and such that the proximal tube is located distal relative to the proximal end.

Optionally, the exemplary method further includes placing a pull wire through the proximal tube such that the proximal tube is slideable along at least a segment of the pull wire. Optionally, the exemplary method further includes attaching the pull wire to the distal tube. Optionally, the step of joining the proximal segments to the distal segments comprises welding the proximal segments to the distal segments. Optionally, after the step of joining the proximal segments to the distal segments, the proximal end forms a claw comprised of between 2 and 4 memory metal strips, the claw memory metal strips configured to move towards each by moving said proximal tube distally and closer to the distal tube, and the claw memory metal strips configured to move away from each other by moving the proximal tube proximally and away from said distal tube. Optionally, the exemplary method further includes not altering the shape of the proximal and distal portions while altering the shape of the middle portion. Optionally, the exemplary method further includes cooling the proximal portion, the middle portion, and the distal portion after step D) and, after cooling, the proximal and distal portions have substantially the same size as the proximal and distal portions had prior to step A). Optionally, the exemplary method of allowing said middle portion to expand comprises heating the middle portion. Optionally, the exemplary method of altering the shape of the middle portion comprises using a mandrel. Optionally, the mandrel is tapered. Optionally, the proximal portion and the distal portion are not cut by the laser. Optionally, prior to cutting the memory metal tube, the exemplary memory metal tube has an outer diameter that is from about 0.28 mm (0.011 inches) to about 1.37 mm) 0.054 inches and an inner diameter that is from about 0.20 mm (0.008 inches) to about 1.30 mm (0.051 inches).

The invention provides a system for removing objects from an interior lumen of an animal that includes:
a pull wire having a proximal end and a distal end;
a distal body attached to the pull wire, the distal body comprising an interior, a proximal end, a distal end, a distal body length extending from the proximal end to the distal end, a proximal hub (preferably in the form of a tube) forming the proximal end of the distal body, a basket comprised of a plurality of cells formed by a plurality of basket strips, a plurality of proximal strips, and, optionally a distal hub (preferably in the form of a tube) forming a distal end of the basket, the basket comprising a basket interior, each proximal strip having a proximal end attached to the proximal hub, and a distal end attached to a cell, the distal body having a relaxed state wherein the distal body has a first height and a first width, and a collapsed state wherein the distal body has a second height and a second width, the second height less than the first height, the second width less than the first width; and
a catheter having an interior, a proximal end leading to the interior and a distal end leading to the interior, the catheter comprised of a biocompatible material and configured to envelope the distal body when the distal body is in the collapsed state,
wherein, in the relaxed state, the basket comprises a first pair of distal crowns not attached to another cell of the basket and pointing generally in the distal direction, the first pair of distal crowns located approximately the same distance from the proximal hub and between about 150 degrees and about 180 degrees relative to each other, and further wherein the basket further comprises a second pair of distal crowns not attached to another cell of the basket and pointing generally in the distal direction, the second pair of distal crowns located distally relative to, and approximately 90 degrees relative to, the first pair of distal crowns (e.g., each distal crown of the second pair of distal crowns is located approximately 60 degrees to 90 degrees relative to a distal crown of the first pair of distal crowns), the distal crowns in the second pair of distal crowns located approximately the same distance from the proximal hub and further wherein each of the distal crowns in the first and second pair of distal crowns comprises an x-ray marker, the x-ray maker more visible under x-ray as compared to the basket strips when the distal body is located in a cranial blood vessel inside the body of a human and the x-ray is taken from outside the human's body. When it is said that the first pair of distal crowns are located approximately the same distance from the proximal hub, it will be understood that if one of the first pair of distal crowns is located X distance from the proximal hub, the other of the first pair of distal crowns is located X distance plus or minus (+/-) 3 mm from the proximal hub, more preferably X distance plus or minus (+/-) 0.5 mm from the proximal hub. Similarly, when it is said that the second pair of distal crowns are located approximately the same distance from the proximal hub, it will be understood that if one of the second pair of distal crowns is located Y distance from the proximal hub, the other of the first pair of distal crowns is located Y distance plus or minus (+/-) 3 mm from the proximal hub, more preferably Y distance plus or minus (+/-) 0.5 mm from the proximal hub. Optionally, instead of a distal hub, the basket includes an open distal end.

Optionally, the x-ray markers are comprised of a material different than the material forming the basket strips. Optionally, in the relaxed state, the basket interior is substantially hollow. Optionally, in the relaxed state, the distal body does not have another x-ray marker that is located approximately the same distance from the proximal hub as the first pair of x-ray markers and the distal body does not have another x-ray marker that is located approximately the same distance from the proximal hub as the second pair of x-ray markers. In other words, the first and second pair of x-ray markers are the only markers their respective distances from the proximal hub. Optionally, each distal crown in the first and second pair of distal crowns forms part of an enlarged cell and further wherein the surface area of each enlarged cell in the relaxed state is greater than the surface area of each of the other individual cells of the basket and further wherein the enlarged cells are configured to allow a thrombus to pass therethrough and into the basket interior. Optionally, in the relaxed state, the distal body does not have another free distal-pointing crown that is located approximately the same distance from the proximal hub as the first pair of distal crowns and the distal body does not have another free distal-pointing crown that is located approximately the same distance from the proximal hub as the second pair of distal crowns. Optionally, the basket strips are comprised of a memory metal. Optionally, each of the distal crowns in the first pair and second pair of distal crowns curve radially inward toward the basket interior in the relaxed state, wherein the distal crowns of the first pair of distal crowns are configured to contact each other when an exterior, external compressive force (such as a thrombus) is exerted on a distal crown of the first pair of distal crowns when the distal body is in the relaxed state, and further wherein the distal crowns of the second pair of distal crowns are configured to contact each other when an exterior, external compressive force (such as a thrombus) is exerted on a distal crown of the second pair of distal crowns when the distal body is in the relaxed state. Optionally, the proximal hub is located approximately in the center of the first height and first width in the relaxed state. For example, preferably the proximal hub is located within 0.5 mm of the center of first width and the first height. Optionally, the catheter is comprised of a polymeric material (i.e., one or more polymeric materials such as silicone, PVC, latex rubber or braided nylon). Optionally, the pull wire is comprised of a biocompatible metallic material (e.g., a biocompatible metal or a biocompatible metal alloy). Optionally, the proximal end of a first proximal strip is located at least about 65 degrees (e.g., between about 65 and about 180 degrees) relative to the distal end of the first proximal strip, wherein the proximal end of a second proximal strip is located at least about 65 degrees (e.g., between about 65 and about 180 degrees) relative to the distal end of the second proximal strip, and further wherein the first and second proximal strips intersect adjacent and distal to the proximal hub (e.g., within about 0 and about 4 mm of the proximal hub). Optionally, each distal crown forms part of a cell that further comprises a proximal crown pointing generally in the proximal direction and connected to a memory metal strip (e.g., a proximal strip comprised of a memory metal or a basket strip comprised of a memory metal). In other words, the proximal crowns are not free. Optionally, the basket, the proximal hub and the proximal strips are comprised of a memory metal, wherein the proximal hub comprises a proximal end and a distal end, and further wherein the proximal strips are integral with the distal end of the proximal hub. Optionally, the length of the distal body from the proximal hub to the distal hub (not including any lead wire) is from about 20 mm to about 65 mm. Optionally, the system is used in an exemplary method of removing a blood clot from a blood vessel of an animal the exemplary method comprising the steps of:
a) providing the system;
b) positioning the system in the lumen;
c) deploying the distal body from the distal end of the catheter;
d) allowing the height and width of the distal body to increase;
e) irradiating the distal body with x-rays;
f) moving the clot into the distal basket interior; and
g) moving the distal body proximally out of the blood vessel.

Optionally, the exemplary method further comprises irradiating the distal body with x-rays at at least two different angles. Optionally, at least one x-ray marker attached to the distal crowns is distal to the clot when the distal body is deployed from the distal end of the catheter. Optionally, the exemplary method further comprises applying contrast dye proximally and distally to the clot. Optionally, the exemplary method further comprises providing a suction catheter having a proximal end and a distal end, and attaching the distal end of the suction catheter to the clot by applying suction to the suction catheter. Optionally, the exemplary method further comprises aspirating by hand a pre-determined volume of fluid from the suction catheter using a syringe and then locking the syringe at the pre-determined volume. Optionally, the exemplary method further comprises delivering the suction catheter adjacent to the clot by advancing the catheter over the pull wire.

In yet another embodiment, the system includes:
a pull wire having a proximal end and a distal end;
a distal body attached to the pull wire, the distal body comprising an interior, a proximal end, a distal end, a distal body length extending from the proximal end to the distal end, a proximal hub (preferably in the form of a tube) forming the proximal end of the distal body, a basket comprised of a plurality of cells formed by a plurality of basket strips, a plurality of proximal strips, and optionally a distal hub (preferably in the form of a tube) forming a distal end of the basket, the basket comprising a basket interior, each proximal strip having a proximal end attached to the proximal hub, and a distal end attached to a cell, the distal body having a relaxed state wherein the distal body has a first height and a first width, and a collapsed state wherein the distal body has a second height and a second width, the second height less than the first height, the second width less than the first width; and
a catheter having an interior, a proximal end leading to the interior and a distal end leading to the interior, the catheter comprised of a biocompatible material and configured to envelope the distal body when the distal body is in the collapsed state,
wherein, in the relaxed state, the basket comprises a first pair of distal crowns not attached to another cell of the basket and pointing generally in the distal direction, the first pair of distal crowns located approximately the same distance from the proximal hub and approximately 180 degrees relative to each other (e.g., between about 150 degrees and about 180 degrees relative to each other), and further wherein the basket further comprises a second pair of distal crowns not attached to another cell of the basket and pointing generally in the distal direction, the second pair of distal crowns located distally relative to, and approximately 90 degrees relative to, the first pair of distal crowns (e.g., each distal crown of the second pair of distal crowns is located approximately 60 degrees to 90 degrees relative to a distal crown of the first pair of distal crowns), the distal crowns in the second pair of distal crowns located approximately the same distance from the proximal hub, wherein each distal crown of the first and second pair of distal crowns form a cell, each cell further comprising a proximal crown pointing generally in the proximal direction and connected to a memory metal strip, wherein each of the distal crowns in the first pair and second pair of distal crowns curve radially inward toward the basket interior in the relaxed state, wherein the distal crowns of the first pair of distal crowns are configured to contact each other when an exterior, external compressive force (e.g., a thrombus) is exerted on a distal crown of the first pair of distal crowns when the distal body is in the relaxed state, and further wherein the distal crowns of the second pair of distal crowns are configured to contact each other when an exterior, external compressive force (e.g., a thrombus) is exerted on a distal crown of the second pair of distal crowns when the distal body is in the relaxed state. When it is said that a proximal crown pointing generally in the proximal direction and is connected to a memory metal strip, it is meant that the proximal crown is either connected to a basket strip or a proximal strip comprised of a memory metal (e.g., nitinol). When it is said that the first pair of distal crowns are located approximately the same distance from the proximal hub, it will be understood that if one of the first pair of distal crowns is located X distance from the proximal hub, the other of the first pair of distal crowns is located X distance plus or minus (+/-) 0.5 mm from the proximal hub. Similarly, when it is said that the second pair of distal crowns are located approximately the same distance from the proximal hub, it will be understood that if one of the second pair of distal crowns is located Y distance from the proximal hub, the other of the first pair of distal crowns is located Y distance plus or minus (+/-) 0.5 mm from the proximal hub. Optionally, instead of a distal hub, the basket includes an open distal end.

Optionally, the proximal hub is located approximately in the center of the first height and first width in the relaxed state. For example, preferably the proximal hub is located within 0.5 mm of the center of first width and the first height. Optionally, the catheter is comprised of a polymeric material (i.e., one or more polymeric materials such as silicone, PVC, latex rubber or braided nylon). Optionally, the pull wire is comprised of a biocompatible metallic material (e.g., a biocompatible metal or a biocompatible metal alloy). Optionally, in the relaxed state, the basket interior is substantially hollow. Optionally, the proximal end of a first proximal strip is located at least about 65 degrees (e.g., between about 65 and about 180 degrees) relative to the distal end of the first proximal strip, wherein the proximal end of a second proximal strip is located at least about 65 degrees (e.g., between about 65 and about 180 degrees) relative to the distal end of the second proximal strip, and further wherein the first and second proximal strips intersect adjacent and distal to the proximal hub (e.g., within about 0 mm and about 4 mm of the proximal hub). Optionally, each distal crown in the first and second pair of distal crowns forms part of an enlarged cell and further wherein the surface area of each enlarged cell in the relaxed state is at least twice as large as the surface area of each other individual cell of the basket and further wherein the enlarged cells are configured to allow a thrombus to pass therethrough and into the basket interior. Optionally, the pull wire is attached to the proximal hub. Optionally, the basket, the proximal hub and the proximal strips are comprised of a memory metal, wherein the proximal hub comprises a proximal end and a distal end, and further wherein the proximal strips are integral with the distal end of the proximal hub. Optionally, the distal body further comprises a lead wire extending distally from the distal hub, the lead wire having a length of from about 3 mm to about 10 mm. Optionally, the distal hub, the proximal hub, and the basket are comprised of a nitinol having the same material composition and further wherein the proximal and the distal hubs are tubular and generally cylindrical in shape and each has an outer diameter and an inner diameter, the inner diameter forming apertures of the proximal and distal hubs and further wherein the outer diameters of the proximal and distal hubs are substantially the same size and further wherein the inner diameters of the proximal and distal hubs are substantially the same size. Optionally, the length of the distal body from the proximal hub to the distal hub (not including any lead wire) is from about 20 mm to about 65 mm.

Optionally, the system is used in an exemplary method of removing a blood clot from a blood vessel of an animal the exemplary method comprising the steps of:
a) providing the system;
b) positioning the system in the lumen;
c) deploying the distal body from the distal end of the catheter;
d) allowing the height and width of the distal body to increase;
e) irradiating the distal body with x-rays;
f) moving the clot into the distal basket interior; and
g) moving the distal body proximally out of the blood vessel.

Optionally, the exemplary method further comprises irradiating the distal body with x-rays at at least two different angles.

In other embodiments the present disclosure provides a system for removing objects within an interior lumen of an animal, the system comprising:
a pull wire having a proximal end, a distal end and a pull wire longitudinal axis extending from the proximal end to the distal end;
a coaxial sheath having a hollow interior, an open proximal end leading to the interior, and an open distal end leading to the interior, the coaxial sheath enveloping the pull wire, the coaxial sheath slideable along at least a segment of the pull wire;
a distal basket comprising an interior, a proximal end, a distal end, a distal basket length extending from the distal basket proximal end to the distal basket distal end, a distal basket height perpendicular to the distal basket length, a plurality of proximal cells defined by a plurality of proximal cell memory metal strips, each proximal cell comprising a proximal crown located at the proximal end of the proximal cell and pointing generally in the proximal direction and a distal crown located at the distal end of the proximal cell and pointing generally in the distal direction, and a plurality of distal cells distal to the proximal cells;
a plurality of proximal strips, each proximal strip having a proximal end extending from the coaxial sheath, a distal end attached to a proximal crown of a proximal cell and a length extending from the proximal end to the distal end; and
a catheter having a hollow interior, a proximal end leading to the interior and a distal end leading to the interior, the catheter comprised of a biocompatible material,
the distal basket comprised of a memory metal and having:
   a relaxed state in which the distal end of the coaxial sheath is located at a first position along the pull wire, the first position located a first distance proximal to the proximal crowns, and in which the distal basket, as measured at the proximal-most crown, has a first height,
   a proximal collapsed state in which the distal end of the coaxial sheath is located at a second position along the pull wire, the second position located a second distance proximal to the proximal crowns, and in which the distal basket, as measured at the proximal-most crown, has a second height, the second distance greater than the first distance, the second height less than the first height, and
   a distal collapsed state in which the distal end of the coaxial sheath is located at a third position along the pull wire, the third position distal to the proximal crowns and located in the basket interior, and in which the distal basket, as measured at the proximal-most crown, has a third height, the third height less than the first height,
   wherein the catheter is configured to envelope the distal basket when the distal basket is in the proximal collapsed state;
   wherein the distal basket is configured to move from the relaxed state to the proximal collapsed state by moving the distal end of the coaxial sheath proximally to the second position while keeping the distal basket at a fixed location along the pull wire; and
   wherein the distal basket is configured to move from the relaxed state to the distal collapsed state by moving the distal end of the coaxial sheath distally to the third position while keeping the distal basket at a fixed location along the pull wire.

Optionally, each proximal crown comprises a proximal tip and further wherein each proximal strip is configured to cover a proximal tip when the distal basket is in the distal collapsed state. Optionally, each proximal crown comprises an eyelet and further wherein each proximal strip passes through an eyelet. Optionally, the distal end of each proximal strip comprises a loop attaching the proximal strip to an eyelet. Optionally, each proximal crown has an interior surface facing the distal basket interior and an exterior surface opposite the interior surface and further wherein each proximal strip contacts an exterior surface of a proximal crown in the proximal collapsed state and in the distal collapsed state. Optionally, the pull wire extends through the distal basket interior and further wherein the proximal crowns are configured to move towards each other and towards the pull wire when the distal basket moves from the relaxed state to the distal collapsed state and when the distal basket moves from the relaxed state to the proximal collapsed state. Optionally, the proximal crowns are configured to remain a fixed distance from the distal end of the distal basket when the distal basket moves from the relaxed state to the distal collapsed state. Optionally, the coaxial sheath is a braided catheter comprised of a plurality of braids, and further wherein the proximal segments of the braids are wound together to form the braided catheter and further wherein an unwound distal segment of each braid forms a proximal strip. Optionally, at least one proximal crown further comprises an x-ray marker. Optionally, the proximal ends of the proximal strips are integral with the coaxial sheath. Optionally, the proximal ends of the proximal strips are attached to the coaxial sheath. Optionally, the system comprises between two and four proximal strips and the proximal strips are spaced substantially evenly apart. Optionally, the proximal strips have a length of from about 5 millimeters to about 40 millimeters in the relaxed state. Optionally, the pull wire extends through the basket interior from the distal basket proximal end to the distal basket distal end. Optionally, the coaxial sheath interior has a size and shape, and further wherein the size and shape of the coaxial sheath interior are configured to prevent a segment of the pull wire located in the basket interior and distal relative to the distal end of the coaxial sheath from moving through the coaxial sheath interior. Optionally, the distal end of the distal basket comprises a distal tube having an open proximal end and an open distal end, the distal tube comprised of a memory metal. Optionally, the distal basket and the distal were prepared from the same memory metal tube. Optionally, the second and third position along the pull wire each comprise an x-ray marker. Optionally, the distal tube is attached to the pull wire such that the distal tube is not slideable along the pull wire. Optionally, all proximal crowns of the proximal cells are attached to a proximal strip. Optionally, the distal basket further comprises a lead wire extending distally from the distal basket. Optionally, the proximal strips and the distal basket have a different material composition. Optionally, the proximal strips are comprised of a polymer. Optionally, the polymer is selected from the group consisting of fluorinated ethylene propylene, polytetrafluoroethylene, and tetrafluoroethtylene. Optionally, the proximal strips are comprised of a material selected from the group consisting of plastic, rubber, nylon, suture material, and braided catheter material.

Optionally, the system is used in an exemplary method of removing a clot from a blood vessel of an animal, the blood vessel having an interior wall forming the blood vessel, the exemplary method comprising the steps of:
a) providing the system, wherein the coaxial sheath is located in the catheter interior and the distal basket is located in the catheter interior in a collapsed state;
b) positioning the catheter in the blood vessel;
c) deploying the distal basket from the distal end of the catheter so that the proximal crowns of the proximal cells are distal to the clot;
d) allowing the distal basket to move to the relaxed state;
e) moving the coaxial sheath distally to a fourth position, the fourth position located distally beyond the proximal crowns and in the basket interior but proximal to the third position (this third position is not sufficiently distal to the proximal crowns to place tension on the proximal strips; thus, the crowns do not begin to move towards each other and the pull wire);
f) capturing the clot in the distal basket interior;
g) moving the coaxial sheath further distally into the basket interior (i.e., to or near) the third position so that the distal basket height, as measured at the proximal-most crown, decreases and the proximal crowns move toward each other and the pull wire; and
h) moving the system proximally out of the blood vessel.

In still further embodiments, the present disclosure provides a system for removing objects within an interior lumen of an animal, the system comprising:
a pull wire having a proximal end, a distal end and a pull wire longitudinal axis extending from the proximal end to the distal end;
a coaxial sheath having an open proximal end and an open distal end, the coaxial sheath enveloping the pull wire, the coaxial sheath slideable along at least a segment of the pull wire;
a distal basket comprising an interior, a proximal end, a distal end, a distal basket length extending from the distal basket proximal end to the distal end, a distal basket height perpendicular to the distal basket length, a plurality of proximal cells defined by a plurality of proximal cell memory metal strips, each proximal cell comprising a proximal crown located at the proximal end of the proximal cell and pointing generally in the proximal direction and a distal crown located at the distal end of the proximal cell and pointing generally in the distal direction, and a plurality of distal cells distal to the proximal cells;
a plurality of proximal strips, each proximal strip having a proximal end extending from the coaxial sheath, a distal end attached to a crown of a proximal cell and a length extending from the proximal end to the distal end; and
a catheter having a hollow interior, a proximal end leading to the interior and a distal end leading to the interior, the catheter comprised of a biocompatible material,
the distal basket comprised of a memory metal,
wherein each proximal crown of each proximal cell comprises an eyelet and further wherein each proximal strip passes through an eyelet.

The present disclosure also provides additional modular, easy-to-manufacture platform of systems for retrieving hard clots and other objects in animal lumens. In some embodiments, the system includes a proximal tube, a distal tube, and a plurality of memory metal strips between the proximal and distal tubes. The plurality of memory metal strips form a wide range of basket designs. Preferably, the proximal tube, memory metal strips, and distal tube are derived from a standard, off-the-shelf single tube of memory metal (e.g., a memory metal alloy such as nitinol), with the proximal tube and distal tube having the same inner diameter and outer diameter as the native tube from which they were derived and with the basket formed by cutting the middle portion of the native tube and expanding and shape-setting this cut portion. Preferably, the proximal tube and distal tube have an outer diameter that is from about 0.51 mm (0.02 inches) to about 0.76 mm (0.03 inches) (e.g., about 0.69 mm (0.027 inches)) so that the device fits inside a standard microcatheter and an inner diameter that is from about 0.25 mm (0.01 inches) to about 0.51 mm (0.02 inches). Preferably, there are no welded parts between the proximal tube and distal tube, which makes the system easy and cheap to reliably manufacture. The system also includes one or more catheters for deploying the system, a pull wire that passes through the hollow interior of the proximal tube, and a coaxial tube. Preferably, the system includes two catheters - a guide catheter and a microcatheter. The coaxial tube envelopes the pull wire, is slideable along at least a segment of the pull wire, and is attached to the proximal hub. The coaxial tube allows a user to move the proximal hub toward and away from the distal hub while keeping the distal hub stationary. Movement of the proximal hub toward and away from the distal hub causes conformational changes in the basket, including (depending on the basket design and the location of the proximal tube), collapsing the basket, expanding the basket, strengthening the basket, and moving the basket around the clot. The plurality of memory metal strips attached to the proximal hub include a plurality of proximal tether memory metal strips, which have a proximal end attached to the distal end of the proximal tube. The length and thickness of the proximal tether memory metal strips vary in the different embodiments described herein, which allows the surgical user to select from the various embodiments in the platform based on the features needed for the particular operation (e.g., vessel anatomy and hardness of the clot).

The present disclosure describes an exemplary method of manufacturing a system for removing objects within an interior lumen of an animal that includes:
a) providing a single tube comprised of a memory metal, the single tube having an exterior, a hollow interior, a wall separating the exterior from the hollow interior, a proximal portion comprising an aperture leading to the hollow interior, a distal portion comprising an aperture leading to the hollow interior, and a middle portion between the proximal portion and the distal portion;
b) cutting the wall of the middle portion with a laser;
c) removing the pieces of the middle portion cut by the laser to form a basket system comprising a proximal tube comprising a hollow interior extending through said proximal tube, said proximal tube having a proximal end and a distal end, a distal tube comprising a hollow interior extending through said distal tube, and a middle portion located between said proximal tube and said distal tube and comprising a plurality of proximal tether memory metal strips, each proximal tether memory metal strip having a proximal end attached to the distal end of the proximal tube and a distal end;
d) altering the shape of the middle portion;
e) allowing the middle portion to expand relative to the distal tube and the proximal tube to form a basket that includes a plurality of cells;
f) optionally, inserting a pull wire through said proximal tube interior so that said proximal tube is slideable along at least a portion of said pull wire, said pull wire having a proximal end and a distal end; and
g) optionally, attaching said pull wire to said distal hub.

Instead of steps f) and g) noted above, the exemplary method may include inserting a pull wire comprising a proximal end, a distal end, a stop located adjacent to said distal end, through said proximal tube interior, said stop having a width and/or height that is greater than said proximal tube interior, said stop located distal relative to said proximal tube interior, so that said proximal tube is slideable distally until the proximal hub reaches said stop, said pull wire not contacting said distal tube. In such examples, the pull wire does not contact the distal hub. Rather in these examples, the exemplary method further includes attaching a leader wire to said distal tube

In some examples, either of the above exemplary methods further include h) providing a coaxial tube, said coaxial tube comprising a hollow interior receiving said pull wire, a proximal end, and a distal end, and i) attaching said distal end of said coaxial tube to said proximal tube. In some examples, the exemplary method of attaching said distal end of said coaxial tube to said proximal tube comprises welding said distal end of said coaxial tube to said proximal tube. In other examples, the exemplary method of attaching said distal end of said coaxial tube to said proximal tube comprises shrink wrapping said distal end of said coaxial tube to said proximal tube. In other examples, the exemplary method of attaching said distal end of said coaxial tube to said proximal tube comprises gluing said distal end of said coaxial tube to said proximal tube.

Optionally, after step e, the basket further comprises a row of proximal cells, each proximal cell defined by a plurality of memory metal strips and comprising a proximal crown located at a proximal end of the cell and pointing in the proximal direction and a distal crown located at a distal end of the cell and pointing in the distal direction and further wherein each of said proximal crowns of said proximal cells is attached to a distal end of a proximal tether memory metal strip. Optionally, after step e, the basket further comprises a row of distal cells located distal to said proximal cells and connected to said distal crowns of said proximal cells, each distal cell defined by a plurality of memory metal strips and comprising a proximal crown located at a proximal end of the cell and pointing in the proximal direction and a distal crown located at a distal end of the cell and pointing in the distal direction, and further wherein the number of distal cells is twice the number of proximal cells. Optionally, after step e, the basket further comprises a row of distal crowns distal to said proximal crowns and pointing in the distal direction and further wherein the number of distal crowns in said row is twice the number of proximal crowns attached to said proximal tether memory metal strip.

Optionally, after step e, the basket system further comprises a row of strut memory metal strips, each strut memory metal strip having a proximal end attached to a distal crown of a proximal cell and a distal end attached to a proximal crown of a distal cell. Optionally, the basket comprises no welded components and said proximal tether memory metal strips are integral with said proximal cell crowns.

Optionally, after step e, the basket system comprises between two and four proximal tether memory metal strips. Optionally, the exemplary method further comprises not altering the shape of the proximal and distal portions while altering the shape of the middle portion. Optionally, the exemplary method further comprises cooling the proximal portion, the middle portion, and the distal portion after step D) and, after cooling, the proximal and distal portions have substantially the same size as the proximal and distal portions had prior to step A). Optionally, the method of allowing said middle portion to expand comprises heating the middle portion. Optionally, the exemplary method of altering the shape of the middle portion comprises using a mandrel. Optionally, the mandrel is tapered. Optionally, the proximal portion and the distal portion are not cut by the laser. Optionally, prior to cutting the memory metal tube, the memory metal tube has an outer diameter that is from about 0.28 mm (0.011 inches) to about 1.37 mm (0.054 inches) and an inner diameter that is from about 0.20 mm (0.008 inches) to about 1.30 mm (0.051 inches). Optionally, after step e), the proximal tube and distal tube have an outer diameter that is from about 0.51 mm (0.02 inches) to about 0.76 mm (0.03 inches) and an inner diameter that is from about 0.25 mm (0.01 inches) to about 0.51 mm (0.02 inches). Optionally, the exemplary method further includes placing said basket inside a catheter comprised of a biocompatible material. Optionally, the exemplary method further includes the steps of placing the basket inside a lumen of an animal and using the basket to retrieve an object located inside said lumen.

The present disclosure also provides several systems for removing objects within an interior lumen of an animal. In some embodiments, the system includes:
a pull wire having a proximal end, a distal end and a pull wire longitudinal axis extending from said proximal end to said distal end;
a distal basket attached to said pull wire, said distal basket comprising a proximal end, a distal end, a distal basket length extending from said distal basket proximal end to said distal end, a distal basket height perpendicular to said distal basket length and said pull wire longitudinal axis, a proximal hub located at said proximal end of the distal basket, said proximal hub comprising a hollow interior, said pull wire passing through said proximal hub hollow interior, said proximal hub slideable along at least a segment of the pull wire, a plurality of proximal tether memory metal strips, a plurality of proximal cells defined by a plurality of proximal cell memory metal strips, each proximal cell comprising a proximal crown located at the proximal end of the proximal cell and pointing generally in the proximal direction and a distal crown located at the distal end of the proximal cell and pointing generally in the distal direction, each proximal tether memory metal strip having a proximal end attached to said proximal hub, a distal end attached to a crown of a proximal cell and a length extending from said proximal end to said distal end, a plurality of distal cells distal to the proximal cells, and a distal hub located at said distal end of said distal basket and comprising a hollow interior,
   said distal basket having
a relaxed state in which said proximal hub is located a first distance proximal to said proximal crowns and wherein said distal basket has a first height, as measured at the proximal-most crown,
a gaping state in which said proximal hub is located a second distance from said proximal crowns and wherein has a second height, as measured at the proximal-most crown, said second height greater than said first height, said second distance less than said first distance,
a proximal collapsed state in which said proximal hub is located a third distance proximal to said proximal crowns and wherein said distal basket has a third height, as measured at the proximal-most crown, said third distance greater than said first distance, said third height less than said first height,
a catheter having a hollow interior, a proximal end leading to said interior and a distal end leading to said interior, said catheter comprised of a biocompatible material and configured to envelope said distal basket when said distal basket is in said proximal collapsed state;
wherein said distal basket is configured to move from said relaxed state to said gaping state by moving said proximal hub distally relative to said distal hub; and
   wherein said distal basket is configured to move from said expanded state to said proximal collapsed state by moving said proximal hub proximally relative to said distal hub.

In some embodiments, the proximal tether memory metal strips have a thickness of between about 25% and 75% of the memory metal strips forming the proximal cell of the distal basket. In these embodiments, translation of the proximal hub toward the stationary distal hub deforms the tethers instead of the distal basket. In other embodiments, the proximal tether memory metal strips are as thick or thicker than the memory metal strips forming the proximal cells of the distal basket (e.g., between about 100% and 175% of the thickness of the memory metal strips forming the proximal cells of the basket). In these embodiments with thicker proximal tether memory metal strips, the proximal tether memory metal strips resist deforming when the proximal hub is translated distally toward the stationary distal hub and instead the proximal tether memory metal strips are bowed out laterally, dissecting through or around the clot and centering, buttressing and strengthening the opening of the basket. Generally, in both embodiments, moving the proximal hub towards the distal hub when the basket is in the relaxed state causes the proximal crowns of the proximal cells to move apart from each other, thereby expanding the opening of the distal basket. Preferably, in the embodiments with the thin tethers, in the relaxed state, the tethers have a length of from about 3 mm to about 10 mm, and in the embodiments with the thick tethers, the tethers have a length of from about 10 mm to about 20 mm.

Optionally, the distal basket further comprises a distal collapsed state in which said proximal hub is located distal to said proximal crowns and wherein said distal basket has a fourth height, as measured at the proximal-most crown, said fourth height less than said first height and, wherein said catheter is configured to envelope said distal basket when said distal basket is in said distal collapsed state, and further wherein said distal basket is configured to move from said gaping state to said distal collapsed state by moving said proximal hub distally relative to said distal hub. Optionally, the system further includes a coaxial tube, said coaxial tube configured to be received in said catheter, said coaxial tube having a proximal end, a distal end attached to said proximal hub, and a hollow interior, said pull wire passing through said coaxial tube hollow interior, said coaxial tube slideable along at least a segment of said pull wire. In some embodiments with the thin proximal memory metal strips, the combined length of two of said proximal tether memory metal strips is within about 2 mm of said second height. In other embodiments with the thin proximal memory metal strips, the combined length of two of said proximal tether memory metal strips is within about 2 mm of said second height multiplied by a factor of two. Optionally, said pull wire extends from said distal basket proximal end to said distal basket distal end. Optionally, said pull wire is not in contact with said distal hub. Optionally, in said gaping state, said proximal hub is located parallel to said proximal crown. Optionally, said pull wire and said proximal hub are offset from the center of the distal basket height, as measured at the proximal-most crown. Optionally, all proximal crowns of said proximal cells are attached to a proximal tether memory metal strip. In other embodiments, the system has four proximal cells, each proximal cell having a proximal crown, and not all (e.g., only two) of the proximal crowns are attached to a proximal tether memory metal strip. Optionally, said distal basket further comprises a plurality of strut memory metal strips and plurality of distal cells defined by a plurality of distal memory metal strips, said distal cells comprising a proximal crown located at a proximal end of said distal cells and a distal crown located at a distal end of said distal cells, said strut memory metal strips having a proximal end attached to a distal crown of a proximal cell and a distal end attached to a proximal crown of a distal cell. Optionally, the distal basket comprises between two and four proximal tether memory metal strips. Optionally, said proximal memory metal strips are integral with said proximal hub. Optionally, said proximal hub is a tube, wherein said interior of said proximal hub has a size and shape, and further wherein said size and shape of said proximal hub interior are configured to prevent a segment of said pull wire distal relative to said proximal hub from moving through proximal hub interior. Optionally, said distal hub is a tube. Optionally, said distal hub is attached to said pull wire such that said distal hub is not slideable along said pull wire. Optionally, said distal basket further comprises a lead wire extending distally from said distal hub. Optionally, said distal hub, said proximal hub, and said basket are comprised of a nitinol having the same material composition. Optionally, said distal basket further comprises an x-ray marker. Optionally, said proximal and said distal hubs are generally cylindrical in shape and each has an outer diameter and an inner diameter, the inner diameter forming apertures of the proximal and distal hubs and further wherein the outer diameters of the proximal and distal hubs are substantially the same size and further wherein the inner diameters of the proximal and distal hubs are substantially the same size. Optionally, the outer diameters of the proximal and distal hubs are from about 0.28 mm (0.011 inches) to about 1.37 mm (0.054 inches), and further wherein the inner diameters of the proximal and distal hubs are from about 0.20 mm (0.008 inches) to about 1.30 mm (0.051 inches). Optionally, the proximal tube and distal tube have an outer diameter that is from about 0.51 mm (0.02 inches) to about 0.76 mm (0.03 inches) and an inner diameter that is from about 0.25 mm (0.01 inches) to about 0.51 mm (0.02 inches). Optionally, the pull wire is generally cylindrical and further wherein the diameter of the pull wire is between about 0.20 mm (0.008 inches) and about 1.30 mm (0.051 inches). Optionally, the first height of the distal basket is between about 2 millimeters and about 8 millimeters. Optionally, said proximal tether memory metal strips rotate about said pull wire longitudinal axis such that a distal end of a proximal tether memory metal strip is located between about 90 and about 270 degrees relative to said proximal end of the same proximal tether memory metal strip.

The present disclosure also provides an exemplary method of removing an object from an interior lumen of an animal, said lumen having an interior wall forming said lumen. In some embodiments, the exemplary method includes:
a) providing the system described above;
b) positioning the system in said lumen, said basket located in said catheter in a collapsed state;
c) deploying said distal basket from said distal end of said catheter so that said proximal crowns of said proximal cells are distal to said obstruction;
d) allowing said distal basket to move to said relaxed state;
e) moving said proximal hub distally relative to said distal hub so that said distal basket height, as measured at the proximal-most crown, increase;
f) moving said distal basket over said obstruction; and
g) removing said distal basket and said obstruction from said lumen.

Optionally, the interior lumen is an intracranial artery and said obstruction is a blood clot. Optionally, the exemplary method further comprises using said blood clot to move said proximal hub distally relative to said distal hub and allow said distal basket to move to said gaping state. Optionally, the exemplary method further comprises using a coaxial tube to push said proximal hub distally relative to said distal hub and allow said distal basket to move to said gaping state. Optionally, the exemplary method further includes, after step e, moving said proximal hub relative to said distal hub so that said distal basket height, as measured at the proximal-most crown, decrease. Optionally, after step e, said pull wire and said proximal hub are offset with respect to the center of said distal basket height, as measured at the proximal-most crown, as measured at the proximal-most crown, and the center of said lumen.

The present disclosure also provides a system for removing objects within an interior lumen of an animal, the system comprising:
a pull wire having a proximal end, a distal end and a pull wire longitudinal axis extending from said proximal end to said distal end;
a proximal basket attached to said pull wire, said proximal basket comprising a proximal end, a distal end, a proximal basket length extending from said proximal basket proximal end to said distal end, a proximal basket height perpendicular to said proximal basket length and said pull wire longitudinal axis, a proximal tube located at said proximal end of the proximal basket, said proximal tube comprising a hollow interior, said pull wire passing through said hollow interior and said proximal tube slideable along at least a segment of said pull wire, a plurality of rows of cells, each cell defined by a plurality of memory metal strips, each cell comprising a proximal crown located at the proximal end of the proximal cell and pointing generally in the proximal direction and a distal crown located at the distal end of the proximal cell and pointing generally in the distal direction,
a distal basket attached to said pull wire, said distal basket comprising a proximal end, a distal end, a distal basket length extending from said distal basket proximal end to said distal end, a distal basket height perpendicular to said distal basket length and said pull wire longitudinal axis, a distal tube located at said distal end of the distal basket, said distal tube comprising a hollow interior, a plurality of rows of cells, each cell defined by a plurality of memory metal strips, each cell comprising a proximal crown located at the proximal end of the proximal cell and pointing generally in the proximal direction and a distal crown located at the distal end of the proximal cell and pointing generally in the distal direction,
a plurality of tether memory metal strips, each tether memory metal strip having a proximal end attached to a distal crown of a cell located at the distal end of said proximal basket and a distal end attached to a proximal crown of a cell located at the proximal end of said distal basket,
said proximal basket having
   a relaxed state wherein said proximal basket has a first height, as measured at the distal-most crown, and said proximal hub is located a first distance proximal to said distal hub;
a collapsed state wherein said proximal basket has a second height, as measured at the distal-most crown, said second height less than said first height;
a gaping state wherein said proximal basket has a third height, as measured at the distal-most crown, and said proximal hub is located a second distance proximal to said distal hub, said third height greater than said first height and said second distance less than said first distance, said proximal basket configured to move from said expanded state to said gaping state by pushing said proximal tube distally relative to said distal tube;
   said distal basket having
a relaxed state wherein said distal basket has a first height and
a collapsed state wherein said distal basket has a second height, said second height less than said first height, and
a catheter having an interior, a proximal end leading to said interior and a distal end leading to said interior, said catheter comprised of a biocompatible material and configured to envelope said distal and said proximal basket when said baskets are in said collapsed state.

Optionally, said proximal tether memory metal strips rotate about said pull wire longitudinal axis such that a distal end of a proximal tether memory metal strip is located between about 90 and about 270 degrees relative to said proximal end of the same proximal tether memory metal strip.

In some arrangements, the system does not include a proximal hub and the system includes soft cords in place of or in addition to the proximal memory metal strips. For example, in one arrangement, the system includes:
a pull wire having a proximal end, a distal end and a pull wire longitudinal axis extending from said proximal end to said distal end;
a coaxial tube having a proximal end, a distal end and a hollow interior, said pull wire passing through said coaxial tube hollow interior, said coaxial tube slideable along at least a segment of said pull wire;
a distal basket attached to said pull wire and said coaxial tube, said distal basket comprising a proximal end, a distal end, a distal basket length extending from said distal basket proximal end to said distal end, a distal basket height perpendicular to said distal basket length and said pull wire longitudinal axis, a plurality of cords, a plurality of proximal cells defined by a plurality of proximal cell memory metal strips, each proximal cell comprising a proximal crown located at the proximal end of the proximal cell and pointing generally in the proximal direction and a distal crown located at the distal end of the proximal cell and pointing generally in the distal direction, each cord having a proximal end attached to said coaxial tube, a distal end attached to a crown of a proximal cell and a length extending from said proximal end to said distal end, a plurality of distal cells distal to the proximal cells, and a distal hub located at said distal end of said distal basket and comprising a hollow interior,
said distal basket having
a relaxed state in which said coaxial tube is located a first distance proximal to said proximal crowns and wherein said distal basket, as measured at the proximal-most crown, has a first height,
a proximal collapsed state in which said coaxial tube is located a second distance proximal to said proximal crowns and wherein said distal basket, as measured at the proximal-most crown, has a second height, said second distance greater than said first distance, said second height less than said first height,
a catheter having a hollow interior, a proximal end leading to said interior and a distal end leading to said interior, said catheter comprised of a biocompatible material and configured to envelope said coaxial tube and said distal basket when said distal basket is in said proximal collapsed state;
wherein said distal basket is configured to move from said relaxed state to said proximal collapsed state by moving said coaxial tube proximally relative to said distal hub.

Optionally, the distal basket further comprises a distal collapsed state in which said coaxial tube is located distal to said proximal crowns and wherein said distal basket, as measured at the proximal-most crown, has a third height, said third height less than said first height, wherein said catheter is configured to envelope said distal basket when said distal basket is in said distal collapsed state, and further wherein said distal basket is configured to move from said relaxed state to said distal collapsed state by moving said coaxial tub distally relative to said distal hub. Optionally said cord is comprised of a material selected from the group consisting of plastic, rubber, nylon, suture material, braided catheter material, platinum coils, and ultrafine nitinol. Optionally, said cords are integral with said coaxial sheath. Optionally, said cords are glued to said coaxial sheath. Optionally, said cords are shrink wrapped to said coaxial sheath. Optionally, said cords have a thickness of about 0.1 mm to about 2.5 mm (about 0.004 to about 0.1 inches) (more preferably, from about 0.1 mm to 0.46 mm (about 0.004 to 0.018 inches)). Optionally, said cords in said relaxed state, have a length of about 3 to about 20 mm. Optionally, said pull wire extends from said distal basket proximal end to said distal basket distal end and said pull wire is attached to said distal hub. Optionally, all proximal crowns of said proximal cells are attached to a cord. Optionally, the basket comprises four proximal cells, each proximal cell having a proximal crown, and not all (e.g., only two) of the proximal crowns are attached to a cord. Optionally, said distal basket further comprises a plurality of strut memory metal strips and plurality of distal cells defined by a plurality of distal memory metal strips, said distal cells comprising a proximal crown located at a proximal end of said distal cells and a distal crown located at a distal end of said distal cells, said strut memory metal strips having a proximal end attached to a distal crown of a proximal cell and a distal end attached to a proximal crown of a distal cell. Optionally, the distal basket comprises between two and four cords. Optionally, said distal hub is attached to said pull wire such that said distal hub is not slideable along said pull wire. Optionally, said distal basket further comprises a lead wire extending distally from said distal hub. Optionally, said distal hub and said basket are comprised of a nitinol having the same material composition. Optionally, said distal basket and/or said coaxial tube further comprises an x-ray marker. Optionally, said distal hub is generally cylindrical in shape and has an outer diameter and an inner diameter, the inner diameter forming the aperture of the distal hub and further wherein the outer diameter of the distal hub from about 0.28 mm (0.011 inches) to about 1.37 mm (0.054 inches), and further wherein the inner diameter of the distal hub is from about 0.20 mm (0.008 inches) to about 1.30 mm (0.051 inches). Optionally, the distal tube has an outer diameter that is from about 0.51 mm (0.02 inches) to about 0.76 mm (0.03 inches) and an inner diameter that is from about 0.25 mm (0.01 inches) to about 0.51 mm (0.02 inches). Optionally, the pull wire is generally cylindrical and further wherein the diameter of the pull wire is between about 0.20 mm (0.008 inches) and about 1.30 mm (0.051 inches). Optionally, the first height of the distal basket, as measured at the proximal-most crown, is between about 2 millimeters and about 8 millimeters. Optionally, said cords are soft.

The present disclosure provides an exemplary method of removing an object from an interior lumen of an animal, said lumen having an interior wall forming said lumen, the exemplary method comprising the steps of:
a) providing the system described above;
b) positioning the system in said lumen, said basket located in said catheter in a collapsed state;
c) deploying said distal basket from said distal end of said catheter so that said proximal crowns of said proximal cells are distal to said obstruction;
d) allowing said distal basket to move to said relaxed state;
e) moving said coaxial tube distally relative to said distal hub so that said coaxial tube moves distally to the proximal-most crown;
f) moving said distal basket, said pull wire and said coaxial tube proximally so that said distal basket moves over said obstruction;
g) moving said coaxial sheath distally relative to said distal hub so that said distal basket height, as measured at the proximal-most crown, decreases and said coaxial tube is closer to said distal hub as compared to the proximal-most crown; and
h) removing said distal basket and said obstruction from said lumen.

In other examples, the exemplary method includes
a) providing the system described above;
b) positioning the system in said lumen, said basket located in said catheter in a collapsed state;
c) deploying said distal basket from said distal end of said catheter so that said proximal crowns of said proximal cells are distal to said obstruction;
d) allowing said distal basket to move to said relaxed state;
e) moving said coaxial tube distally relative to said distal hub so that said coaxial tube moves distally to the proximal-most crown;
f) moving said distal basket, said pull wire and said coaxial tube proximally so that said distal basket moves over said obstruction;
g) moving said coaxial sheath proximally relative to said distal hub so that said distal basket height, as measured at the proximal-most crown, decreases;
h) moving said catheter distally relative to said distal hub so that said catheter re-sheaths said coaxial sheath and partially re-sheaths said cords, thereby decreasing said distal basket height, as measured at the proximal-most crown;
i) removing said distal basket and said obstruction from said lumen.

Optionally, said interior lumen is an intracranial artery and said obstruction is a blood clot.

In other arrangements that do not include a proximal hub, the system includes
a pull wire having a proximal end, a distal end and a pull wire longitudinal axis extending from said proximal end to said distal end;
a coaxial tube having a proximal end, a distal end and a hollow interior, said pull wire passing through said coaxial tube hollow interior, said coaxial tube slideable along at least a segment of said pull wire;
a distal basket attached to said pull wire and said coaxial tube, said distal basket comprising a proximal end, a distal end, a distal basket length extending from said distal basket proximal end to said distal end, a distal basket height perpendicular to said distal basket length and said pull wire longitudinal axis, a plurality of proximal tether memory metal strips, a plurality of cords, a plurality of proximal cells defined by a plurality of proximal cell memory metal strips, each proximal cell comprising a proximal crown located at the proximal end of the proximal cell and pointing generally in the proximal direction and a distal crown located at the distal end of the proximal cell and pointing generally in the distal direction, each proximal tether memory metal strip having a proximal end attached to said coaxial tube and a distal end, each cord having a proximal end attached to a distal end of a proximal tether memory metal strip and a distal end attached to a crown of a proximal cell and a length extending from said proximal end to said distal end, and a plurality of distal cells distal to the proximal cells, and a distal hub located at said distal end of said distal basket and comprising a hollow interior,
said distal basket having
a relaxed state in which said distal basket, as measured at the proximal-most crown, has a first height,
a collapsed state in which said distal basket, as measured at the proximal-most crown, has a second height, said second height less than said first height,
a catheter having a hollow interior, a proximal end leading to said interior and a distal end leading to said interior, said catheter comprised of a biocompatible material and configured to envelope said coaxial tube and said distal basket when said distal basket is in said collapsed state.

Optionally, said cord is comprised of a material selected from the group consisting of plastic, rubber, nylon, suture material, braided catheter material, platinum coils and ultrafine nitinol. Optionally, said proximal tether memory metal strips are integral with said coaxial sheath. Optionally, said cords are glued to said proximal tether memory metal strips. Optionally, said cords are shrink wrapped to said proximal tether memory metal strips. Optionally, said cords have a thickness of from about 0.1 mm and about 2.5 mm (about 0.004 and about 0.1 inches) (more preferably about 0.1 mm to about 0.46 mm (about 0.004 to about 0.018 inches)) and said cords have a length of from about 3 mm to about 10 mm in said relaxed state. Optionally, said pull wire extends from said distal basket proximal end to said distal basket distal end and said pull wire is attached to said distal hub. Optionally, all proximal crowns of said proximal cells are attached to a cord. Optionally, the basket comprises four proximal cells, each proximal cell having a proximal crown, and not all (e.g., only two) of the proximal crowns are attached to a cord. Optionally, said distal basket further comprises a plurality of strut memory metal strips and plurality of distal cells defined by a plurality of distal memory metal strips, said distal cells comprising a proximal crown located at a proximal end of said distal cells and a distal crown located at a distal end of said distal cells, said strut memory metal strips having a proximal end attached to a distal crown of a proximal cell and a distal end attached to a proximal crown of a distal cell. Optionally, the distal basket comprises between two and four cords. Optionally, said distal hub is attached to said pull wire such that said distal hub is not slideable along said pull wire. Optionally, said distal basket further comprises a lead wire extending distally from said distal hub. Optionally, said distal hub and said basket are comprised of a nitinol having the same material composition. Optionally, said distal basket and/or said coaxial tube further comprises an x-ray marker. Optionally, said distal hub is generally cylindrical in shape and has an outer diameter and an inner diameter, the inner diameter forming the aperture of the distal hub and further wherein the outer diameter of the distal hub is from about 0.28 mm (0.011 inches) to about 1.37 mm (0.054 inches), and further wherein the inner diameter of the distal hub is from about 0.20 mm (0.008 inches to about 1.30 mm (0.051 inches). Optionally, the distal tube has an outer diameter that is from about 0.51 mm (0.02 inches) to about 0.76 mm (0.03 inches) and an inner diameter that is from about 0.25 mm (0.01 inches) to about 0.51 mm (0.02 inches). Optionally the pull wire is generally cylindrical and further wherein the diameter of the pull wire is between about 0.20 mm (0.008 inches) and about 1.30 mm (0.051 inches). Optionally, the first height of the distal basket, as measured at the proximal-most crown, is between about 2 millimeters and about 8 millimeters. Optionally, the cords are soft.

In some examples, the above system is used in an exemplary method of removing an object from an interior lumen of an animal, said lumen having an interior wall forming said lumen that includes
a) providing the above system;
b) positioning the system in said lumen, said basket located in said catheter in a collapsed state;
c) deploying said distal basket from said distal end of said catheter so that said proximal crowns of said proximal cells are distal to said obstruction, said coaxial sheath is proximal to said obstruction, said proximal tether memory metal strips are proximal to said obstruction, and said cords are adjacent to said obstruction;
d) allowing said distal basket to move to said relaxed state;
e) moving said coaxial tube distally relative to said distal hub so that said proximal tether memory metal strips move distally relative to the proximal-most crown and said obstruction is sandwiched between said proximal tether memory metal strips and said proximal crowns of said proximal cells;
f) removing said distal basket and said obstruction from said lumen.

Optionally said interior lumen is an intracranial artery and said obstruction is a blood clot.

In still further embodiments, the system includes a first wire that is attached to the proximal tube (but not the distal tube) and a second wire that is attached to the distal tube (but not the proximal tube). Preferably, in such embodiments, the system includes two catheters - a guide catheter and a microcatheter. The plurality of memory metal strips attached to the proximal hub include a plurality of proximal tether memory metal strips, which have a proximal end attached to the distal end of the proximal tube. In some examples, the present disclosure provides an exemplary method of manufacturing a system for removing objects within an interior lumen of an animal comprising:
a) providing a single tube comprised of a memory metal, the single tube having an exterior, a hollow interior, a wall separating the exterior from the hollow interior, a proximal portion comprising an aperture leading to the hollow interior, a distal portion comprising an aperture leading to the hollow interior, and a middle portion between the proximal portion and the distal portion;
b) cutting the wall of the middle portion with a laser;
c) removing the pieces of the middle portion cut by the laser to form a basket system comprising a proximal tube comprising a proximal end, a distal end, and a hollow interior extending through said proximal tube, a distal tube comprising a hollow interior extending through said distal tube, and a middle portion located between said proximal tube and said distal tube and comprising a plurality of proximal memory metal tether strips, each proximal memory metal tether strip having a proximal end attached to the distal end of said proximal tube and a distal end,
d) altering the shape of the middle portion;
e) allowing the middle portion to expand relative to the distal tube and the proximal tube;
f) attaching a first wire to the proximal tube; and
g) attaching a second wire to the distal tube.

Optionally, after step e, the basket system further comprises a row of proximal cells, each proximal cell defined by a plurality of memory metal strips and comprising a proximal crown located at a proximal end of the cell and pointing in the proximal direction and a distal crown located at a distal end of the cell and pointing in the distal direction and further wherein each of said proximal crowns of said proximal cells is attached to a distal end of a proximal tether memory metal strip.

Optionally, after step e, the basket system further comprises a row of distal cells located distal to said proximal cells and connected to said distal crowns of said proximal cells, each distal cell defined by a plurality of memory metal strips and comprising a proximal crown located at a proximal end of the cell and pointing in the proximal direction and a distal crown located at a distal end of the cell and pointing in the distal direction, and further wherein the number of distal cells is twice the number of proximal cells. Optionally, after step e, the basket system further comprises a row of strut memory metal strips, each strut having a proximal end attached to a distal crown of a proximal cell and a distal end attached to a proximal crown of a distal cell. Optionally, after step e, the basket system further comprises a row of distal crowns located distal to said proximal crowns and pointing in the distal direction, and further wherein the number of distal crowns in said row is twice the number of proximal crowns attached to said proximal tether memory metal strips. Optionally, the step of attaching said first wire to said proximal tube comprises placing said first wire inside said aperture of said proximal tube and gluing said first wire to said proximal tube. Optionally, the step of attaching said first wire to said proximal tube comprises placing said first wire inside said aperture of said proximal tube and welding said first wire to said proximal tube. Optionally, the step of attaching said first wire to said proximal tube comprises shrink wrapping said first wire to said proximal tube. Optionally, after step e, the basket system comprises between two and four proximal tether memory metal strips. Optionally, the exemplary method further comprises not altering the shape of the proximal and distal portions while altering the shape of the middle portion. Optionally, the exemplary method further comprises cooling the proximal portion, the middle portion, and the distal portion after step D) and, after cooling, the proximal and distal portions have substantially the same size as the proximal and distal portions had prior to step A). Optionally, the exemplary method of allowing said middle portion to expand comprises heating the middle portion. Optionally, the exemplary method of altering the shape of the middle portion comprises using a mandrel. Optionally, the mandrel is tapered. Optionally, the proximal portion and the distal portion are not cut by the laser. Optionally, prior to cutting the memory metal tube, the memory metal tube has an outer diameter that is from about 0.28 mm (0.011 inches) to about 1.37 mm (0.054 inches) and an inner diameter that is from about 0.20 mm (0.008 inches) to about 1.30 mm (0.051 inches). Optionally, after step e), the proximal tube and distal tube have an outer diameter that is from about 0.51 mm (0.02 inches) to about 0.76 mm (0.03 inches) and an inner diameter that is from about 0.25 mm (0.01 inches) to about 0.51 mm (0.02 inches). Optionally, the exemplary method further includes placing said basket inside a catheter comprised of a biocompatible material.

The present disclosure also provides a system for removing objects within an interior lumen of an animal. In some embodiments, the system includes
a pull wire having a proximal end, a distal end and a pull wire longitudinal axis extending from said proximal end to said distal end;
a distal basket attached to said pull wire, said distal basket comprising a proximal end, a distal end, a distal basket length extending from said distal basket proximal end to said distal end, a distal basket height perpendicular to said distal basket length and said pull wire longitudinal axis, a proximal tube located at said proximal end of the distal basket, said proximal tube comprising a hollow interior, a plurality of proximal tether memory metal strips, a row of proximal cells defined by a plurality of proximal cell memory metal strips, each proximal cell comprising a proximal crown located at the proximal end of the proximal cell and pointing generally in the proximal direction, each proximal tether memory metal strip having a proximal end attached to said proximal tube, a distal end attached to a crown of a proximal cell and a length extending from said proximal end to said distal end, a row of distal crowns located distal to said proximal cells pointing in the distal direction, and further wherein the number of distal crowns in said row is twice the number of proximal crowns attached to said proximal tether memory metal strips, and a distal tube located at said distal end of said distal basket,
said distal basket having
a relaxed state wherein said distal basket has a first height and
a collapsed state wherein said distal basket has a second height, said second height less than said first height, and
a catheter having an interior, a proximal end leading to said interior and a distal end leading to said interior, said catheter comprised of a biocompatible material and configured to envelope said distal body when said distal basket is in said collapsed state.

Optionally, said proximal tether memory metal strips rotate about said pull wire longitudinal axis such that a distal end of a proximal tether memory metal strip is located between about 90 and about 270 degrees relative to said proximal end of the same proximal tether memory metal strip. Optionally, said proximal tether memory metal strips and said proximal cell memory metal strips each have a thickness and further wherein said thickness of said proximal tether memory metal strips is between about 100 to about 175 percent of the thickness of the proximal cell memory metal strips. Optionally, the length of said proximal tether memory metal strips is about 10 mm to about 20 mm in the relaxed (and the length of the remainder of the basket is about 10 to about 20 mm in the relaxed state so that the total basket length is between about 20 to about 40 mm in the relaxed state). Optionally, said distal end of said pull wire is attached to said proximal tube. Some or all of the proximal crowns of said proximal cells may be attached to a proximal tether memory metal strip. Optionally, said distal basket further comprises a row of strut memory metal strips, each strut memory metal strip having a proximal end attached to a distal crown of a proximal cell and a distal end attached to a proximal crown of a distal cell. Optionally, the distal basket comprises between two and four proximal tether memory metal strips. Optionally, said proximal tether memory metal strips are integral with said proximal tube. Optionally, said distal body further comprises a lead wire extending distally from said distal tube. Optionally, said distal tube, said proximal tube, and said basket are comprised of a nitinol having the same material composition. Optionally, said distal body further comprises an x-ray marker. Optionally, said proximal and said distal tubes are generally cylindrical in shape and each has an outer diameter and an inner diameter, the inner diameter forming the apertures of the proximal and distal tubes and further wherein the outer diameters of the proximal and distal tubes are substantially the same size and further wherein the inner diameters of the proximal and distal tubes are substantially the same size. Optionally, the outer diameters of the proximal and distal tubes are from about 0.28 mm (0.011 inches) to about 1.37 mm (0.054 inches), and further wherein the inner diameters of the proximal and distal tubes are from about 0.20 mm (0.008 inches) to about 1.30 mm (0.051 inches). Optionally, the pull wire is generally cylindrical and further wherein the diameter of the pull wire is between about 0.20 mm (0.008 inches) and about 1.30 mm (0.051 inches). Optionally, the first height is between about 2 millimeters and about 8 millimeters.

The present disclosure also provides an exemplary method of removing an object from an interior lumen of an animal, said lumen having an interior wall forming said lumen, the exemplary method comprising the steps of:
a) providing the system described above;
b) positioning the system in said lumen, said basket located in said catheter in said collapsed state;
c) deploying said distal basket from said distal end of said catheter so that said proximal crowns of said proximal cells are distal to said obstruction;
d) allowing said distal basket to move to said relaxed state;
e) moving said distal basket over said obstruction; and
f) removing said distal basket and said obstruction from said lumen.

Optionally, said interior lumen is an intracranial artery and said obstruction is a blood clot.

In other embodiments, the system includes:
a pull wire having a proximal end, a distal end and a pull wire longitudinal axis extending from said proximal end to said distal end;
a proximal basket attached to said pull wire, said proximal basket comprising an interior, an exterior, a proximal end, a distal end, a proximal basket length extending from said proximal basket proximal end to said distal end, a proximal basket height perpendicular to said proximal basket length and said pull wire longitudinal axis, a proximal tube located at said proximal end of the proximal basket, said proximal tube comprising a hollow interior, a plurality of rows of cells, each cell defined by a plurality of memory metal strips, each cell comprising a proximal crown located at the proximal end of the proximal cell and pointing generally in the proximal direction and a distal crown located at the distal end of the proximal cell and pointing generally in the distal direction,
a distal basket attached to said pull wire, said distal basket comprising an interior, an exterior, a proximal end, a distal end, a distal basket length extending from said distal basket proximal end to said distal end, a distal basket height perpendicular to said distal basket length and said pull wire longitudinal axis, a distal tube located at said distal end of the distal basket, said distal tube comprising a distal tube aperture, a plurality of rows of cells, each cell defined by a plurality of memory metal strips, each cell comprising a proximal crown located at the proximal end of the proximal cell and pointing generally in the proximal direction and a distal crown located at the distal end of the proximal cell and pointing generally in the distal direction,
a plurality of tether memory metal strips, each tether memory metal strip having a proximal end attached to a distal crown of a cell located at the distal end of said proximal basket and a distal end attached to a proximal crown of a cell located at the proximal end of said distal basket,
said proximal basket having
a relaxed state wherein said proximal basket has a first height and
a collapsed state wherein said proximal basket has a second height, said second height less than said first height and said second width less than said first width,
said distal basket having
a relaxed state wherein said distal basket has a first height and a first width and
a collapsed state wherein said distal basket has a second height and a second width, said second height less than said first height, and
a catheter having an interior, a proximal end leading to said interior and a distal end leading to said interior, said catheter comprised of a biocompatible material and configured to envelope said distal and said proximal basket when said baskets are in said collapsed state.

Optionally, said tether memory metal strips rotate about said pull wire longitudinal axis such that a distal end of a tether memory metal strip is located between about 90 and about 270 degrees relative to said proximal end of the same proximal tether memory metal strip.

### BRIEF DESCRIPTION OF THE DRAWINGS

FIG. 1A illustrates a side, elevation view of a memory metal tube prior to being cut by a laser.
FIG. 1B illustrates a side, elevation view of the memory metal tube of FIG. 1A being cut by a laser.
FIG. 2A illustrates a side, elevation view of the memory metal tube of FIG. 1B after being cut by a laser; in FIG. 2A, the tube is shown as though it were flat for purposes of illustrating the cut pattern only.
FIG. 2B illustrates a side, perspective view of the memory metal tube of FIG. 1B after being cut by a laser.
FIG. 2C illustrates another side, perspective view of the memory metal tube of FIG. 1B after being cut by a laser; in FIG. 2C, the tube is rotated as compared to FIG. 2B.
FIGs. 3A-3H illustrate an exemplary method of manufacturing a distal body using the laser cut memory metal tube of FIGs. 1 and 2; in FIGs. 3A-3H, the basket portion of the distal body is not shown for simplicity of illustration.
FIGs. 4A-4D illustrate the welding steps of the exemplary method of manufacturing shown in FIG. 3; in FIGs. 4A-4D, the basket portion of the distal body is not shown for simplicity of illustration.
FIGs. 5 and 6 illustrate different locations that connector strips may be welded to the proximal memory metal strips.
FIG. 7 illustrates a side, elevation view of a catheter and the distal body of FIG. 6.
FIG. 8 illustrates a side, elevation view of a deployable system of one embodiment of the present invention being used to capture a blood clot; in FIG. 8, the basket portion of the distal body is not shown for simplicity of illustration.
FIG. 9 illustrates a side, elevation view of a claw of one embodiment of the present invention being closed by a claw actuator tube; in FIG. 9, the basket portion of the distal body is not shown for simplicity of illustration.
FIG. 10 illustrates a side, elevation view of a deployable system of one embodiment of the present invention being used to capture a blood clot; in FIG. 10, the basket portion of the distal body is not shown for simplicity of illustration.
FIG. 11 illustrates a first, perspective view of a distal body of an alternate embodiment of the present invention; the distal body is in what is referred to herein as "Orientation 1".
FIG. 12A illustrates a second, perspective view of the distal body of FIG. 11; the distal body is in what is referred to herein as "Orientation 2".
FIG. 12B illustrates a proximal, elevation view of the proximal strips of the distal body of FIG. 11.
FIG. 13 illustrates a close-up, perspective view of two unattached distal-pointing crowns of the distal body of FIG. 11.
FIG. 14A illustrates a native memory metal tube used to manufacture the distal body of FIG. 11; the native tube has been rolled out flat and the lines in the tube indicate where the tube has been cut by a laser.
FIG. 14B illustrates a first, perspective view of the distal body manufactured from the native tube of FIG. 14A; the distal body is in Orientation 1.
FIG. 14C illustrates a second, perspective view of the distal body manufactured from the native tube of FIG. 14A; the distal body is in Orientation 2.
FIGs. 15A-G illustrate stepwise use of the distal body of FIG. 11 in retrieving a soft clot; the distal body is in Orientation 1.
FIGs. 16A-H illustrate stepwise use of the distal body of FIG. 11 in retrieving a hard clot; the distal body is in Orientation 1.
FIGs. 17A-G illustrate stepwise use of the distal body of FIG. 11 in retrieving a soft clot; the distal body is in Orientation 2.
FIGs. 18A-G illustrate stepwise use of the distal body of FIG. 11 in retrieving a hard clot; the distal body is in Orientation 2.
FIGs. 19A-N illustrate stepwise use of the distal body of FIG. 11 in retrieving a deformable, cohesive adherent clot; the distal body is in Orientation 2.
FIG. 20A illustrates a view of a native memory metal tube used to manufacture a distal body of yet another embodiment of the present invention; the native tube has been rolled out flat, the lines in the tube indicate where the tube has been cut by a laser, and the distal body of FIGs. 20A-20C is slightly shorter than the distal body of FIGs. 11-19 and is meant for use in tortuous blood vessels.
FIG. 20B illustrates a first, perspective view of the distal body manufactured from the native tube of FIG. 20A; the distal body is in Orientation 1.
FIG. 20C illustrates a second, perspective view of the distal body manufactured from the native tube of FIG. 20A; the distal body is in Orientation 2.
FIG. 21 shows a perspective view of a clot retrieval system that includes the distal body of FIGs. 20B-C being delivered in a blood vessel using a delivery catheter.
FIG. 22 shows a perspective view of the distal body of FIG. 21, after deployment of the distal body and retraction of the delivery catheter, in a blood vessel.
FIG. 23 shows a perspective view of the distal body of FIG. 21; as compared to FIG. 22, the distal body has been moved proximally and tension has been exerted on the pull wire.
FIG. 24 shows a perspective view of a suction catheter that is being delivered over the pull wire of the system of FIG. 21.
FIG. 25 shows a perspective view of the distal end of the suction catheter of FIG. 24 being pushed into a clot; a syringe is sucking the clot to the suction catheter because the user has pulled back on the lever of the syringe.
FIG. 26 shows a perspective view of the distal end of the suction catheter of FIG. 24 being pushed into a clot; in FIG. 26, the user has locked the syringe lever at the desired volume.
FIG. 27 shows a perspective view of the system of FIG. 24; in FIG. 27, the suction catheter has partially sucked the distal body and clot into the suction catheter.
FIG. 28 shows a perspective view of the system of FIG. 24; in FIG. 28, the suction catheter has completely sucked the distal body and clot into the suction catheter.
FIG. 29 shows a perspective view of the system of FIG. 24; the system, and captured clot, is being removed proximally from the vessel.
FIG. 30A illustrates a front, perspective view of a system of another embodiment of the present invention that includes a delivery catheter, a coaxial tube slideable along a pull wire, and proximal strips that extend from the distal end of the coaxial tube and are attached to a distal basket; in FIG. 30A, the distal basket is in the relaxed state.
FIG. 30B illustrates a front, perspective view of the system of FIG. 30A; in FIG. 30B, the system is in a partially collapsed state due to distal movement of the catheter.
FIG. 30C illustrates a proximal, elevation view of the proximal strips of the system of FIG. 30A.
FIG. 30D illustrates a proximal, elevation view of an alternate embodiment of FIGs. 30A and 30B that includes two proximal strips.
FIG. 30E illustrates a proximal, elevation view of an alternate embodiment of FIGs. 30A and 30B that includes four proximal strips.
FIG. 31A illustrates a front, perspective view of the system of FIG. 30A; in FIG. 31A, the system is between the proximal collapsed state and the relaxed state.
FIG. 31B illustrates a front, perspective view of the system of FIG. 30A; in FIG. 31A, the system is in the distal collapsed state.
FIG. 32A-F illustrates a front, perspective view of the system of FIG. 30A and stepwise use of the system in retrieving a clot in a human intracranial artery.
FIG. 33 illustrates a front, perspective view of an alternate embodiment of the system of FIGs. 31-32 in which the proximal ends of the proximal strips are attached to the distal end of the coaxial sheath.
FIG. 34 illustrates a front, perspective view of an alternate embodiment of the system in which the coaxial sheath is a braided catheter comprised of a plurality of braids and further wherein the distal segment of each braid forms a proximal strip.
FIG. 35A-C illustrate a front, perspective view of an embodiment of the system of FIGs. 30-34 in which the proximal strips cover the proximal tip of the proximal crowns; in particular, FIG. 35A is an exploded view, FIG. 35B shows the proximal strip attached to the proximal crown via a loop and an eyelet, and FIG. 35C shows how the proximal strips bend backwards to cover the proximal tips when the distal body is in the distal collapsed state.
FIGS. 36A-36D illustrate a side, perspective view of a stepwise sequence of making an embodiment of the basket system of the present invention.
FIGs. 37A-37B illustrate a side, perspective view of stepwise deployment and use of a basket system with proximal tether memory metal strips that are about the same length as the rest of the basket (as measured from the proximal-most crown to the distal tube).
FIGs. 38A-38E illustrate a side, perspective view of stepwise deployment and use of the basket system of FIGs. 37A-37B in a blood vessel to retrieve a clot.
FIG. 39A illustrates a side, perspective view of the basket system of FIGs. 37A and 37B; as shown, all proximal crowns of the proximal cells are attached to a proximal tether memory metal strip.
FIG. 39B illustrates an alternative embodiment in which one proximal crown of a proximal cell is not attached to a proximal tether memory metal strip.
FIG. 40 illustrates a side, perspective view of a basket system with relatively thick proximal tether memory metal strips; in this FIG. 40, as shown, the proximal tether memory metal strips are thicker than the memory metal strips forming the proximal-most crown.
FIG. 41 illustrates a side, perspective view of a basket system with a proximal basket and a distal basket.
FIG. 42 illustrates a side, perspective view of a basket system with a proximal basket and a distal basket in which the proximal tether memory metal strips rotate 180 degrees about both the longitudinal axis of the proximal tether memory metal strips and about the longitudinal axis of the pull wire.
FIGs. 43A-43B illustrate a side, perspective view of a basket system in which the proximal tether memory metal strips rotate 90 degrees about both the longitudinal axis of the proximal tether memory metal strips and about the longitudinal axis of the pull wire.
FIG. 43C illustrates a front, elevation view of the basket system of FIGs. 43A-43B.
FIGs. 43D and 43E illustrate a front, elevation view and a side, perspective view of a basket system in which the proximal tether memory metal strips rotate 180 degrees about both the longitudinal axis of the proximal tether memory metal strips and about the longitudinal axis of the pull wire.
FIGs. 44A-44E illustrate a side, perspective view of stepwise deployment and use of a basket system with a proximal basket and a distal basket in a blood vessel to retrieve a clot.
FIGS. 45A-45D illustrate a side, perspective view of a stepwise sequence of making an embodiment of the basket system of the present invention.
FIGs. 46A-46E illustrate a side, perspective view of stepwise deployment and use of a basket system with relatively thin and short proximal tether memory metal strips.
FIGs. 47A-47H illustrate a side, perspective view of stepwise deployment and use of the basket system of FIGs. 46A-46E in a blood vessel to retrieve a clot.
FIGs. 48A-48B illustrate a side, perspective view of stepwise deployment and use of a basket system with relatively thick and short proximal tether memory metal strips.
FIGs. 49A-49C illustrate a side, perspective view of stepwise deployment and use of a basket system with three relatively thin and short proximal tether memory metal strips; the system is deployed in a blood vessel to retrieve a clot.
FIG. 50A illustrates a side, perspective view of a basket system with relatively thin and short proximal tether memory metal strips; in FIG. 50A, all proximal crowns of the proximal cells are attached to a proximal tether memory metal strip.
FIG. 50B illustrates a side, perspective view of a basket system with relatively thin and short proximal tether memory metal strips; in FIG. 50B, one proximal crowns of a proximal cell is not attached to a proximal tether memory metal strip.
FIG. 50C illustrates a front view of a basket system with two proximal tether memory metal strips.
FIG. 50D illustrates a front view of a basket system with three proximal tether memory metal strips.
FIG. 50E illustrates a front view of a basket system with four proximal tether memory metal strips.
FIG. 51 illustrates a side, perspective view of a basket system with relatively thin and short proximal tether memory metal strips; in this FIG. 51, as shown, the proximal tether memory metal strips are not as thick as the memory metal strips forming the proximal-most crown; further, the thickness of the memory metal strips gradually decreases from the proximal-most crown along the basket length to the distal hub.
FIG. 52 illustrates a side, perspective view of a basket system with relatively thin, short proximal tether memory metal strips.
FIGs. 53A-53C illustrate a side, perspective view of stepwise deployment and use of a basket system with relatively long and thin proximal tether memory metal strips; the system is used in a blood vessel to retrieve a clot.
FIGs. 54A-54C illustrate a side, perspective view of a basket system with a proximal basket connected to a distal basket by proximal tether memory metal strips.
FIGs. 55A-55B illustrate a side, perspective view of a basket system in which the proximal tether memory metal strips rotate 90 degrees about both the longitudinal axis of the proximal tether memory metal strips and about the longitudinal axis of the pull wire.
FIG. 55C illustrates a front, elevation view of the basket system of FIGs. 55A-55B.
FIGs. 55D and 55E illustrate a front, elevation view and a side, perspective view of a basket system in which the proximal tether memory metal strips rotate 180 degrees about both the longitudinal axis of the proximal tether memory metal strips and about the longitudinal axis of the pull wire.
FIG. 56 illustrates a side, perspective view of a basket system with relatively thick and short proximal tether memory metal strips.
FIG. 57A-F illustrates a side perspective view of deployment a basket system in which the proximal tether memory metal strips are thicker than the memory metal strips forming the proximal cells of the distal basket.
FIGs. 58A-58B illustrates a side perspective view of a basket system with relatively long cords, instead of proximal tether memory metal strips.
FIGs. 59A-59B illustrates a side perspective view of a basket system with relatively short cords, instead of proximal tether memory metal strips.
FIGs. 60A-F illustrate a perspective view of deployment of the basket system of FIGs. 59A-59B.
FIG. 61 illustrates a side perspective view of a basket system with cords and proximal tether memory metal strips.
FIGs. 62A- 62C illustrate a perspective view of deployment of the basket system of FIG. 61.
FIG. 63 illustrates a right side perspective view of a mandrel used to prepare unattached distal-pointing crowns that curve radially toward the basket interior.
FIG. 64 illustrates a right side elevation view of the mandrel of FIG. 63.

### DETAILED DESCRIPTION

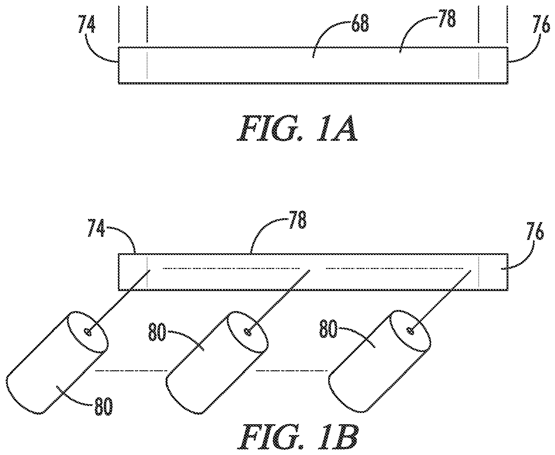

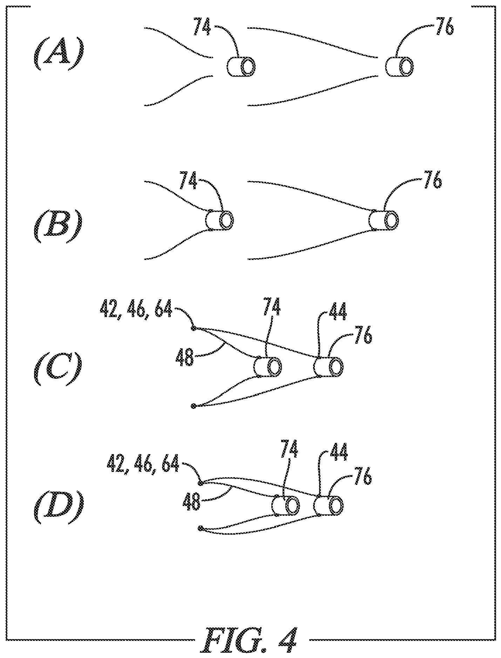

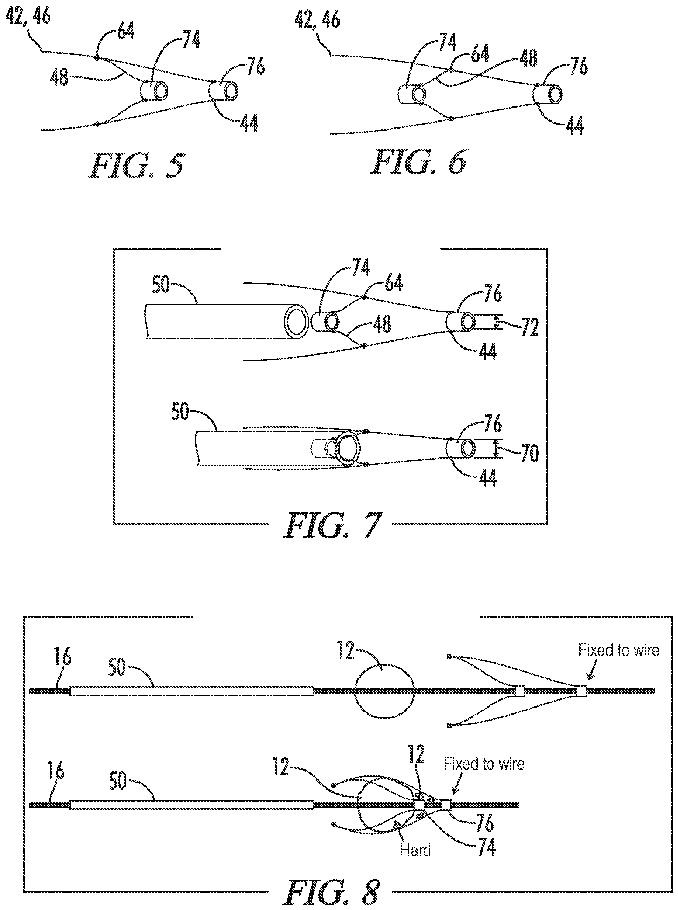

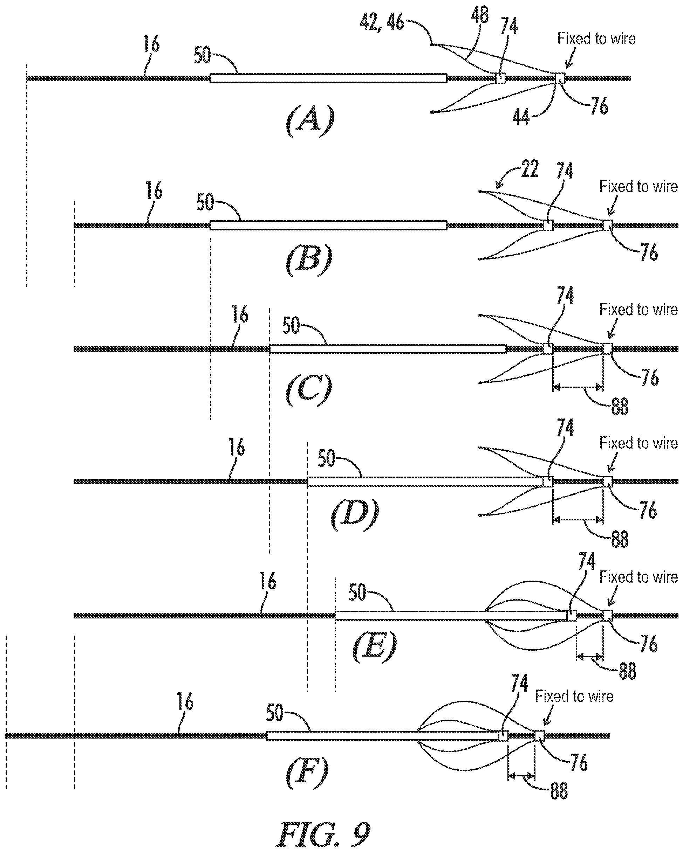

With reference to FIGs. 1-10, the present disclosure provides a deployable system, generally designated by the numeral **10**, for removing an obstruction such as a blood clot **12** or other object from a blood vessel **14** or other interior lumen of an animal. In addition to a blood clot **12**, the obstruction may be, for example, extruded coils during aneurysm treatment, intravascular embolic material such as onyx or other obstructions requiring mechanical intravascular removal from small distal vessels. In the drawings, not all reference numbers are included in each drawing for the sake of clarity.

Referring further to FIGs. 1-10, the deployable system **10** includes a pull wire **16** that has a proximal end (not shown) and a distal end **20.** Optionally, the diameter of the pull wire is between about 0.20 mm (0.008 inches) and about 1.30 mm (0.051 inches). Preferably, the pull wire **16** is comprised of a biocompatible metallic material.

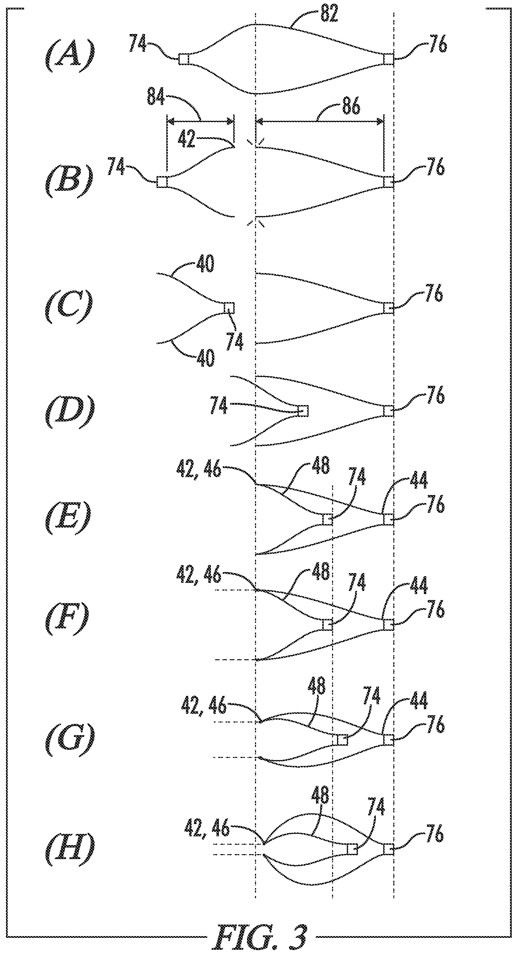

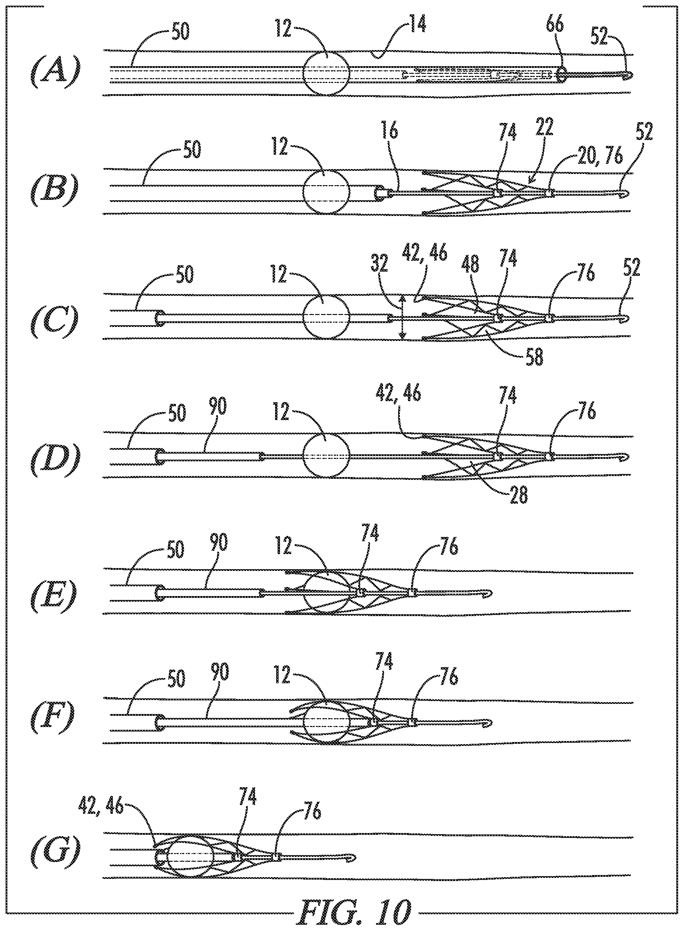

The system **10** further includes a distal body **22**, which is attached to the pull wire **16.** The distal body **22** has a proximal end **24**, a distal end **26**, an interior **28**, and an exterior **30.** The distal body **22** has a collapsed state, wherein the distal body **22** has a first height and width and is configured to fit into a catheter **50** (see FIG. 10A), and a relaxed state wherein the distal body **22** has a different height **32** and width and is configured to expand to about the height and width of a human blood vessel **14** when the distal body **22** is deployed from the catheter **50** (see FIGS. 10B-G). The distal body **22** further includes a proximal hub **74** and a distal hub **76** that is located distal relative to the proximal hub **74.** In some embodiments, the distal body **22** includes a plurality of strips **40** comprised of a memory metal (e.g., a memory metal alloy such as nitinol) that form the proximal end **24** of the distal body **22.** Optionally, the proximal memory metal strips **40** each have a distal end **44** and a proximal end **42** that forms an openable and closeable claw **46.** Optionally, the proximal memory metal strips **40** are attached to the proximal hub **74** through connector memory metal strips **48.** In such embodiments, the proximal hub **74** may be slideable along at least a segment of the pull wire **16**, in contrast to the distal hub **76**, which is optionally fixed to the pull wire **16** and not slideable along the pull wire **16.** Moving the proximal hub **74** distally and closer to the distal hub **76** (i.e., shortening the distance **88** between the proximal hub **74** and distal hub **76** by moving the proximal hub **74** distally while keeping the distal hub **76** stationary) exerts tension on the connector memory metal strips **48** and, in turn, the proximal memory metal strips **40.** This tension, in turn, causes the proximal ends **42** of the proximal memory metal strips **40** to move radially toward each other and the pull wire **16.** As the proximal ends **42** of the proximal memory metal strips **40** move radially toward each other and the pull wire **16**, the claw **46** (formed by the proximal memory metal strips **40**) is brought from the open position to at least a partially closed position, which in turn, separates the obstruction **12** from the wall of the human lumen **14** and captures the obstruction **12.** See FIG. 3H, FIG. 8, FIG. 9F, and FIG. 10F and 10G. Conversely, preferably, movement of the proximal hub **74** proximally and away from the distal hub **76** (i.e., increasing the distance **88** between the hubs **74** and **76**) releases the tension in the proximal memory metal strips **40**, which in turn, causes the proximal ends **42** of the proximal memory metal strips **40** to move away from each other and the pull wire **16**, opening the claw **46.** The claw **46** and proximal hub **74** form several functions. First, as described, closing of the claw **46** captures the obstruction **12.** Second, closing the claw **46** retracts the claw **46** from the wall of the lumen **14** so that the claw **46** does not scrape against (and damage) the lumen wall while capturing the obstruction **12.** Third, closing the claw **46** reduces the height and width of the distal body **22**, which allows the distal body **22** to be re-sheathed in the catheter **50**, which may be desired, for example, if the operator seeks to re-deploy the distal body **22** in another location in the body (which may be the case if the operator originally deploys the distal body **22** in the wrong location in the lumen **14**). For purposes of the present invention, "closing the claw" embraces both partially closing the claw **46** (where the proximal ends **42** of the proximal memory metal strips **40** do not contact the pull wire **16**) and fully closing the claw **46** (where the proximal ends **42** contact the pull wire **16**).

The claw **46** may be comprised of any number of proximal memory metal strips **40.** Preferably, however, between 2 and 4 proximal memory metal strips **40** comprise the claw **46** (it being understood that the connector strips **48**, if present, merely serve to tether the claw **46** to the proximal hub **74**). Preferably, the proximal memory metal strips **40** have a length of between about 10 and about 60 millimeters. The proximal memory metal strips **40** can be thought of as arms of the claw **46.**

In some embodiments, the connector strips **48** are integral with the proximal hub **74** (i.e., formed from the same piece of memory metal). In other embodiments, the proximal hub **74** may be welded to the connector strips **48.** Optionally, in the relaxed state, the proximal memory metal strips **42** are distributed substantially evenly about a perimeter of the distal body **22.**

Optionally, the distal body **22** includes a lead wire **52** extending distally from the distal body **22.** Optionally, the lead wire **52** extends distally from the distal hub **76.** If present, the lead wire **52** may be used to facilitate movement of the system **10** in the lumen **14.**

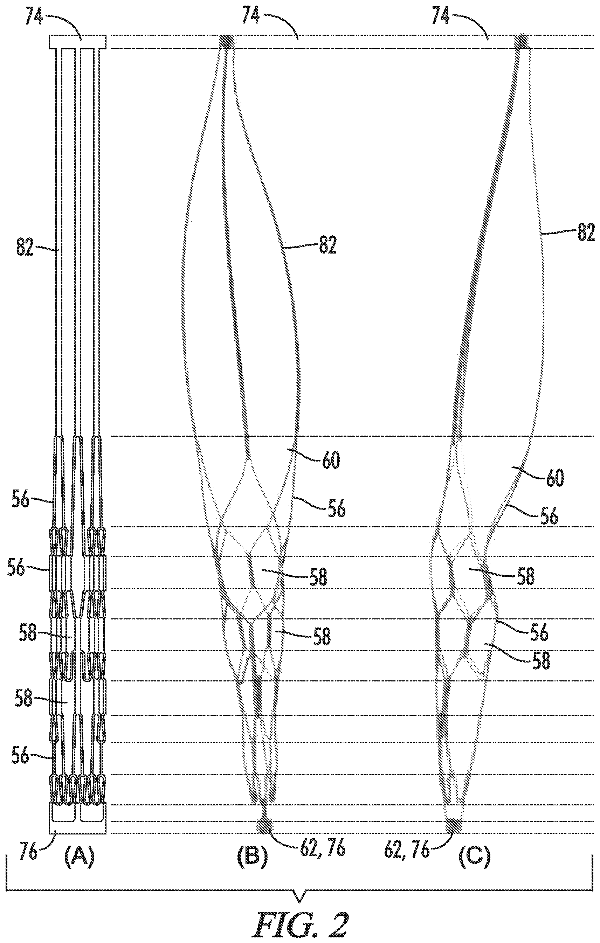

Optionally, the distal body **22** includes a basket **54** distal to the proximal memory metal strips **40**, the basket **54** comprised of a plurality of memory metal strips **56** distal relative to the proximal memory metal strips **40.** The distal memory metal strips **56** may, for example, form a basket **54** with a plurality of mesh openings **58.** Optionally, the size of the mesh openings **58** in the basket **54** when the distal body **22** is in its relaxed state is less (preferably significantly less) than the diameter of an average-sized ischemic blood clot **12** so that the blood clot **12** does not escape from the distal basket **54** after being captured by the distal body **22.** Optionally, the basket **54** has an open proximal end **60** and a substantially closed distal end **62**, which is formed by distal tube **76.** Optionally, the distal and proximal hubs **74** and **76** and the distal basket **54** are comprised of a nitinol having the same material composition. Optionally, the size of the mesh openings **58** decreases from the proximal end **60** of the basket **54** to the distal end **62.** The distal basket **54** is best seen in FIG. 2 and can be comprised of a different number of cell patterns. The distal basket **54** is not shown in FIGs. 3-10 for ease of illustrating the other components in the system **10.**

Optionally, the proximal hub **74** and the distal hub **76** are cylindrical tubes comprising substantially circular apertures that span the length of the hubs **74** and **76** and the hubs **74** and **76** have approximately the same inner diameter **72** and the same outer diameter **70.** Preferably, the inner diameter **72** is at least slightly larger than the diameter of the pull wire **16** so that the pull wire **16** can slide through the proximal hub **74.** In some embodiments, the outer diameters **70** of the proximal and distal hubs **74** and **76** may be from about 0.28 mm (0.011 inches) to about 1.37 mm (0.054 inches) and the inner diameters **72** of the proximal and distal hubs **74** and **76** may be from about 0.20 mm (0.008 inches) to about 1.30 mm (0.051 inches).

Optionally, the distal body **22** further comprises an x-ray marker **64** that is more visible under x-ray as compared to the proximal memory metal strips **40** when the distal body **22** is located in a cranial blood vessel inside the body of a human and the x-ray is taken from outside the human's body. If the connector strips **48** are welded to the proximal memory metal strips **40**, the x-ray markers **64** may be, for example, located at the welding site. In some cases, the increased thickness at the welding site may in of itself comprise the x-ray marker **64.** Preferably, the x-ray marker **64** is comprised of a radiopaque material. Some examples of radiopaque materials can include, but are not limited to, gold, platinum, palladium, tantalum, tungsten alloy, polymer material loaded with radiopaque filler, and the like. Preferably, the proximal memory metal strips **40** are comprised of nitinol and the x-ray marker **64** is comprised of a material having a density greater than the nitinol.

A catheter **50** with an open proximal end (not shown) and an open distal end **66** initially envelopes the system **10.** As used herein, the term "catheter" generally refers to any suitable tube through which the system **10** can be deployed. Preferably, the catheter **50** is sterile and comprised of a biocompatible material (i.e., a material that does not irritate the human body during the course of a 45 minute operation that involves using the system **10** to remove a clot **12** from an intracranial blood vessel **14**). The catheter **50** can be any suitable shape, including but not limited to generally cylindrical. Preferably, the catheter **50** is a microcatheter. For purposes of the present invention, when it is said that the catheter **50** envelopes the system **10**, it will be understood that the catheter **50** envelopes at least one component of the system **10** (preferably, the distal body **22**, the lead wire **52**, and the pull wire **16**). In some embodiments, the catheter **50** is about 0.833 mm (2.5 French) in diameter. Optionally, the catheter **50** is delivered to the region of the lumen **14** that has the obstruction **12** as follows: a guide wire is delivered to the obstruction region past the obstruction **12**; the catheter **50** is delivered over the guide wire; the guide wire is removed; and the system **10** is delivered with its pull wire **16** and lead wire **52** through the catheter **50.** Optionally, the pull wire **16** is used to push the system **10** through the catheter **50** as well as to retrieve the distal body **22** after capturing the obstruction **14** as described below. The system **10** may utilize a plurality of catheters **50**, such as, for example, a wider catheter that travels to the brain and a very flexible, smaller diameter microcatheter that is delivered from the first catheter and travels through the small arteries of the brain. Preferably, the catheter **50** is comprised of a biocompatible, polymeric material (i.e., one or more polymeric materials such as silicone, PVC, latex rubber or braided nylon).

In the relaxed, opened-claw state, the distal body **22** or optionally just the distal basket **54** has a tapered shape (e.g., substantially conical or bullet in shape) so that the distal body **22** or just the distal basket **54** tapers from the distal body **22** or the distal basket's **54** proximal end to the distal end.

The proximal end of the system **10** is shown at the left end of FIGs. 1 and 3-10 and the distal end of the system **10** is shown at the right end of FIGs. 1 and 3-10 because a principal use of the system **10** is to remove a blood clot **12** from a human intracranial artery **14**, in which case the system **10** generally will enter the artery **14** at its proximal end by the surgeon entering the patient's body near the groin and pushing the catheter **50** towards the brain. The diameter of human arteries **14** generally decrease from their proximal end to their distal end. However, when used in other types of lumens, the distal body **22** may be located proximally relative to the catheter **50** as the term proximally and distally are used in that lumen.

The surgeon may deploy the distal body **22** by, for example, moving the catheter **50** proximally so as to unsheathe the distal body **22** or by pushing the distal body **22** out of the catheter **50.**

Use of the system **10** will now be described to remove a blood clot **12** from an intracranial artery **14** of a human ischemic stroke patient, however, it will be appreciated that the system **10** may be used to remove other objects from other interior lumens.

A catheter **50**, which contains the collapsed distal body **22** is positioned in the lumen **14** distal to the clot **12.** See FIG. 10A.

The distal body **22** is deployed from the catheter **50** and the height and width of the distal body **22** expand to about the height and width of the blood vessel **14.** See FIG. 10B.

The catheter **50** is pulled proximally and a claw-actuator tube **90** is deployed into the blood vessel **14.** See FIG. 10C.

The distal body **22** is moved proximally so that the clot **12** is located in the interior **28** of the distal body **22.** See FIGs. 10D and 10E.

The claw-actuator tube **90** is moved distally, which pushes the proximal hub **74** distally so that the distance **88** between the proximal hub **74** and the distal hub **76** (which is fixed to the pull wire **16** and kept stationary) decreases. Distal movement of the proximal hub **74** exerts tension on the connector and proximal memory metal strips **40** and **48**, which in turn, closes the claw **46.** See FIG. 10F. (The claw actuator tube **90** should float on the pull wire **16** - i.e., have an aperture extending the tube's length that has a diameter larger than the diameter of the pull wire **16** - and the aperture of the claw actuator tube **90** should be smaller than the diameter of the proximal hub **74** so that the claw actuator tube **90** pushes the proximal hub **74**).

The system **10** is withdrawn proximally and removed from the body. See FIG. 10G.

To test the efficacy of the system **10**, a distal body **22** with a distal basket **54**, proximal and distal hubs **74** and **76**, and a claw **46** comprised of three proximal memory metal strips **42** was tested in a flow model that included a tube and a moist cotton ball located in the tube. The cotton ball was used to simulate a blood clot. The system **10** was deployed distal to the cotton ball. The claw **46** was closed by moving the proximal hub **74** distally to capture the cotton ball. The system **10** and cotton ball were withdrawn proximally in the tube.

In some examples, the distal body **22** is prepared by a process that includes one or more of the following steps, as illustrated in FIGs. 1-4
a) providing a single tube **68** comprised of a memory metal such as nitinol, the single tube **68** having an exterior, a substantially hollow interior, a wall separating the exterior from the substantially hollow interior, an open proximal end **74**, an open distal end **76**, a middle portion **78** between the open proximal end **74** and the open distal end **76** (see FIG. 1A);
b) cutting the wall of the middle portion **78** with a laser **80** (see FIG. IB);
c) removing the pieces of the middle portion **78** cut by the laser **80** to form a proximal tube **74**, a distal tube **76** and a middle portion **78** comprising a plurality of memory metal strips **82** attached to the proximal tube **74**;
d) altering the shape of the middle portion **78** using a mandrel and allowing the middle portion **78** to expand relative to the distal tube **76** and proximal tube **74** to form the distal basket **54**;
e) quenching the middle portion **78** at room temperature;
f) removing the mandrel from the middle portion **78** (see FIGs. 2 and 3A);
g) mechanically or chemically electropolishing the middle portion **78** to remove oxides;
h) cutting the memory metal strips **82** to form a first segment **84** comprising the proximal tube **74** and a proximal segment of the memory metal strips **82** and a second segment **86** comprising the distal tube **76** and a distal segment of the memory metal strips **82** (see FIG. 3B); and
i) joining the proximal segments to the distal segments such that the distal segments form the proximal end **24** of the distal body **22**, such that the proximal tube **74** is located inside the interior **28** of the distal body **22**, and such the proximal tube **74** is located distal relative to the distal body proximal end **24** (see FIGs. 3C-3E).

In some examples, the exemplary method further includes placing the pull wire **16** through the proximal tube **74** so that the proximal tube **74** is slideable along at least a segment of the pull wire **16.**

In some examples, the exemplary method further includes attaching the pull wire **16** to the distal tube **76** so that the distal tube **76** is not slideable along the pull wire **16** but instead the distal tube **76** moves with the pull wire **16.**

In some examples , after step i, the proximal end **24** of the distal body **22** forms a claw **46** comprised of between 2 to 4 proximal memory metal strips **40**, the claw proximal memory metal strips **40** configured to move towards each other and the pull wire **16** by moving the proximal tube **74** distally and toward the distal tube **76** (i.e., decreasing the distance **88** between the tubes **74** and **76**) and the claw memory metal strips **40** configured to move away from each other and away from the pull wire (i.e., increasing the distance **88** between the tubes **74** and **76**) by moving the proximal tube **76** proximally and away from the distal tube **76** (as described previously).

In some examples, the middle portion **78** is expanded by heating the mandrel and the middle portion **78** by, for example, placing the mandrel and the middle portion **78** in a fluidized sand bath at about 500°C for about 3 to about 7 minutes. As the middle portion **78** is heated, the heating causes the crystalline structure of the memory metal tube **68** to realign. Preferably, the mandrel is tapered (e.g., substantially conical or bullet in shape) so that the distal basket **54** formed from the middle portion **78** tapers from the proximal end **60** to the distal end **62.** Preferably, the proximal and distal ends of the tube **74** and **76** are not shape set by the mandrel and are not cut by the laser **80** so that the proximal and distal ends **74** and **76** do not change in shape and only slightly expand in size under heating and return to the size of the native tube **68** after the heat is removed. Preferably, the laser cuts are programmed via a computer. To ensure that the laser cuts only one surface of the tube wall at the time (and not the surface directly opposite the desired cutting surface), the laser **80** is preferably focused between the inner and outer diameter of the desired cutting surface and a coolant is passed through the memory metal tube **68** so that the laser **80** cools before reaching the surface directly opposite the desired cutting surface.

The portions of the wall not cut by the laser **80** create the distal basket **53**, proximal and distal tubes **74** and **76**, and memory metal strips **40**, **48** and **56**, as described.

Preferably, the memory metal selected for the native tube **68** has a heat of transformation below average human body temperature (37°C) so that the distal body **22** has sufficient spring and flexibility after deployment from the catheter **50** in the human blood vessel **14.**

In some embodiments, the native tube **68** (and hence the distal and proximal tubes **74** and **76**) have an outer diameter of less than about 1.33 mm (4 French), e.g., a diameter of about 0.33 mm to about 1.33 mm (about 1 to about 4 French). In some embodiments, the diameter of the pull wire **16** is between about 0.20 mm (0.008 inches) and about 1.30 mm (0.051 inches), as noted above, and in such embodiments, the diameter of the pull wire **16** may be approximately equal to the inner diameter **72** of the native nitinol tube **68.**

Without being bound by any particular theory, it is believed that manufacturing the distal body **22** from a single memory metal tube **68** provides ease of manufacturing and safety from mechanical failure and provides tensile strength necessary for the system **10** to remove hard thrombus **12** and other obstructions.

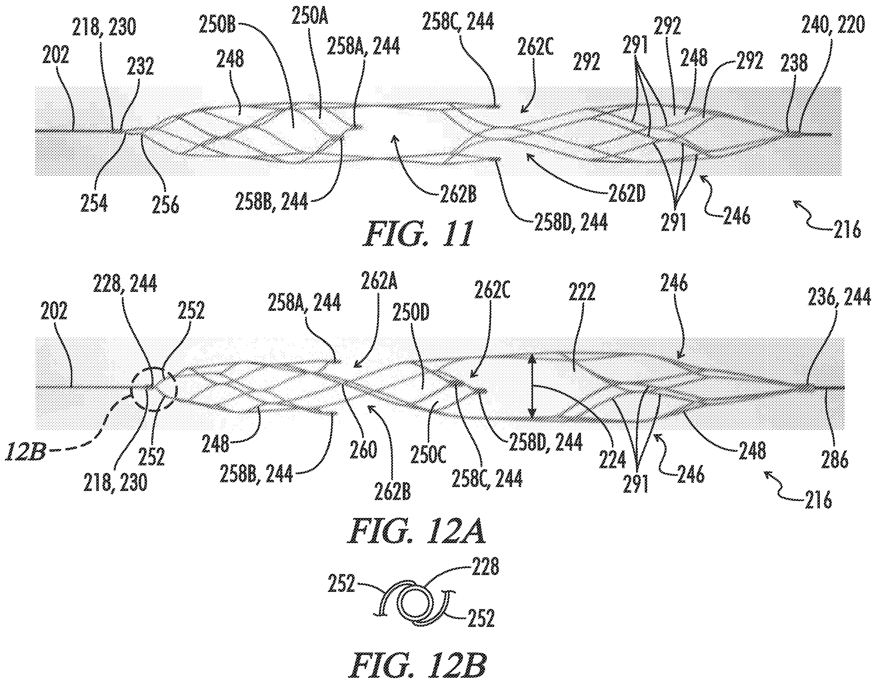

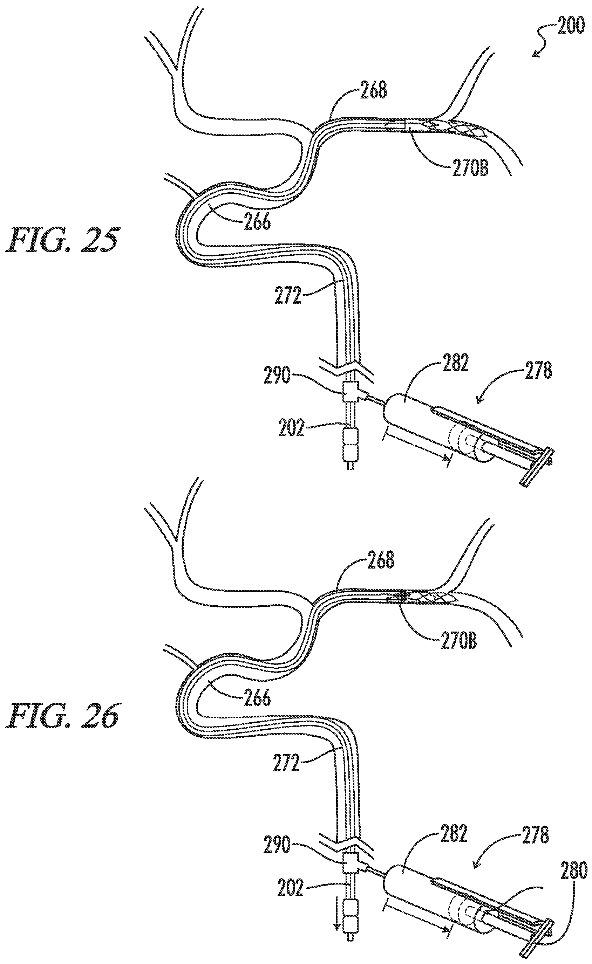

### The embodiments of Figures 11-29

Figures 11-29 illustrate an alternate embodiment **200** that includes one or more of the following additional features, as described below: twisting proximal strips/tethers **252**, unattached/free distal-pointing crowns **258** that optionally curve inward and have x-ray markers **244**, and enlarged openings/drop zones **262** in the basket **246** immediately distal to the unattached, distal-pointing crowns **258** that allow the obstruction or other object **270** to enter the distal basket interior **222.**

More specifically, as shown in FIGs. 11-29, the system **200** may include a pull wire **202** having a proximal end **204** and a distal end **206**, as described above, a distal body **216** attached to the pull wire **202**, the distal body **216** comprising an interior **222**, a proximal end **218**, a distal end **220**, a distal body length **226** extending from the proximal end **218** to the distal end **220**, a distal body height **224**, a proximal hub **228** (preferably in the form of a tube and which has a proximal end **230** and a distal end **232**) forming the proximal end **218** of the distal body **216**, a basket **246** comprised of a plurality of cells/openings **248** formed by a plurality of basket strips **291** that preferably are comprised of a memory metal, optionally a distal hub **236** that forms the distal end **220** of the basket **246** (preferably in the form of a tube that has a proximal end **238** and a distal end **240**), and a plurality of proximal strips **252** (preferably the proximal strips **252** are comprised of a memory metal), each proximal strip **252** having a proximal end **254** attached to the proximal hub/tube **228**, and a distal end **256** attached to a cell **248** (more specifically a proximal-pointing crown of a cell **248** located at the proximal end of the basket **246**), the basket comprising a basket interior **292**, the distal body **216** having a relaxed state wherein the distal body **216** has a first height and width, a collapsed state wherein the distal body **216** has a second height and width, the second height less than the first height, the second width less than the first width; and a delivery catheter **208** for delivering the distal body **216**, as described above, having an interior **210**, a proximal end **212** leading to the interior **210** and a distal end **214** leading to the interior **210**, the delivery catheter **208** comprised of a biocompatible (preferably polymeric) material and configured to envelope the distal body **216** when the distal body **216** is in the collapsed state. Optionally, the basket interior **292** is substantially hollow - i.e., unlike U.S. Patent Publication No. 2013/0345739, the basket interior **292** does not contain an inner elongate body. Optionally, instead of a distal hub **236**, the basket **246** includes an open distal end. Optionally, at least two cells **250** of the basket **246** comprise a proximal crown **260** pointing generally in the proximal direction and a distal crown **258** pointing generally in the distal direction, and the distal crowns **258** of the at least two cells **250** are not attached to another cell **248** of the basket **246.** In other words, the distal crowns **258** of at least two cells **250** are free floating and are not attached to any strip except for the strips forming part of the at least two cells **250**; such distal crowns **258** are referred to below as unattached, distal-pointing crowns **258.** Preferably, the distal tips of the unattached, distal-pointing crowns **258** terminate at an x-ray marker **244.** (Cells labeled with the numerals **250, 250A, 250B, 250C,** and **250D** refer to the at least two cells that include a proximal crown **260** pointing generally in the proximal direction and an unattached, distal-pointing crown **258,** cells labeled with the numerals **262, 262A, 262B, 262C,** and **262D** refer to the enlarged cells/drop zones adjacent to (preferably immediately distal to) an unattached, distal-pointing crown **258,** and cells designated with numeral **248** refer to generally the cells of the basket **246**). (When it is said that the enlarged cells/drop zones **262** are preferably immediately distal to an unattached, distal-pointing crown **258,** it will be understood that at least a portion of an enlarged cell/drop zone **262** is immediately distal to an unattached, distal-pointing crown **258,** and that a portion of the enlarged cell/drop zone **262** may be proximal to an unattached, distal-pointing crown **258,** as shown in FIGs. 11-12 due to the shape of the enlarged cells/drop zones **262**). It will be understood that part number **250** refers generally to one or more of the at least two cells, whereas part numbers **250A, 250B, 250C,** and **250D** refer to a specific one of the at least two cells. Similarly, it will be understood that part number **262** refers generally to one or more of the enlarged cells/drop zones, whereas part numbers **262A, 262B, 262C,** and **262D** refer to a specific one of the enlarged cells/drop zones. Similarly, it will be understood that part number **258** refers generally to one or more of the unattached, distal-pointing crowns, whereas part numbers **258A, 258B, 258C,** and **258D** refer to a specific one of the unattached, distal-pointing crowns.

At least two of the unattached, distal-pointing crowns **258** are located about 150 to about 180 degrees relative to each other and approximately the same distance from the proximal hub/tube **228,** as best seen in FIG. 12A. Optionally, the basket **246** comprises a first pair of unattached, distal-pointing crowns **258A** and **258B,** each of the first pair of unattached, distal-pointing crowns **258A** and **258B** is located approximately the same distance from the proximal hub/tube **228** and approximately 180 degrees relative to each other, and the basket **246** further comprises a second pair of unattached, distal-pointing crowns **258C** and **258D** located distally relative to, and approximately 90 degrees (e.g., between about 60 and about 90 degrees) relative to, the first pair of unattached, distal-pointing crowns **258A** and **258B.** Optionally, the second pair of unattached, distal-pointing crowns **258C** and **258D** form cells **250C** and **250D** that are adjacent to, but offset from, the cells **250A** and **250B** formed by the first pair of unattached, distal-pointing crowns **258A** and **258B.** (In other words, optionally, the center of cell **250A** is about 90 degrees relative to the centers of cells **250C** and **250D** and optionally the center of cell **250B** is also about 90 degrees relative to the centers of cells **250C** and **250D**). Optionally, at least one of (and preferably all) the unattached, distal-pointing crowns **258A, 258B, 258C or 258D** comprise an x-ray marker **244** that is more visible under x-ray as compared to the basket strips **291** when the distal body **216** is located in a cranial blood vessel **266** inside the body of a human and the x-ray is taken from outside the human's body. Preferably, the x-ray marker **244** is a radiopaque material. Some examples of radiopaque materials can include, but are not limited to, gold, platinum, palladium, tantalum, tungsten alloy, polymer material loaded with radiopaque filler, and the like. Preferably, the basket strips **291** are comprised of nitinol and the x-ray marker **244** is comprised of a material having a density greater than the nitinol. In some embodiments, the x-ray markers **244** comprise a heavy metal welded to the unattached, distal-pointing crowns **258.** Optionally, the unattached, distal-pointing crowns **258** curve subtly towards the interior **222** of the distal basket **246,** which decreases the likelihood that the unattached, distal-pointing crowns **258** will rub against and damage the vessel wall **268.** Optionally, the basket **246** comprises at least two cells proximal to the at least two cells **250** that include the unattached, distal-pointing crowns **258.** Optionally, the unattached, distal-pointing distal crowns **258** are located about at least 5 mm (e.g., about 5 to about 30 mm) from the proximal hub/tube **228.** Optionally, the unattached, distal-pointing crowns **258** are located at least about 5 mm from the distal hub/tube **236.** Optionally, the unattached, distal-pointing crowns **258** of the at least two cells **250** also each form part (namely a portion of the proximal boundary) of an enlarged cell **262** (which is the entry point of hard thrombus **270B** into the basket interior **222**) and further wherein the surface area of the enlarged cells **262** in the relaxed state is greater than the surface area of the other cells of the basket **246** in the relaxed state. Optionally, the unattached, distal-pointing crowns **258** serve several functions: 1) they form flex points of the basket **246,** which makes it easier for the system **200** to navigate the curves of the blood vessels **266** of the brains; 2) through the use of x-ray markers **244** on the unattached, distal-pointing crowns **258,** they allow the operator to locate the enlarged cells **262** of the basket **246** that form the point at which hard thrombuses **270B** enter the basket **246;** and 3) they allow the operator to ratchet or force the object **270** into the basket **246** by moving the unattached, distal-pointing crowns **258** proximally and distally relative to the object **270.** (As explained below, the numeral **270** refers to clots/thrombuses and other objects generally, and **270A** refers to a soft clot, **270B** refers to a hard clot and **270C** refers to a deformable, cohesive, adherent clot). Optionally, the proximal end **254** of a proximal strip **252** is located about 65-180 degrees (preferably approximately 180 degrees) relative to the distal end **256** of the same proximal strip **252,** as best seen in FIG. 12B. In other words, preferably the proximal end **254** of a first proximal strip **252** is attached to the 12 o'clock position on the proximal tube **228** and the distal end **256** of the first proximal strip **252** (which terminates at a proximal cell **248** of the basket **246)** is located at the 6 o'clock position (i.e., 180 degrees from the start position), and the proximal end **254** of a second proximal strip **252** is attached to the 6 o'clock position on the proximal tube **228** and the distal end **254** (which terminates at a cell **248** of the basket **246)** of the second proximal strip **252** is located at the 12 o'clock position (i.e., 180 degrees from the start position). This twisting feature serves two functions: 1) it allows the proximal strips **252** to surround the object **270;** and 2) it allows the manufacturer to insert a mandrel into the basket **246** during the shape-setting procedure. Optionally, the pull wire **202** is attached to the proximal tube **228** (e.g., by gluing, welding or the like). Preferably, the pull wire **202** does not extend through the distal basket interior **222.** Optionally, the proximal strips **252** are integral with the distal end **232** of the proximal tube **228** and the entire distal body **216** is created from a single tube **264** of a memory metal. Optionally, the proximal crowns **260** of the at least two cells **250** that include the unattached, distal pointing-crowns **258** are each attached to another cell **248** of the basket **246.** In other words, preferably the basket **246** does not have any free-floating proximal-pointing crowns, as free-floating proximal-pointing crowns could damage the vessel **266** when the distal body **216** is pulled proximally. Optionally, the system **200** further comprises a lead wire **286** extending distally from the distal tube **236,** the lead wire **286** having a length of from about 3 mm to about 10 mm. Optionally, the distal hub/tube **236,** the proximal hub/tube **228,** and the basket **246** are comprised of a nitinol having the same material composition. In other words, as with the prior embodiment of FIGs. 1-10, optionally the entire distal body **216** is manufactured from a single tube of nitinol **264.** Optionally, the proximal and distal hubs/tubes **228** and **236** comprise an x-ray marker **244** that is more visible under x-ray as compared to the basket strips **291** when the distal body **216** is located in a cranial blood vessel **266** inside the body of a human and the x-ray is taken from outside the human's body. Preferably, the x-ray marker **244** is a radiopaque material. Some examples of radiopaque materials can include, but are not limited to, gold, platinum, palladium, tantalum, tungsten alloy, polymer material loaded with radiopaque filler, and the like. Preferably, the basket strips **291** are comprised of nitinol and the x-ray marker **244** is comprised of a material having a density greater than the nitinol. In some embodiments, the proximal and distal hubs/tube interiors **234** and **242** may comprise tantalum welded or otherwise attached to the interior **234** and **242** of the proximal and distal hubs/tubes **228** and **236.** Optionally, the proximal and the distal tubes **228** and **236** are generally cylindrical in shape and each has an outer diameter and an inner diameter, the inner diameter forming apertures of the proximal and distal tubes **228** and **236** and further wherein the outer diameters of the proximal and distal tubes **228** and **236** are substantially the same size and further wherein the inner diameters of the proximal and distal tubes **228** and **236** are substantially the same size. Optionally, the outer diameters of the proximal and distal tubes **228** and **236** are from about 0.28 mm (0.011 inches) to about 1.37 mm (0.054 inches), and further wherein the inner diameters of the proximal and distal tubes **228** and **236** are from about 0.20 mm (0.008 inches) to about 1.30 mm (0.051 inches). Optionally, the pull wire **202** is generally cylindrical and further wherein the diameter of the pull wire **202** is between about 0.20 mm (0.008 inches) and about 1.30 mm (0.051 inches). Optionally, the distal body **216** has a length of between about 10 and about 60 millimeters. Optionally, the first height **224** and first width **226** of the distal body **216** are between about 2 millimeters and about 6 millimeters.

The present disclosure also provides an exemplary method of removing a clot or other object **270** from an interior lumen **266** of an animal, the exemplary method comprising the steps of:
a) providing the system **200** of Figures 11-29, wherein at least two cells **250** of the basket **246** comprise a proximal crown **260** pointing generally in the proximal direction and a distal crown **258** pointing generally in the distal direction, and the distal crowns **258** of the at least two cells **250** are not attached to another cell **248** of the basket **246** (i.e., free-floating), and further wherein at least one of the unattached, distal-pointing crowns **258** comprises an x-ray marker **244**;
b) positioning the system **200** in the lumen **266**;
c) deploying the distal body **216** from the distal end **214** of the delivery catheter **208**;
d) allowing the height and width **224** and **226** of the distal body **216** to increase;
e) irradiating the x-ray marker **244** with x-ray radiation and
f) moving the object **270** into the distal basket interior **222.**

Optionally, the object **270** enters the distal basket interior **222** adjacent to (preferably adjacent and immediately distal to) at least one of the unattached, distal-pointing crowns **258** - i.e., in the enlarged cells/drop zones **262.** In some embodiments, the distal body **216** is deployed so that at least one (e.g., preferably the two proximal **258A** and **258B**) of the unattached, distal-pointing crowns **258** is distal to the object **270.** As explained below, the x-ray markers **244** of the unattached, distal-pointing crowns **258** are used to locate the distal body **216** relative to the clot or other object **270.** It will be appreciated that clots **270** can generally be located in blood vessels **266** by injecting a contrast dye, for example, into the blood vessel **266** proximal and distal to the believed area of obstruction and viewing on an x-ray where the fluid stops moving in the blood vessel **266.** It will also appreciated that if the object **270** is not a blood clot but is a radio-opaque object, the object **270** may be viewed on an x-ray.

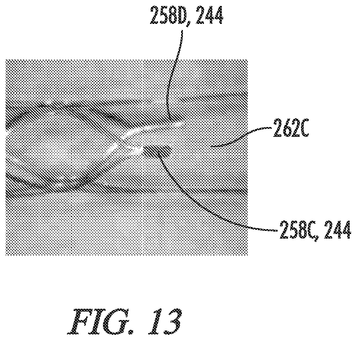

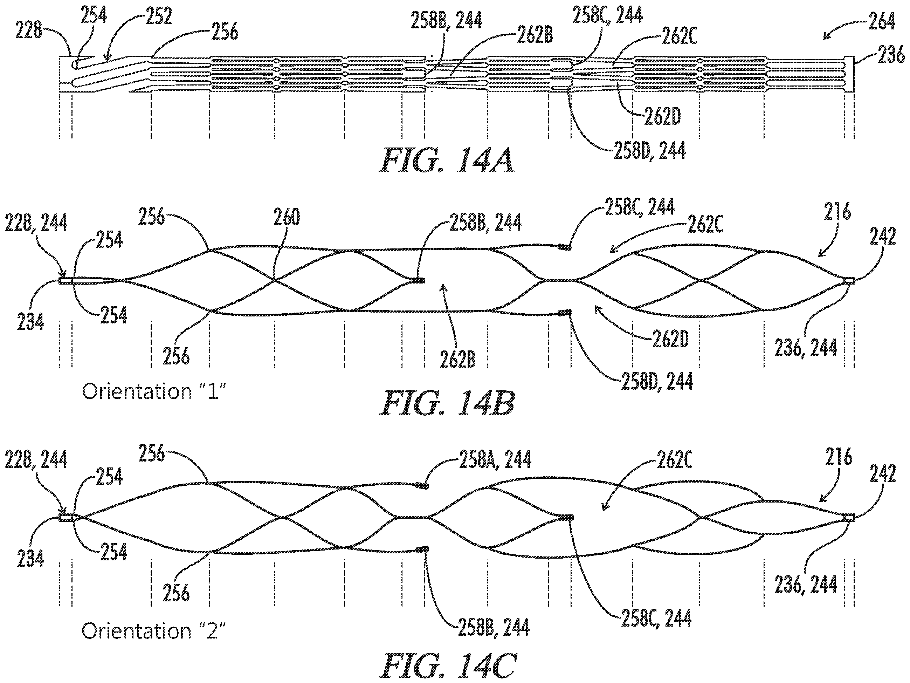

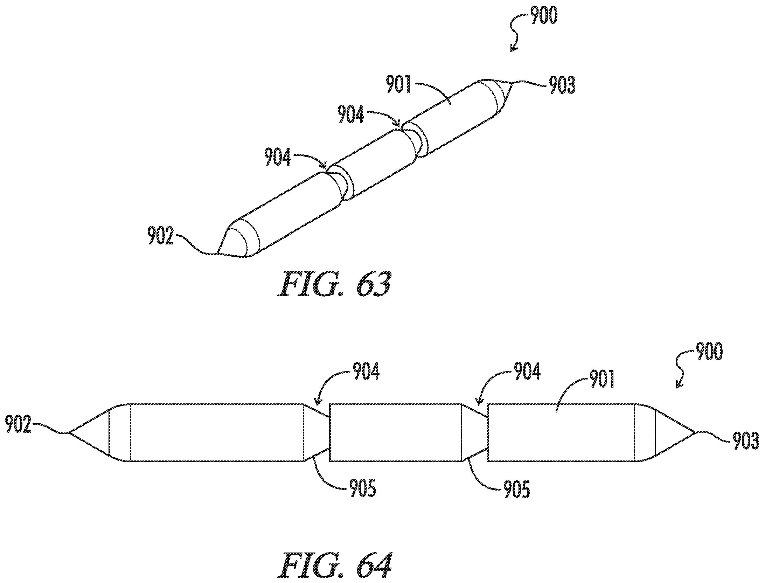

FIGs. 11 and 14B illustrate a first, perspective view of one embodiment of a distal body **216** with twisting proximal strips **252**, unattached distal-pointing crowns **258** that subtly curve inward and have x-ray markers **244**, and enlarged openings/drop zones **262** in the basket **246** that allow the obstruction or other object **270** to enter. In FIGs. 11 and 14B, the distal body **216** is in Orientation 1. (To prepare a basket **246** with unattached distal-pointing crowns **258** that curve inward toward the basket interior **292,** a mandrel **900** such as that illustrated in FIGs. 63 and 64 may be used. The mandrel **900** includes a generally cylindrical body **901** with tapered proximal and distal ends **902** and **903** that slope like the ends of a pencil. The cylindrical body **901** includes two grooves **904** that extend around the circumference of the cylindrical body **901.** The grooves **904** include tapered portions **905** that slope towards the distal end **903,** which are designed to shape the unattached distal-pointing crowns **258.** The grooves **904** are generally in the shape of a truncated cone, as shown in FIGs. 63-64). The two proximal, unattached distal-pointing crowns **258A** and **258B** are located approximately the same distance from the proximal hub/tube **228** and are oriented approximately 180 degrees relative to each other. The two distal, unattached distal-pointing crowns **258C** and **258D** are located approximately the same distance from the proximal hub/tube **228** as each other (and distal to the two proximal, unattached distal-pointing crowns **258A** and **258B)** and are oriented approximately 180 degrees relative to each other and approximately 90 degrees to the proximal, unattached distal-pointing crowns **258A** and **258B.** The two proximal enlarged openings/drop zones **262A** and **262B** distal to the proximal, unattached distal pointing crowns **258A** and **258B** are located approximately the same distance from the proximal hub/tube **228** and the centers of the two proximal enlarged openings/drop zones **262A** and **262B** are oriented approximately 180 degrees relative to each other. (As noted above, preferably, the proximal, unattached distal-pointing crowns **258A** and **258B** form part of the proximal boundary of the proximal, enlarged cells/drop zones **262A** and **262B,** and the distal, unattached distal-pointing crowns **258C** and **258C** form part of the proximal boundary of the distal, enlarged cells/drop zones **262C** and **262D**). The two distal, enlarged openings/drop zones **262C** and **262D** distal to the distal, unattached distal pointing crowns **258C** and **258D** are located approximately the same distance from the proximal hub/tube **228** and the centers of the distal, enlarged openings/drop zones **262C** and **262D** are oriented approximately 180 degrees relative to each other and approximately 90 degrees relative to the proximal enlarged openings/drop zones **262A** and **262B.** FIGs. 12A and 14C illustrate a second view of the distal body **216** of FIG. 11 (Orientation 2). FIG. 13 is a close-up view of two unattached, distal-pointing crowns **262.** The lines in FIG. 14 show how a nitinol tube **264** is cut with a laser to create the distal body **216** shown in FIG. 14B and FIG. 14C. It will be appreciated that FIG. 14B is a simplified view of the distal body **216** and orientation shown in FIG. 11 and FIG. 14C is a simplified view of the distal body **216** and orientation shown in FIG. 12A.

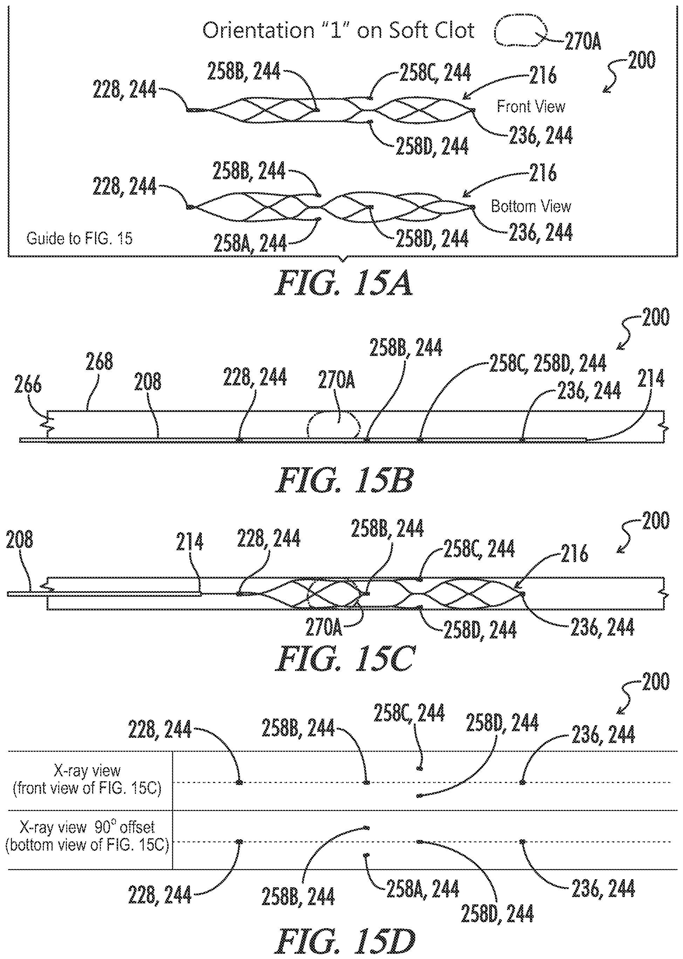

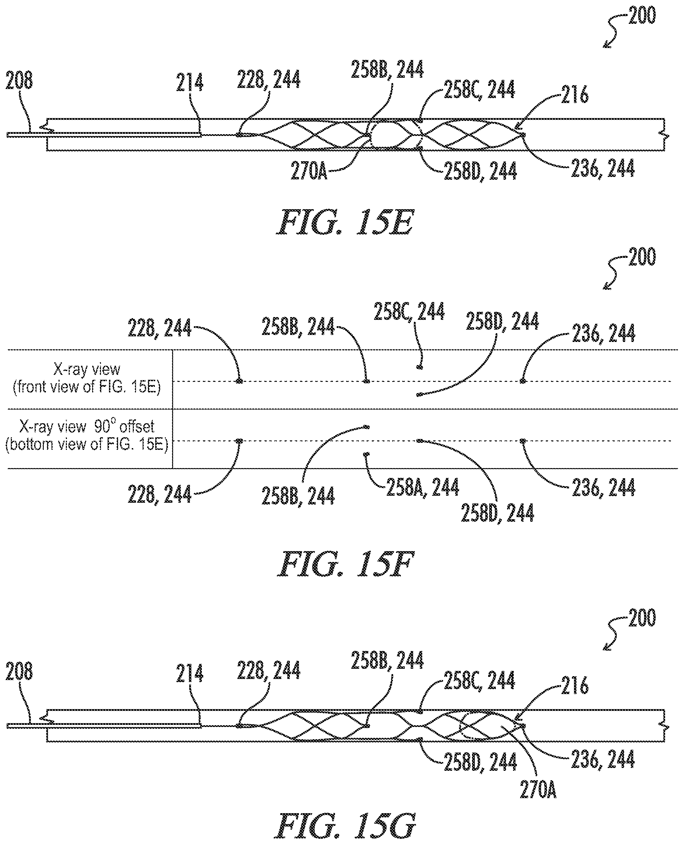

As described below, FIGs. 15-19 describe how the distal body **216** is used to retrieve, soft clots **270A,** hard clots **270B,** and deformable, cohesive adhesive clots **270C** in a human intracranial artery **266.** (In FIGs. 15-19, the center of the artery **266** is denominated by the dashed line). As explained below, the distal body **216** has four rows of x-ray markers namely, 1) a first row of one x-ray marker, which is located inside the proximal tube denominated by the numeral **228, 244**; 2) a second row of two x-ray markers, which are located at the two proximal, unattached distal-pointing crowns (the two markers are oriented 180 degrees relative to each other) denominated by the numerals **258A, 244** and **258B, 244**; 3) a third row of two x-ray markers, which are located at the two distal, unattached distal-pointing crowns (these two markers are oriented 180 degrees relative to each other and 90 degrees relative to the two proximal, unattached distal-pointing crowns) denominated by the numerals **258C, 244** and **258D, 244;** and 4) a fourth row of one x-ray marker, which is located inside the distal tube denominated by the numeral **236, 244.** (It will be appreciated that the first number in the sequence describes the position of the x-ray marker and the second number, **244**, represents the fact that the item is an x-ray marker). As explained below, upon deploying the distal body **216** so that the two proximal, unattached distal-pointing crowns **258A, 244** and **258B, 244** are immediately distal to the clot **270,** the surgeon interventionalist (i.e., operator of the distal body **216**) detects the four rows of x-ray markers using x-ray radiation from a first vantage point and from a second vantage point that is offset from the first vantage point (e.g. 90 degrees). Next, the surgeon moves the distal body **216** proximally relative to the clot **270** and takes additional x-rays from the first and second vantage points. As explained in greater detail below, the surgeon uses the x-ray markers of the proximal and distal, unattached distal-pointing crowns, namely **258A, 244; 258B, 244; 258C, 244;** and **258D, 244** (more specifically, the convergence or lack thereof of the proximal and distal, unattached distal-pointing crowns **258A, 244; 258B, 244; 258C, 244;** and **258D, 244** as shown on the x-ray) to determine whether the clot **270** is located inside the distal body interior **222** or whether the clot **270** is collapsing the distal body **216.**

More specifically, FIGs. 15A-G illustrate stepwise use of the distal body **216** in retrieving a soft clot **270A** in a human intracranial artery **266.** (The distal body **216** in FIGS. 15A-15G is in Orientation 1). First, as always, the surgeon determines the location of the clot **270A** in the vessel **266** using, for example, a contrast dye injected proximal and distal to the clot **270A.** Next, the delivery catheter **208**, which is enveloping the distal body **216**, is positioned in the blood vessel **266** so that the two proximal, unattached distal-pointing crowns **258A** and **258B** are immediately distal to the clot **270A.** See FIG. 15B. The distal body **216** is then deployed from the delivery catheter **208** by moving the catheter **208** proximally. The soft clot **270A,** which is unable to collapse the distal body **216**, then enters the distal body interior **222.** See FIG. 15C. However, at this time, the surgeon is unaware that the clot **270A** has entered into the distal body interior **222.** Thus, without moving the distal body **216**, the surgeon irradiates the four rows of x-ray markers at a first vantage point (i.e., from the front of the distal body **216** in the orientation shown in FIGs. 15A-G; i.e., into the page). As shown in FIG. 15D, the first vantage point shows four rows of x-ray markers. The first row is a single point, which represents the x-ray marker located in the proximal tube **228, 244**; the proximal tube x-ray marker **228, 244** always appears as a single point. The second row is a single point, which represents the x-ray marker located at the front, proximal, unattached distal-pointing crown **258B, 244**; the reason that this second row of markers is a single point is that the rear x-ray marker of the second row **258A, 244** is hidden from view because it is directly behind the front x-ray marker of the second row **258B, 244.** The third row has two points, which represents the two x-ray markers located at the distal, unattached distal-pointing crowns **258C, 244** and **258D**, **244**; the reason that this third row of markers has two points is that neither marker in the third row **258C, 244** and **258D, 244** is hidden from view on the x-ray at this angle - rather, one marker **258C, 244** is located above the other marker **258D, 244** - and as shown in FIG. 15C, the distal body **216** is not collapsed at the distal, unattached distal-pointing crowns **258C, 244** and **258D, 244.** The fourth row is a single point, which represents the x-ray marker located in the distal tube **236, 244**; the distal tube x-ray marker **236, 244** always appears as a single point. Without moving the distal body **216**, the surgeon then irradiates the four rows of x-ray markers from a second vantage point 90 degrees offset from the first vantage point (i.e., from the bottom of the distal body **216** in the orientation shown in FIG. 15A). As shown, the first row is, as always, a single point, which represents the x-ray marker located in the proximal tube **228, 244.** The second row has two points, which represents the two x-ray markers located at the proximal, unattached distal-pointing crown **258A, 244** and **258B**, **244**; the reason that this second row of markers shows up as two points is that neither marker **258A, 244** and **258B, 244** in the second row is hidden from view on the x-ray at this offset angle - rather, one marker **258B, 244** is located above the other marker **258A, 244** - and the distal body **216** is not collapsed at the proximal, unattached distal-pointing crowns **258A, 244** and **258B, 244.** The third row is a single point, which represents the x-ray marker located at the bottom, distal, unattached distal-pointing crown **258D, 244**; the reason that this third row of markers is a single point is that the top x-ray marker of the third row **258C, 244** is directly behind the bottom x-ray marker of the third row **258D**, **244**, and thus, hidden from view. The fourth row is, as always, a single point, which represents the x-ray marker located in the distal tube **236, 244.** The surgeon, thus, concludes that neither the x-ray markers at the second row **258A, 244** and **258B, 244** nor the x-ray markers at the third row **258C, 244** and **258D, 244** (i.e., the x-ray markers at both the proximal and distal unattached distal pointing-crowns) have converged. As shown in FIG. 15E, the surgeon then moves the distal body **216** proximally relative to the soft clot **270A** so that the distal, unattached distal-pointing crowns **258C, 244** and **258D, 244** are immediately distal to the clot **270A** and then the surgeon irradiates the four rows of x-ray markers again from the first vantage point and the second vantage point. As shown in FIG. 15F, the results are the same as FIG. 15D. With the results from FIGs. 15D and 15F, the surgeon concludes that neither x-ray markers at the second row **258A, 244** and **258B, 244** nor the x-ray markers at the third row **258C, 244** and **258D, 244** (i.e., the x-ray markers at both the proximal and distal unattached distal pointing-crowns) converged at either the original position of the distal body **216** (FIGs. 15C and 15D) or the position after moving the distal body **216** proximally (FIGs. 15E and 15F), and, thus, the distal body **216** was expanded in the vessel **266** in both positions. Thus, the surgeon concludes that the clot is a soft clot **270A** that has entered into the distal body interior **222** and the surgeon removes the distal body **216** and the soft clot **270A**, captured by the distal body **216**, by moving the distal body **216** proximally out of the vessel **266**, as shown in FIG. 15G.

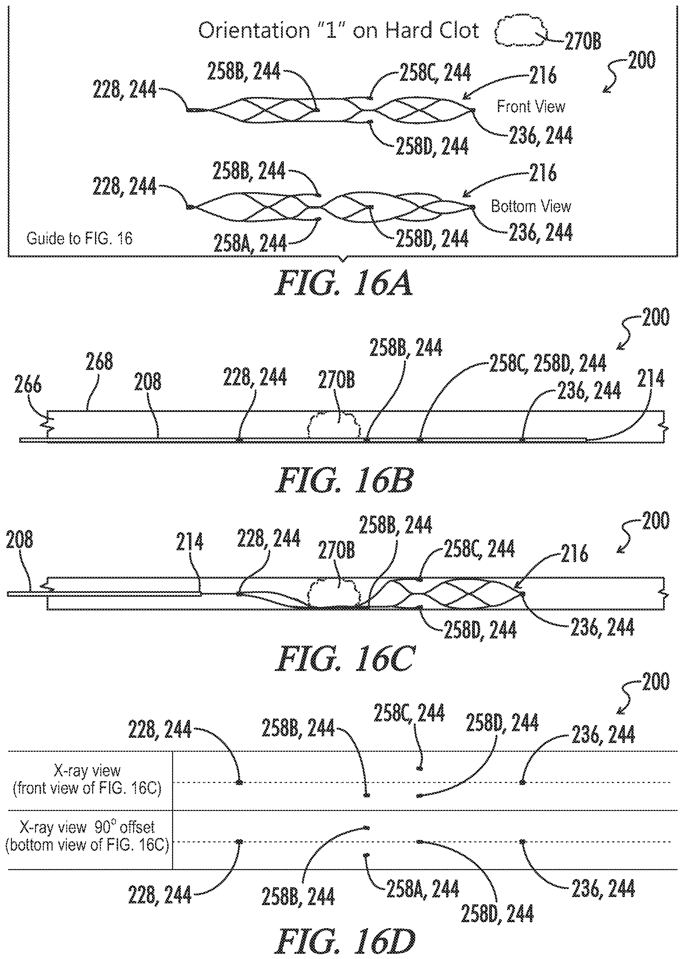

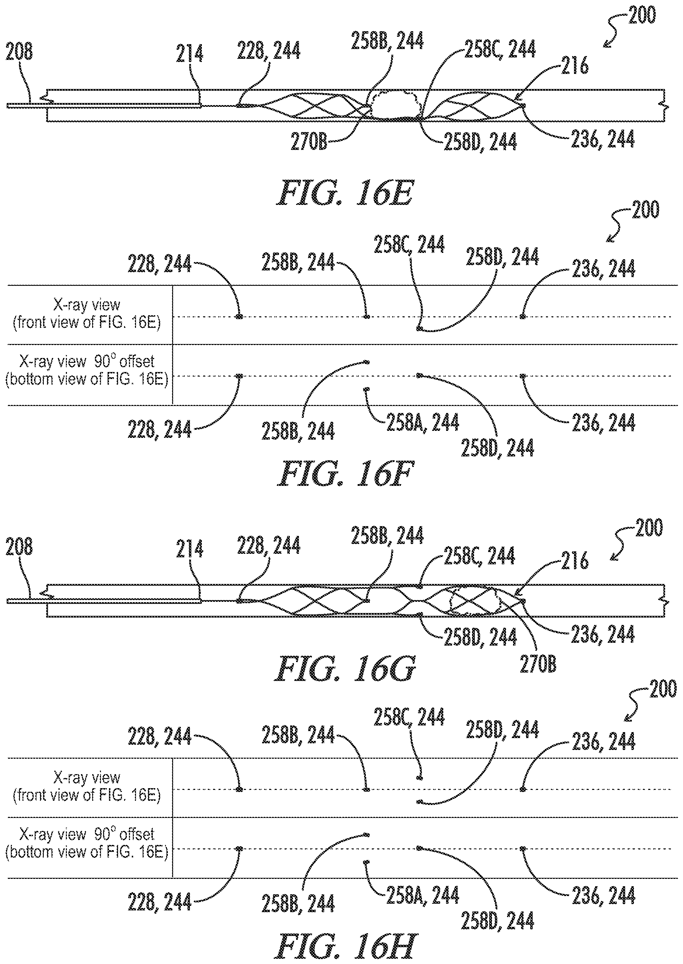

FIGs. 16A-H illustrate stepwise use of the distal body **216** in retrieving a hard clot **270B** in a human intracranial artery **266.** (In FIGs. 16A-H, the distal body **216** is in Orientation 1). First, as always, the surgeon determines the location of the clot **270B** in the vessel **266** using, for example, a contrast dye injected proximal and distal to the clot **270B.** Next, the delivery catheter **208**, which is enveloping the distal body **216**, is positioned in the blood vessel **266** so that the two proximal, unattached distal-pointing crowns **258A** and **258B** are immediately distal to the clot **270B.** See FIG. 16B. The distal body **216** is then deployed from the delivery catheter **208** by moving the catheter **208** proximally. The hard clot **270B**, which is located above the distal body **216**, collapses the distal body **216**, as shown in FIG. 16C. However, at this time, the surgeon is unaware that the clot **270B** has collapsed the distal body **216.** Thus, without moving the distal body **216**, the surgeon irradiates the x-ray markers at a first vantage point (i.e., from the front of the distal body **216**; i.e., into the page). As shown in FIG. 16D, the first vantage point shows four rows of x-ray markers. The first row is, as always, a single point, representing the x-ray marker located in the proximal tube - i.e., **228, 244.** The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has two points, which represents the two x-ray markers located at the distal, unattached distal-pointing crowns **258C, 244** and **258D, 244;** the reason that this third row of markers has two points is that neither marker in the third row is hidden from view on the x-ray at this angle - rather, one marker **258C, 244** is located above the other marker **258D, 244** - and as shown in FIG. 16C, the distal body **216** is not collapsed at the distal, unattached distal-pointing crowns **258C, 244** and **258D, 244.** The fourth row is, as always, a single point, representing the x-ray marker located in the distal tube **236, 244.** Without moving the distal body **216**, the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has two points, which represents the two x-ray markers located at the proximal, unattached distal-pointing crowns **258A, 244** and **258B, 244;** the reason that this second row of markers shows up as two points is that neither marker in the second row is hidden from view on the x-ray at this offset angle - rather, one marker **258B, 244** is located above the other marker **258A, 244** - and although the distal body **216** is collapsed at the proximal, unattached distal-pointing crowns as shown in FIG. 16C, the second row of x-ray markers have not converged because the clot **270B** is on top of the second row of x-ray markers. The third row is a single point, which represents the x-ray marker located at the bottom, distal, unattached distal-pointing crown **258D, 244**; the reason that this third row of markers is a single point is that the top x-ray marker of the third row **258C, 244** is directly behind the bottom x-ray marker of the third row **258D, 244,** and thus, hidden from view. The fourth row is, as always, a single point, which represents the x-ray marker located in the distal tube **236, 244.** The surgeon, thus, concludes that neither the second row **258A, 244** and **258B, 244** nor the third row **258C, 244** and **258D, 244** of x-ray markers (i.e., the x-ray markers at both the proximal and distal unattached distal pointing-crowns) has converged. As shown in FIG. 16E, the surgeon then moves the distal body **216** proximally so that the distal, unattached distal-pointing crowns **258C, 244** and **258D, 244** are immediately distal to the clot **270B** and the surgeon then irradiates the x-markers again from the first vantage point. As shown in FIG. 16F, the first row is, as always, a single point, representing the x-ray marker located in the proximal tube **228, 244.** The second row is a single point, which represents the x-ray marker located at the front, proximal, unattached distal-pointing crown **258B, 244;** the reason that this second row of markers is a single point is that the rear x-ray marker of the second row **258A, 244** is hidden from view because it is directly behind the front x-ray marker of the second row **258B, 244.** The third row has only one point because the clot **270B**, which is on top of the third row of x-ray markers **258C, 244** and **258D, 244** (i.e., the markers at the distal, unattached distal-pointing crowns), has pushed the third row of x-ray markers **258C, 244** and **258D, 244** together. The fourth row is, as always, a single point, representing the x-ray marker located in the distal tube **236, 244.** Without moving the distal body **216**, the surgeon then irradiates the markers from a second vantage point 90 degrees offset from the first vantage point (i.e., from the bottom of the distal body). As shown, the first row is, as always, a single point, which represents the x-ray marker located in the proximal tube **228, 244.** The second row has two points, which represents the two x-ray markers located at the proximal, unattached distal-pointing crown **258A, 244** and **258B, 244;** the reason that this second row of markers shows up as two points is that neither marker in the second row is hidden from view on the x-ray at this offset angle and the distal body **216** is not collapsed at the proximal, unattached distal-pointing crowns **258A, 244** and **258B, 244.** The third row is a single point, which represents the x-ray marker located at the bottom, distal, unattached distal-pointing crown **258D, 244;** the reason that this third row of markers is a single point is that the bottom x-ray marker of the third row **258D, 244** is directly in front of the top x-ray marker of the third row **258C, 244,** and thus, the top x-ray marker of the third row **258C, 244** is hidden from view. The fourth row is, as always, a single point, which represents the x-ray marker located in the distal tube **236, 244.** Knowing that the distal, unattached distal-pointing crowns **258C, 244** and **258D, 244** have converged as shown in FIG. 16F, the surgeon moves the distal body **216** proximally and the hard clot **270B** falls into the distal body interior **222** in the enlarged cell/drop zone **262C** immediately distal to the top, distal, unattached distal-pointing crown **258C.** See FIG. 16G. To confirm that the hard clot **270B** has entered the distal body interior **222**, the surgeon takes x-rays from the first and second vantage points. The results are shown in FIG. 16H. As compared to 16F, the front x-ray view of FIG. 16H shows that the distal, unattached distal-pointing crowns **258C, 244** and **258D, 244** are not converged, and, thus, the surgeon concludes that the hard clot **270B** has entered the distal body interior **222.** The surgeon then removes the distal body **216** and the hard clot **270B**, captured by the distal body **216**, by moving the distal body **216** proximally out of the vessel **266.**

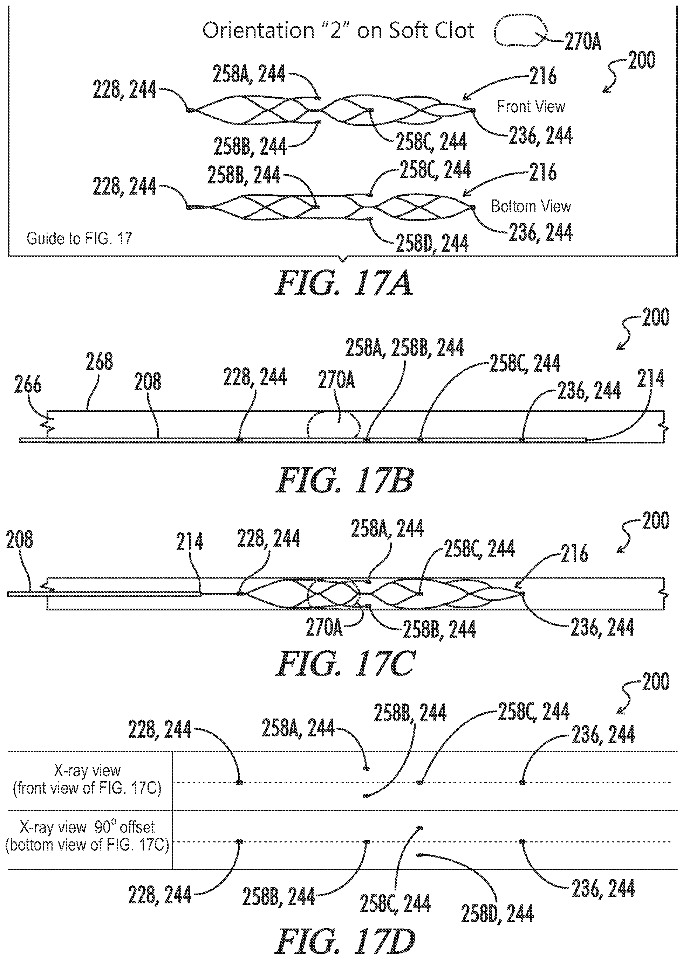

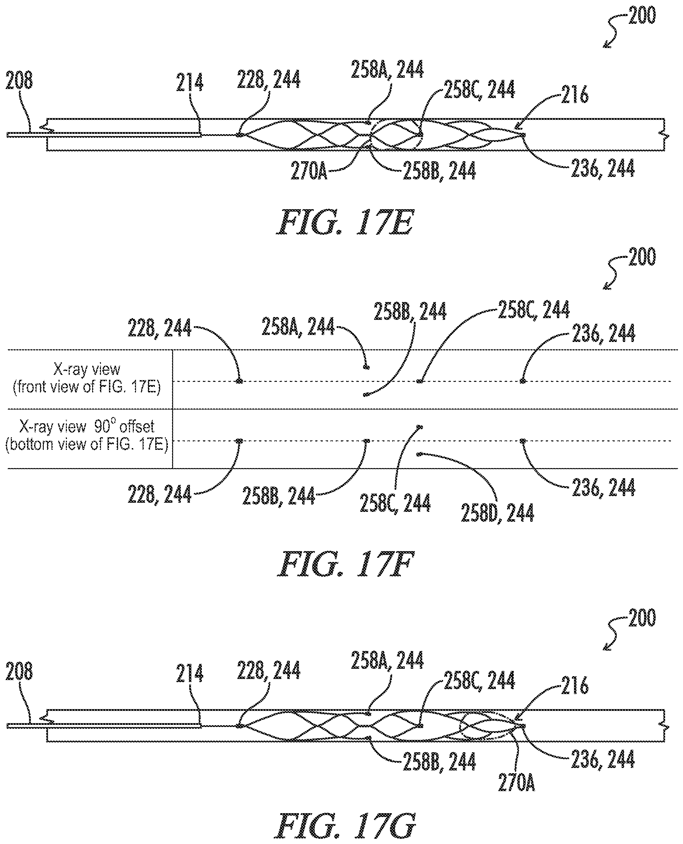

FIGs. 17A-G illustrate stepwise use of the distal body **216** in retrieving a soft clot **270A** in a human intracranial artery **266.** (In FIGs. 17A-G, the distal body **216** is in Orientation 2). First, as always, the surgeon determines the location of the clot **270A** in the vessel **266** using, for example, a contrast dye injected proximal and distal to the clot **270A.** Next, the delivery catheter **208**, which is enveloping the distal body **216**, is positioned in the blood vessel **266** so that the two proximal, unattached distal-pointing crowns **258A** and **258B** are immediately distal to the clot **270A.** See FIG. 17B. The distal body **216** is then deployed from the catheter **208** by moving the catheter **208** proximally. The soft clot **270A,** which is unable to collapse the distal body **216**, then enters the distal body interior **222.** See FIG. 17C. However, at this time, the surgeon is unaware that the clot **270A** has entered into the distal body interior **222.** Thus, without moving the distal body **216,** the surgeon irradiates the x-ray markers at a first vantage point (i.e., from the front of the distal body; into the page). As shown in FIG. 17D, the first vantage point shows four rows of x-ray markers. The first row is, as always, a single point, representing the x-ray marker located in the proximal tube **228, 244.** The second row has two points, which represents the two x-ray markers located at the proximal, unattached distal-pointing crowns **258A, 244** and **258B, 244**; the reason that this second row of markers has two points is that neither marker in the second row is hidden from view on the x-ray at this angle - rather, one marker **258A, 244** is located above the other marker **258B, 244** - and as shown in FIG. 17C, the distal body **216** is not collapsed at the proximal, unattached distal-pointing crowns **258A, 244** and **258B, 244.** The third row has a single point, which represents the x-ray marker located at the front (in Orientation 2), distal, unattached distal-pointing crown **258C, 244**; the reason that this third row of markers is a single point is that the rear (in Orientation 2) x-ray marker **258D, 244** of the third row is hidden from view because it is directly behind the front x-ray marker **258C, 244** of the third row. The fourth row is, as always, a single point, representing the x-ray marker located in the distal tube **236, 244.** Without moving the distal body, the surgeon then irradiates the markers from a second vantage point 90 degrees offset from the first vantage point (i.e., from the bottom of the distal body, as shown in this view). As shown, the first row is, as always, a single point, which represents the x-ray marker located in the proximal tube **228, 244.** The second row is a single point, which represents the x-ray marker located at the bottom (in Orientation 2), proximal, unattached distal-pointing crown **258B, 244**; the reason that this second row of markers is a single point is that the top (in Orientation 2) x-ray marker of the second row **258A, 244** is directly behind the bottom x-ray marker of the second row **258B, 244**, and thus, hidden from view. The third row has two points, which represents the two x-ray markers located at the distal, unattached distal-pointing crowns **258C, 244** and **258D, 244**; the reason that this third row of markers shows up as two points is that neither marker in the third row is hidden from view on the x-ray at this offset angle and the distal body **216** is not collapsed at the distal, unattached distal-pointing crowns **258C, 244** and **258D, 244.** The fourth row is, as always, a single point, which represents the x-ray marker located in the distal tube **236, 244.** The surgeon, thus, concludes that neither the second row **258A, 244** and **258B, 244** nor the third row of x-ray markers **258C, 244** and **258D, 244** (i.e., the x-ray markers at both the proximal and distal unattached distal pointing-crowns) has converged. As shown in FIG. 17E, the surgeon then moves the distal body **216** proximally relative to the clot **270A** so that the distal, unattached distal-pointing crowns **258C, 244** and **258D, 244** are immediately distal to the clot **270A** and then the surgeon irradiates the x-markers again from the first vantage point and the second vantage point. As shown in FIG. 17F, the results are the same as FIG. 17D. With the results from FIGs. 17D and 17F, the surgeon concludes that neither the second row **258A, 244** and **258B, 244** nor the third row of x-ray markers **258C, 244** and **258D, 244** (i.e., the x-ray markers at both the proximal and distal unattached distal pointing-crowns) were converged at either the original position of the distal body **216** (FIG. 17C and 17D) or the position after moving the distal body **216** proximally (FIG. 17E and 17F), and, thus, the distal body **216** was expanded in the vessel **266** in both positions. Thus, the surgeon concludes that the clot **270A** is a soft clot **270A** that has entered into the distal body interior **222** and the surgeon removes the distal body **216** and the soft clot **270A**, captured by the distal body **216**, by moving the distal body **216** proximally out of the vessel **266**, as shown in FIG. 17G.

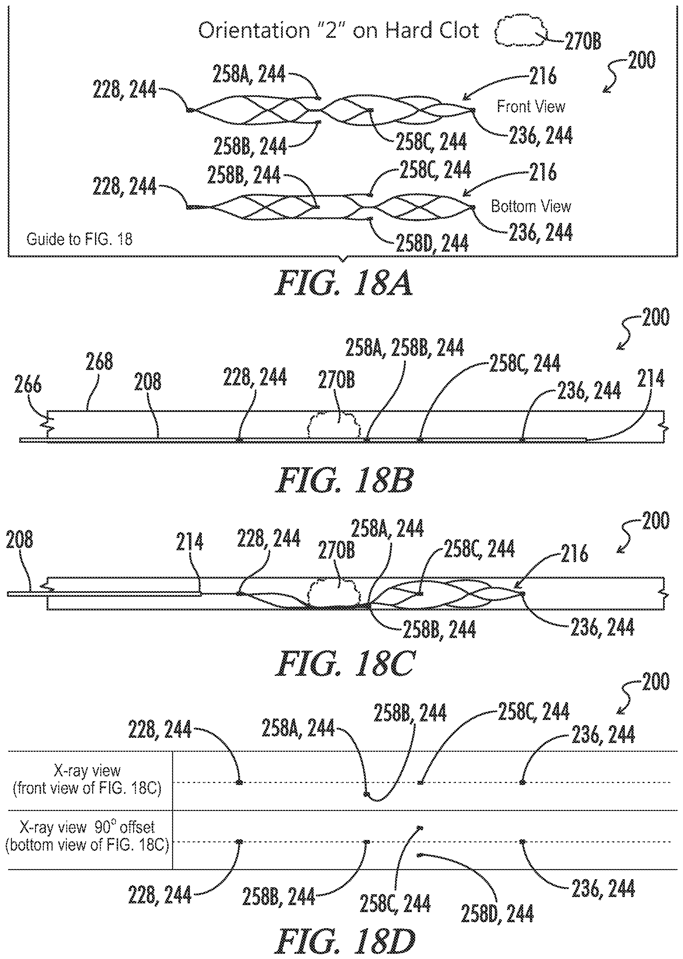

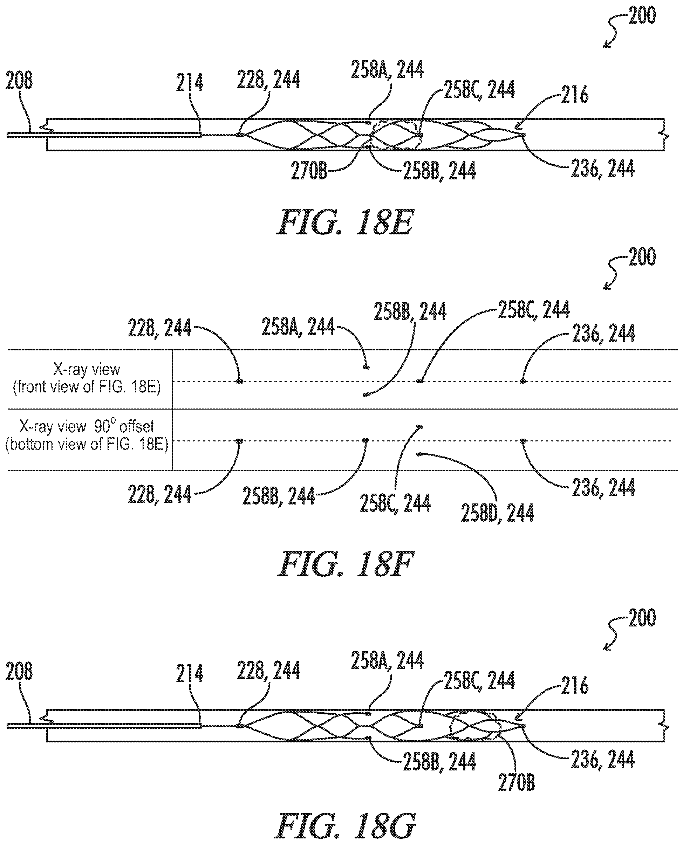

FIGs. 18A-G illustrate stepwise use of the distal body **216** in retrieving a hard clot **270B** in a human intracranial artery **266.** (In FIGS. 18A-G, the distal body **216** is in Orientation 2). (As described below, the primary differences between FIGs 18A-G and FIGs. 16A-G is that the clot **270B** enters the distal body interior **222** in an enlarged cell/drop zone **262A** immediately distal to one of the proximal, unattached distal-pointing crowns **258A** in FIGs. 18A-G, as compared to FIGs. 16A-G where the clot **270B** enters the distal body interior **222** in an enlarged cell/drop zone **262C** immediately distal to one of the distal, unattached distal-pointing crowns **258C**). First, as always, the surgeon determines the location of the clot **270B** in the vessel **266** using, for example, a contrast dye injected proximal and distal to the clot **270B.** Next, the delivery catheter **208**, which is enveloping the distal body **216**, is positioned in the blood vessel **266** so that the two proximal, unattached distal-pointing crowns **258A** and **258B** are immediately distal to the clot **270B.** See FIG. 18B. The distal body **216** is then deployed from the catheter **208** by moving the catheter **208** proximally. The hard clot **270B**, which is located above the distal body **216**, collapses the distal body **216**, as shown in FIG. 18C. However, at this time, the surgeon is unaware that the clot **270B** has collapsed the distal body **216.** Thus, without moving the distal body **216**, the surgeon irradiates the x-ray markers at a first vantage point (i.e., from the front of the distal body in Orientation 2; into the page). As shown in FIG. 18D, the first vantage point shows four rows of x-ray markers. The first row is, as always, a single point, representing the x-ray marker located in the proximal tube **228, 244.** The second row has only one point because the clot **270B**, which is on top of the second row of x-ray markers **258A, 244** and **258B, 244** (i.e., the markers at the proximal, unattached distal-pointing crowns), has pushed them together. The third row has only one point, which represents the x-ray marker located at the front (in Orientation 2), proximal, unattached distal-pointing crown **258C, 244**; the reason that this third row of markers is a single point is that the rear (in this view) x-ray marker of the third row **258D, 244** is hidden from view because it is directly behind the front x-ray marker of the third row **258C, 244.** The fourth row is, as always, a single point, representing the x-ray marker located in the distal tube **236, 244.** Without moving the distal body, the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has a single point because the top (in Orientation 2) x-ray marker of the second row **258A, 244** is located behind the bottom (in Orientation 2) x-ray marker **258B, 244** and thus, the top x-ray marker of the second row **258A, 244** is hidden from view. The third row has two points, which represents the x-ray markers located at the distal, unattached distal-pointing crowns **258C, 244** and **258D**, **244;** in this x-ray view neither of the x-ray markers of the third row is hidden from view. The fourth row is, as always, a single point, which represents the x-ray marker located in the distal tube **236, 244.** The surgeon, thus, concludes that the second row of x-ray markers **258A, 244** and **258B, 244** (i.e., the x-ray markers at the proximal, unattached distal pointing-crowns) has converged. As shown in FIG. 18E, the surgeon then moves the distal body **216** proximally so that the distal, unattached distal-pointing crowns **258C, 244** and **258D, 244** are immediately distal to the clot **270B.** Unbeknownst to the surgeon, the clot **270B** enters the distal body interior **222** immediately distal to the top (in Orientation 2), proximal unattached distal-pointing crown **258A** and the distal body **216** is no longer collapsed. The surgeon then irradiates the x-markers again from the first vantage point. As shown in FIG. 18F, the first row is, as always, a single point, representing the x-ray marker located in the proximal tube **228, 244.** The second row has two x-ray markers because the distal body **216** is not collapsed and neither the top (in Orientation 2) **258A, 244** nor the bottom **258B, 244** (in Orientation 2) x-ray marker of the second row (i.e., the marker at the proximal, unattached distal-pointing crowns) is hidden from view. The third row has only one point because the rear (in Orientation 2), distal unattached distal-pointing crown **258D, 244** is hidden behind the front (in Orientation 2), distal, unattached distal pointing-crown **258C, 244.** The fourth row is, as always, a single point, representing the x-ray marker located in the distal tube **236, 244.** Without moving the distal body **216**, the surgeon then irradiates the markers from a second vantage point 90 degrees offset from the first vantage point (i.e., from the bottom of the distal body **216**). As shown, the first row is, as always, a single point, which represents the x-ray marker located in the proximal tube **228, 244.** The second row has a single point because the x-ray marker at the top (in Orientation 2), proximal, unattached distal-pointing crown **258A, 244** is hidden behind the bottom (in Orientation 2), proximal, unattached-distal pointing crown **258B, 244.** The third row has two points because neither the front nor the rear x-ray markers at the distal, unattached, distal-pointing crowns **258C, 244** and **258D, 244** is hidden from view. The fourth row is, as always, a single point, which represents the x-ray marker located in the distal tube **236, 244.** Based on the information from FIGs. 18D and 18F, the surgeon concludes that the clot **270B** has entered into the distal body interior **222.** The surgeon then removes the distal body **216** and the hard clot **270B**, captured by the distal body **216**, by moving the distal body **216** proximally out of the vessel **266**, as shown in FIG. 18G. Upon comparing FIGs. 16A-G and FIGs. 18A-G it will be appreciated that the orientation of the enlarged cells/drop zone **262A-D** relative to the orientation of a hard clot **270B** determine which enlarged cell/drop zone **262A, 262B, 262C,** or **262D,** the hard clot **270** enters the distal body interior **222** through. For example, in FIG. 16C, the hard clot **270B** is located above the distal body **216**, and thus, the hard clot **270B** must enter through the enlarged cell/drop zone located at the top of the distal body, which in the orientation of the distal body shown in FIGs. 16A-G, is the enlarged cell/drop zone **262C** immediately distal to the top, distal, unattached, distal-pointing crown **258C.** In FIG. 18C, the hard clot **270B** is again located above the distal body and, thus, the hard clot **270B** must enter through the enlarged cell/drop zone located at the top of the distal body. However, in FIG. 18C, the enlarged cell/drop zone located at the top of the distal body **216**, in the orientation of the distal body **216** shown in FIGs. 18A-G, is the enlarged cell/drop zone **262A** immediately distal to the top, proximal, unattached, distal-pointing crown **258A.**

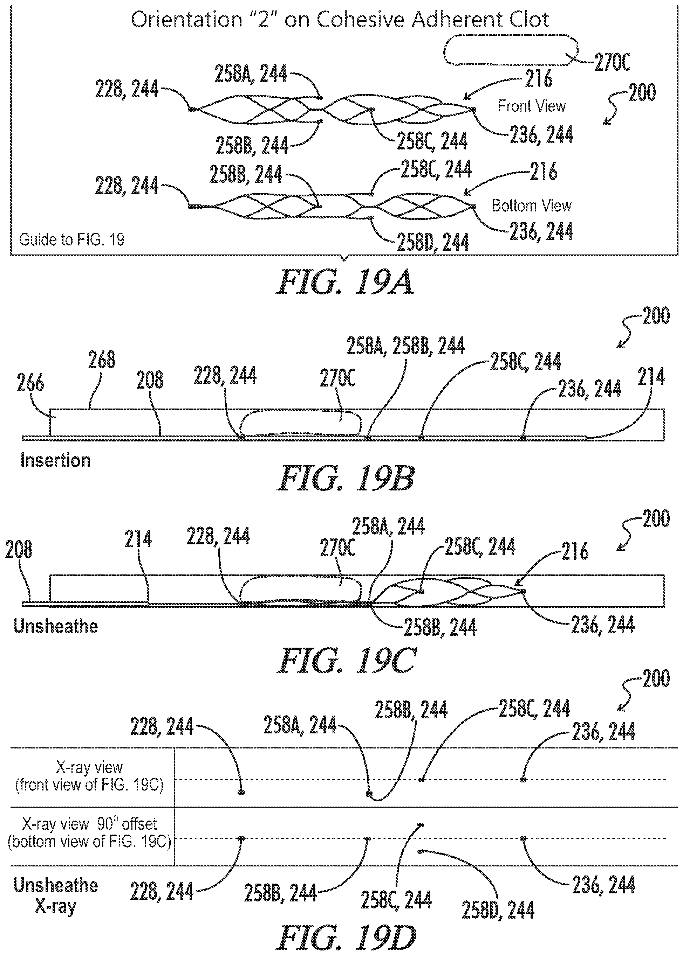

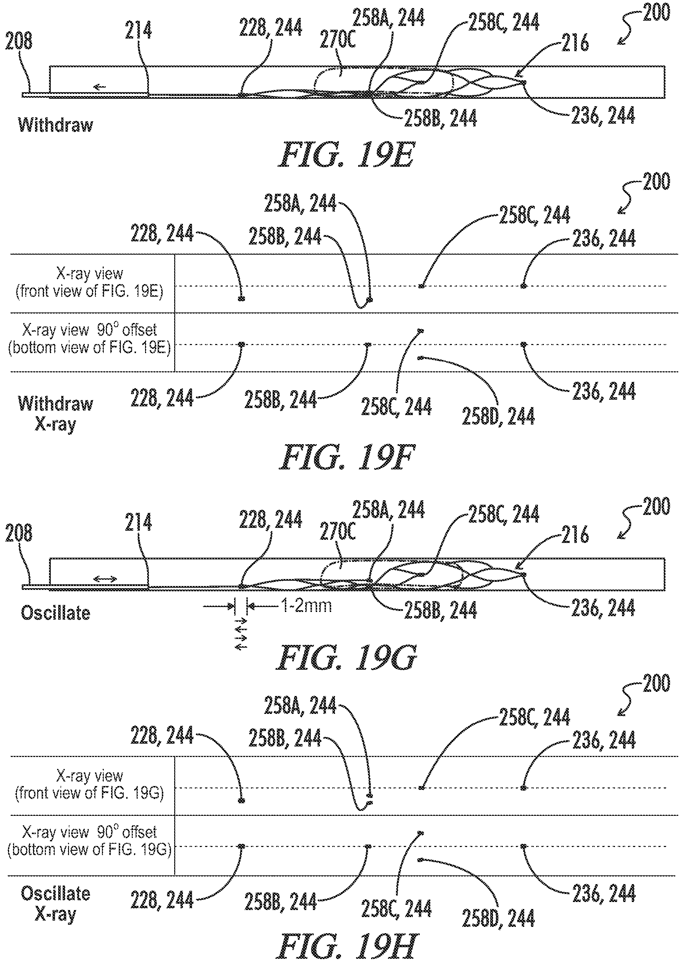

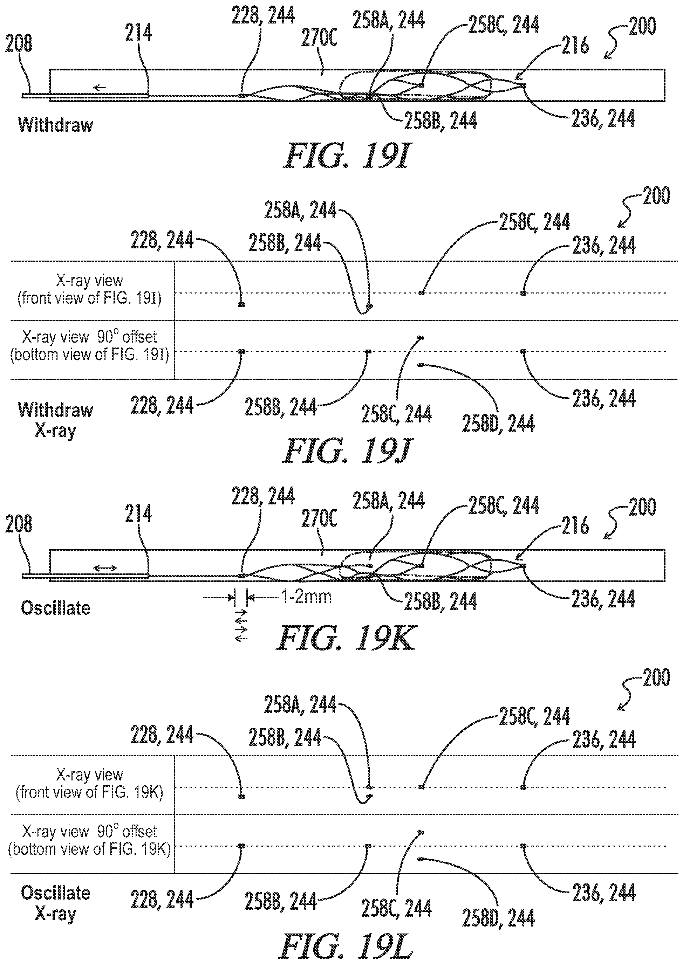

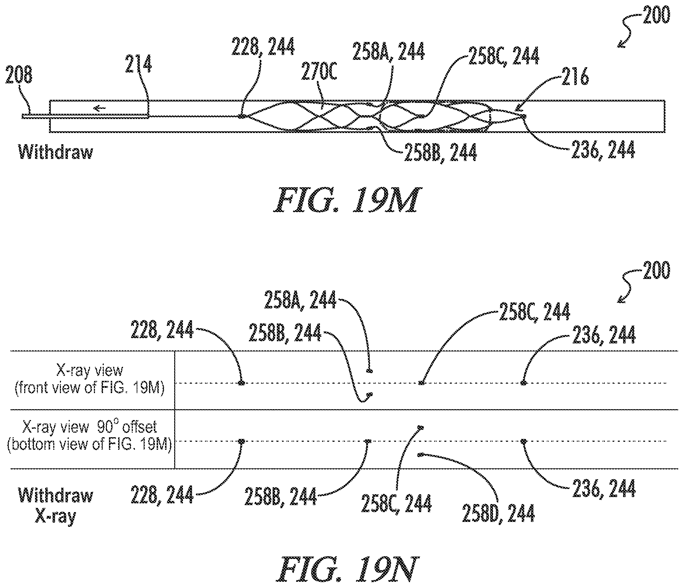

FIGs. 19A-N illustrate stepwise use of the distal body **216** in retrieving a deformable cohesive, adherent clot **270C**- i.e., a clot that is difficult to break up and is tightly adhered to the vessel wall **268** - in a human intracranial artery **266.** (In FIGS. 19A-N, the distal body **216** is in Orientation 2). First, as always, the surgeon determines the location of the clot **270C** in the vessel **266** using, for example, a contrast dye injected proximal and distal to the clot **270C.** Next, the delivery catheter **208**, which is enveloping the distal body **216**, is positioned in the blood vessel **266** so that the two proximal, unattached distal-pointing crowns **258A** and **258B** are immediately distal to the clot **270C.** See FIG. 19B. The distal body **216** is then deployed from the catheter **208** by moving the catheter **208** proximally. The deformable, cohesive adherent clot **270C**, which is located above the distal body **216**, collapses the distal body **216**, as shown in FIG. 19C. However, at this time, the surgeon is unaware that the clot **270C** has collapsed the distal body **216.** Thus, without moving the distal body **216**, the surgeon irradiates the x-ray markers at a first vantage point (i.e., from the front of the distal body; i.e., into the page). As shown in FIG. 19D, the first vantage point shows four rows of x-ray markers. The first row is, as always, a single point, representing the x-ray marker located in the proximal tube **228, 244.** The second row has a single point, corresponding to the top (in Orientation 2) and bottom (in Orientation 2), proximal, unattached distal-pointing crowns **258A, 244** and **258B, 244**, which have converged because the clot **270C** is collapsing the distal body **216.** The third row has a single point, which represents the x-ray marker located at the front (in Orientation 2), distal, unattached distal-pointing crown **258C, 244**; the x-ray marker located at the rear, distal, unattached distal-pointing crown **258D, 244** is hidden from view. The fourth row is, as always, a single point, representing the x-ray marker located in the distal tube **236, 244.** Without moving the distal body **216**, the surgeon then irradiates the markers from a second vantage point 90 degrees offset from the first vantage point (i.e., from the bottom of the distal body). As shown, the first row is, as always, a single point, which represents the x-ray marker located in the proximal tube **228, 244.** The second row has a single point, which corresponds to the bottom (in Orientation 2), proximal, unattached distal-pointing crown **258B, 244;** the top (in Orientation 2), proximal, unattached distal-pointing crown **258A, 244** is located behind the bottom, proximal, unattached distal-pointing crown **258B, 244** and hidden from view. The third row has two points, which correspond to the front (in Orientation 2) **258C, 244** and rear **258D, 244** (in Orientation 2), distal, unattached distal-pointing crowns, neither of which is blocked in this view. The fourth row is, as always, a single point, which represents the x-ray marker located in the distal tube **236, 244.** As shown in FIG. 19E, the surgeon then moves the distal body **216** proximally (i.e., slightly withdraws the distal body **216**). The surgeon then irradiates the x-markers again from the first and second vantage points. As shown in FIG. 19F, the results are exactly the same as in FIG. 19D. Based on the observation that the proximal, unattached distal-pointing crowns **258A, 244** and **258B, 244** have converged at both the original position (FIGs. 19C and 19D in which the proximal, unattached distal-pointing crowns **258A, 244** and **258B, 244** are immediately distal to the clot **270C**) and the second position (FIGs. 19E and 19F), the surgeon concludes that the clot **270C** is a deformable cohesive, adherent clot **270C.** The surgeon then oscillates the distal body **216** proximally and distally a small distance (e.g., about 1mm to about 2 mm) in the vessel **266**, and the clot **270C** begins to enter the distal body **216**, as shown in FIG. 19G. The surgeon then irradiates the x-markers again from the first and second vantage points. As shown in FIG. 19H, the results are exactly the same as in FIG. 19D and FIG. 19F except that the second row of markers **258A, 244** and **258B, 244** (at the proximal, unattached distal-pointing crowns) are beginning to move apart. The surgeon then moves the distal body **216** proximally again, as shown in FIG. 19I. The surgeon then irradiates the x-markers again from the first and second vantage points. As shown in FIG. 19J, the results are exactly the same as in FIGs. 19D and 19F, as the clot **270C** has caused the second row of markers **258A, 244** and **258B, 244** to re-converge. The surgeon then oscillates the distal body **216** proximally and distally a small distance (e.g., about 1mm to about 2 mm) in the vessel **266**, and the clot **270C** begins to further enter the distal body interior **222**, as shown in FIG. 19K. The surgeon then irradiates the x-markers again from the first and second vantage points. As shown in FIG. 19L, the results are the same as in FIG. 19H. The surgeon then moves the distal body **216** again proximally, and, instead of collapsing the second row of markers **258A, 244** and **258B, 244,** the clot **270C** fully enters the distal body interior **222**, as shown in FIG. 19M. The surgeon then irradiates the x-markers again from the first and second vantage points. As shown in FIG. 19N, the results show that the second row of markers **258A, 244** and **258B, 244** (at the proximal, unattached distal-pointing crowns) have moved apart. Satisfied that the x-ray markers in the second row **258A, 244** and **258B, 244** (at the proximal, unattached distal-pointing crowns) are sufficiently far apart and that the x-ray markers in the third row (at the distal, unattached distal-pointing crowns) **258C, 244** and **258D, 244** have stayed far apart, the surgeon concludes that the deformable cohesive, adherent clot **270C** has been sufficiently captured by the distal body **216** and the surgeon then removes the distal body **216** and the clot **270C**, captured by the distal body **216**, by moving the distal body **216** proximally out of the vessel **266.**

Several observations can be made from FIGs. 15-19, as indicated above. For example, the x-ray markers at the proximal and distal, unattached distal-pointing crowns **258A-D, 244** provide the surgeon feedback concerning the interaction between the distal body **216** and the clot **270** in the blood vessel **266.** In addition, the guiding principle of a soft clot **270A** is that the soft clot **270A** does not collapse the distal body **216**, and thus, x-ray markers at the proximal and distal, unattached distal-pointing crowns **258A-D, 244** always appear as two points except when a marker is hidden behind another marker (due to the view). When it comes to a hard clot **270B,** the hard clot **270B** is generally able to enter the distal body interior **222** without needing to oscillate the distal body **216** proximally and distally (unlike a deformable cohesive, adherent clot **270C**). However, to capture the hard clot **270B,** the hard clot **270B** must be oriented properly relative to the enlarged cell/drop zones **262A, 262B, 262C,** or **262D.** (This is the reason that the distal body **216** has four enlarged cells/drop zones: one enlarged cells/drop zone at 0 degrees **262B**, one enlarged cells/drop zone at 90 degrees **262C,** one enlarged cells/drop zone at 180 degrees **262A** and one enlarged cells/drop zone at 270 degrees **262D**). As a guiding principle, an enlarged cell/drop zone **262A, 262B, 262C,** or **262D** is properly oriented to the clot **270B** when the x-ray markers at the proximal, unattached distal-pointing crowns **258A, 244** and **258B, 244** or the distal, unattached distal pointing crowns **258C, 244** and **258D, 244** are together at both a first x-ray view and a second x-ray view 90 degrees relative to the first x-ray view, and the hard clot **270B** can enter the enlarged cell/drop zone **262A, 262B, 262C,** or **262D** by moving the distal body **216** proximally. See FIG. 16F and 18D. Finally, the guiding principal of retrieval of deformable cohesive, adherent clots **270C** is that oscillation of the distal body **216** causes the deformable cohesive, adherent clots **270C** to gradually enter the distal basket interior **222** over time.

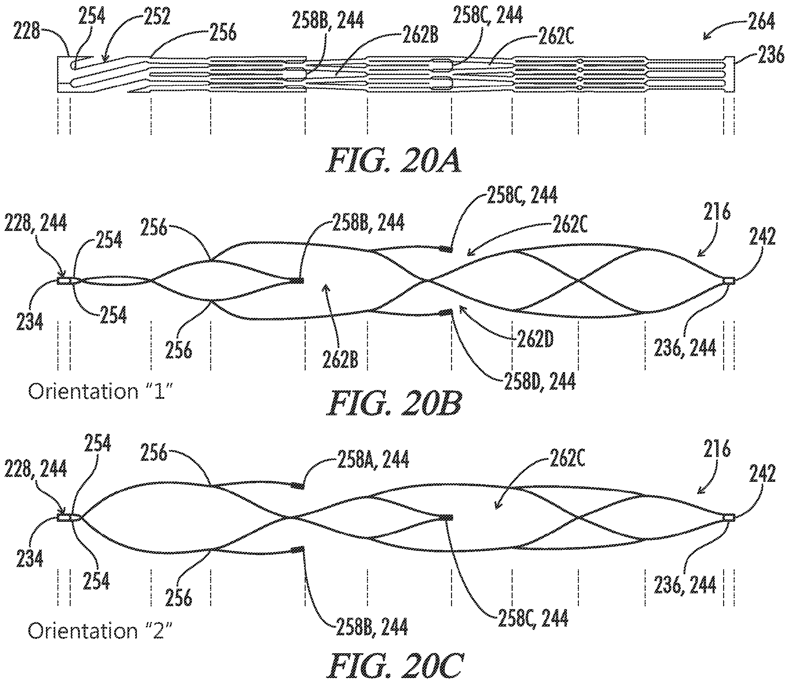

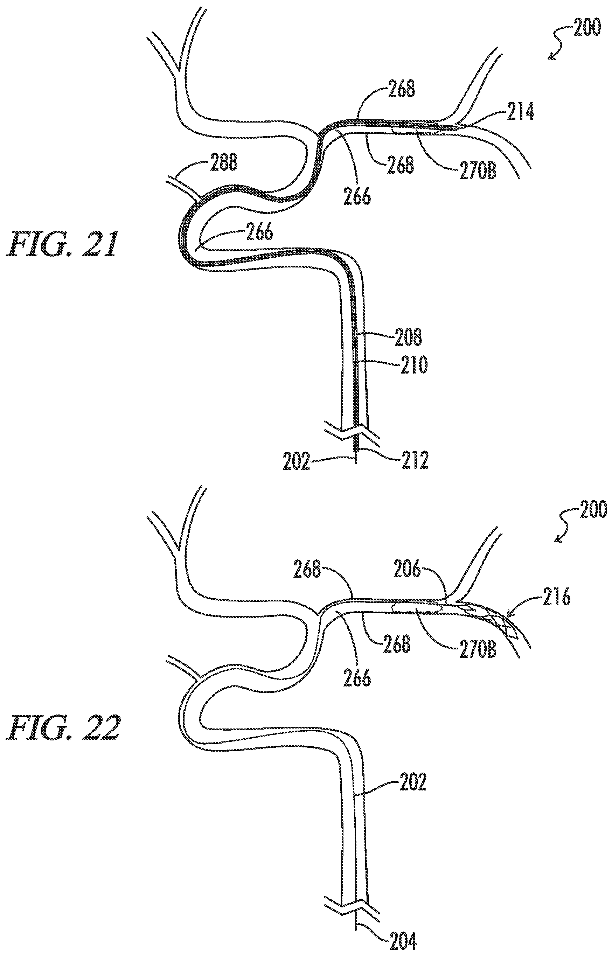

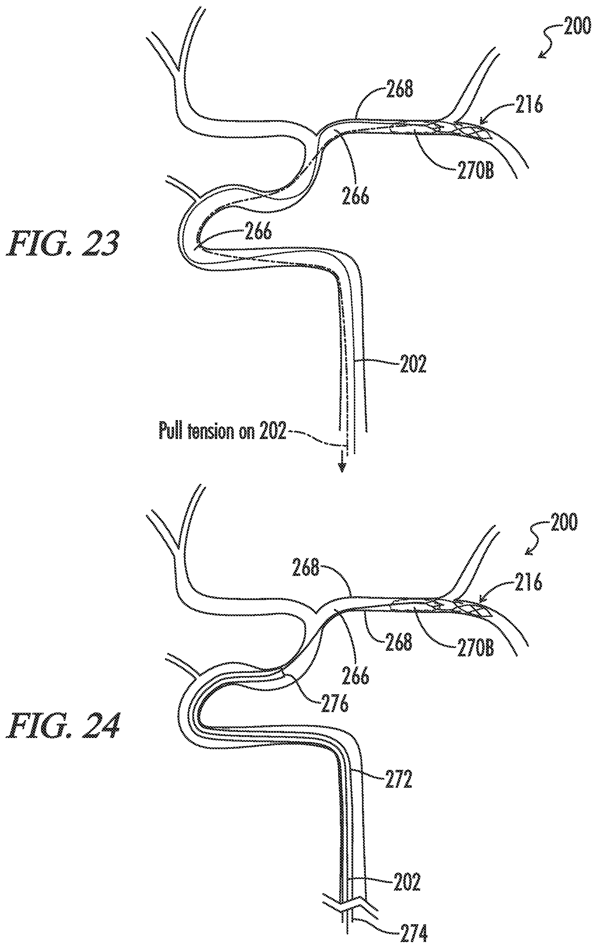

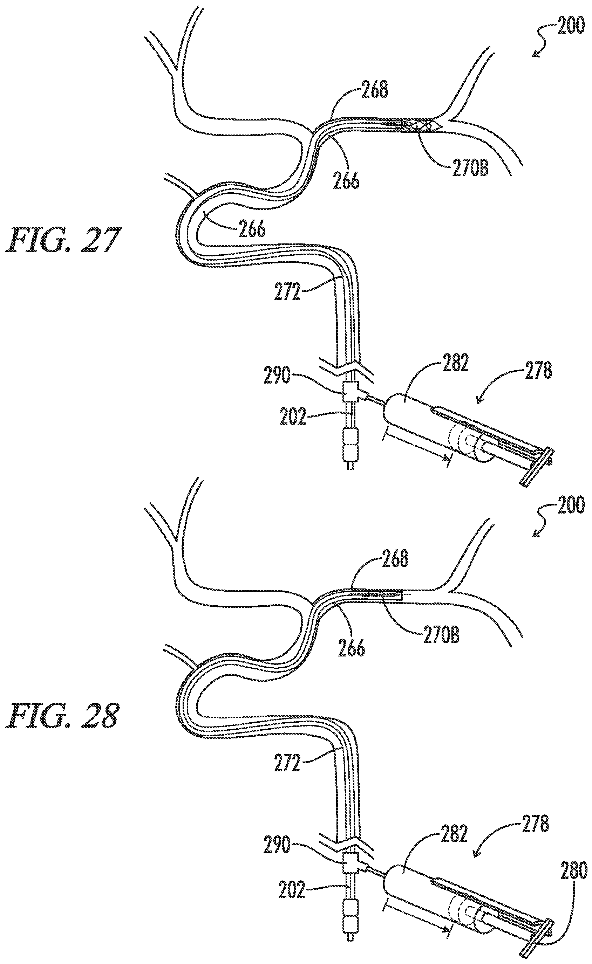

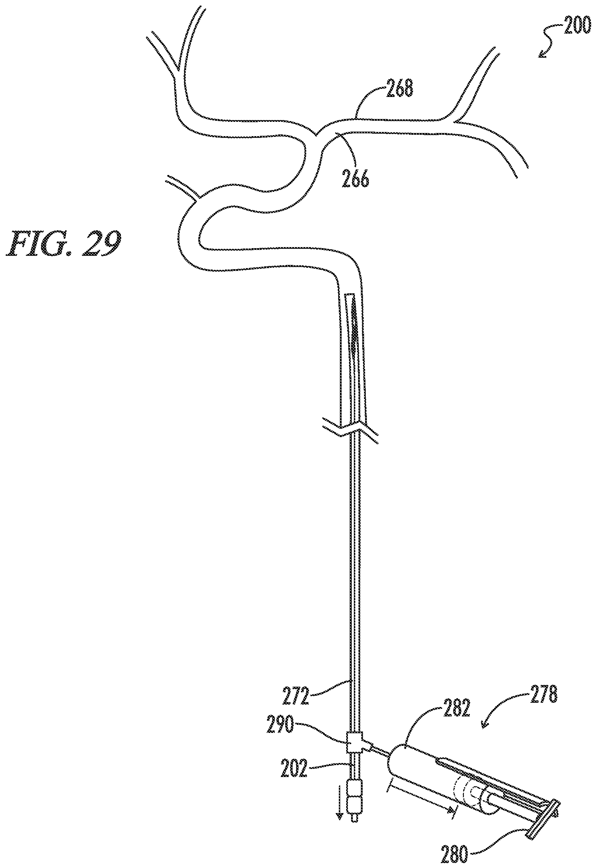

FIGs. 20A, 20B and 20C show a distal body **216** that is similar to the distal body **216** of FIGs. 14A, 14B and 14C except that the distal body **216** of FIGs. 20A, 20B and 20C is slightly shorter and its unattached, distal-pointing crowns **258A, 258B, 258C,** and **258D** are closer to the proximal tube **228.** The shortened distal body **216** of FIGs. 20A, 20B and 20C is particularly adapted for tortuous blood vessels **266.** FIG. 21-29 show stepwise deployment of the distal body **216** of FIGs. 20A, 20B and 20C in use with a manual (i.e., hand-operated), volume-dependent (i.e. volume locked) suction catheter **272** that is locked at between about 10 to about 60 cubic centimeters (cc). Optionally, the suction catheter **272** has an outer diameter of between about 1.27 mm (0.05 inches) and about 2.3 mm (0.09 inches) and its outer diameter is substantially larger than the outer diameter of the delivery catheter **208.** The clot **270** is located in the vessel **266** through the use of, for example, contrast dye injected proximal and distal to the clot **270.** As shown in FIG. 21, a delivery catheter **208** containing the distal body **216** of FIGs. 20A, 20B and 20C is positioned in the tortuous vessel **266** distal to the clot **270.** The delivery catheter **208** is withdrawn, deploying the distal body **216.** See FIG. 22. The distal body **216** is moved proximally relative to the clot **270** and tension is exerted on pull wire **202.** See FIG. 23. While maintaining tension on the pull wire **202**, a suction catheter **272** having a proximal end **274** and a distal end **276** is delivered over the pull wire **202** that is attached to the distal body **216.** See FIG. 24. (The reason for exerting tension on the pull wire **202** is that the pull wire **202** serves as the guide/track for the movement of the suction catheter **272** and without tension, the suction catheter **272** and pull wire **202** could end up in the ophthalmic artery **288**). The distal end **276** of the suction catheter **272** is positioned against the clot **270.** A syringe **278** is attached to the suction catheter **272** using a rotating hemostatic valve **290**, which allows the surgeon to aspirate while a pull wire **202** is in the system. The surgeon aspirates the syringe **278** by pulling back on the lever **280** to a mark on the base **282** corresponding to between about 10 and about 60 cubic centimeters of fluid. The surgeon then locks the lever **280** (and attached plunger) into place, leaving the suction catheter **272** under suction. The surgeon captures the clot **270** in the distal body **216** using the techniques described in FIGs. 15-19. The distal body **216** and clot **270** become captured by the suction catheter **272.** See FIGs. 27 and 28. The surgeon then removes the suction catheter **272** and the distal body **216** and the clot **270**, captured by the suction catheter **272**, by moving the suction catheter **272** proximally out of the vessel **266.** See FIG. 29. It is believed that the suction catheter **272** would be helpful in the event that a small portion of the clot **270** breaks off when retrieving the clot **270** using the distal body **216.**

To examine effectiveness of the systems **200**, the systems **200** of FIGs. 11-20, without the use of a suction catheter **272**, were used to retrieve soft and hard clots **270A** and **270B** induced in a pig weighing between 30 to 50 kg. The weight of the pig was chosen so that the size of its vessels **266** would be approximate to the size of a human vessel. The pig was anesthetized. Several hard clots **270B** were prepared by mixing pig blood and barium and incubating the mixture for 2 hours. Several soft clots **270A** were prepared by mixing pig blood, thrombin and barium and incubating the mixture for 1 hour. The clots **270A** and **270B**, each of which had a width of 4 to 6 mm and a length of 10 to 40 mm, were then inserted into a vessel **266** having a diameter of 2 to 4 mm. (Only one clot **270A** and **270B** was located in the vessel **266** at a time). Angiograms were then performed to confirm occlusion. After waiting ten minutes after confirming occlusion, the distal bodies **216** of FIGs. 11-20 were then delivered distal to the clots **270A** and **270B** as described above and were used to retrieve the clots **270A** and **270B** as described in FIGs. 11-19. In each case, the distal bodies **216** were successful in retrieving the clots **270A** and **270B.**

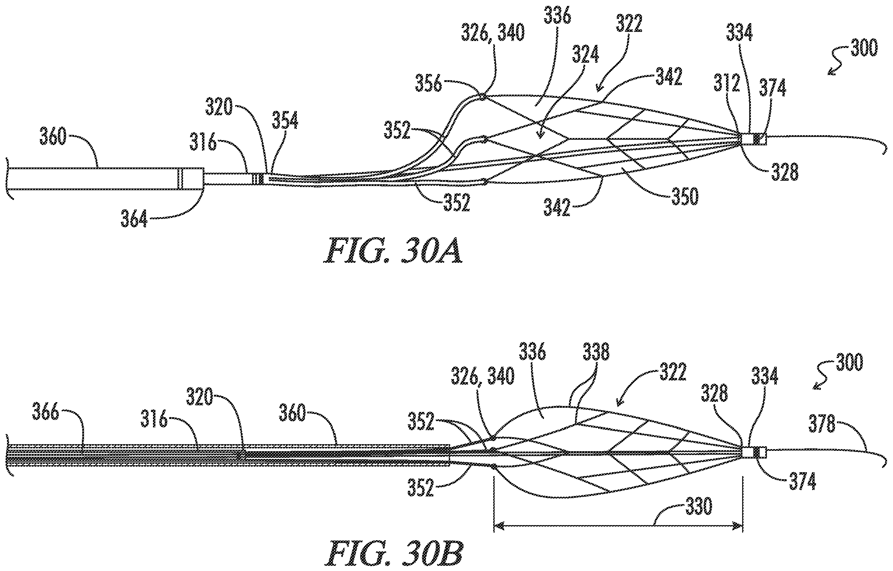

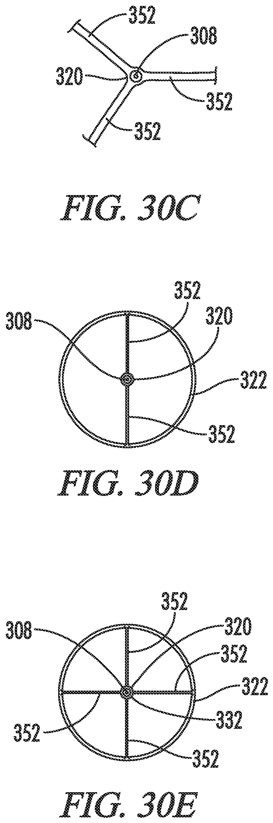

### The Embodiments of FIGs. 30-35

Figures 30-35 illustrate additional embodiments of object retrieval system. Optionally, the system **300** of FIGs. 30-35 includes:
a pull wire **308** having a proximal end **310**, a distal end **312** and a pull wire longitudinal axis **314** extending from the proximal end **310** to the distal end **312**;
a coaxial sheath/tube **316** having a hollow interior, an open proximal end **318** leading to the hollow interior, and an open distal end **320** leading to the hollow interior, the coaxial sheath **316** enveloping the pull wire **308**, the coaxial sheath **316** slideable along at least a segment of the pull wire **308**;
a distal basket **322** comprising an interior **324**, a proximal end **326**, a distal end **328**, a distal basket length **330** extending from the distal basket proximal end **326** to the distal basket distal end **328**, a distal basket height **332** perpendicular to the distal basket length **330**, a plurality of proximal cells **336** defined by a plurality of proximal cell memory metal strips **338**, each proximal cell **336** comprising a proximal crown **340** located at the proximal end of the proximal cell **336** and pointing generally in the proximal direction and a distal crown **342** located at the distal end of the proximal cell **336** and pointing generally in the distal direction, and a plurality of distal cells **350** distal to the proximal cells **336**;
a plurality of proximal strips **352**, each proximal strip **352** having a proximal end **354** extending from the coaxial sheath distal end **320**, a distal end **356** attached to a proximal crown **340** of a proximal cell **336** and a length **358** extending from the proximal end **354** to the distal end **356**; and
a delivery catheter **360**, as described above, and having a hollow interior **366**, a proximal end **362** leading to the interior **366** and a distal end **364** leading to the interior **366**, the delivery catheter **360** comprised of a biocompatible material.

Optionally, the distal basket **322** is comprised of a memory metal and has:
a relaxed state in which the distal end **320** of the coaxial sheath **316** is located a first distance proximal to the proximal crowns **336** and wherein the distal basket **322**, as measured at the proximal-most crown **336**, has a first height,
a proximal collapsed state in which the distal end **320** of the coaxial sheath **316** is located a second distance proximal to the proximal crowns **336** and wherein the distal basket **322**, as measured at the proximal-most crown **336**, has a second height, the second distance greater than the first distance, the second height less than the first height, and
a distal collapsed state in which the distal end **320** of the coaxial sheath **316** is located distal to the proximal crowns **336** and in the basket interior **324** and wherein the distal basket **322**, as measured at the proximal-most crown **336**, has a third height, the third height less than the first height,
wherein the delivery catheter **366** is configured to envelope the distal basket **322** when the distal basket **322** is in the proximal collapsed state;
wherein the distal basket **322** is configured to move from the relaxed state to the proximal collapsed state by moving the distal end **320** of the coaxial sheath **316** proximally relative to the proximal crowns **336**; and
wherein the distal basket **322** is configured to move from the relaxed state to the distal collapsed state by moving the distal end **320** of the coaxial sheath **316** distally beyond the proximal crowns **336** and into the distal basket interior **324.**

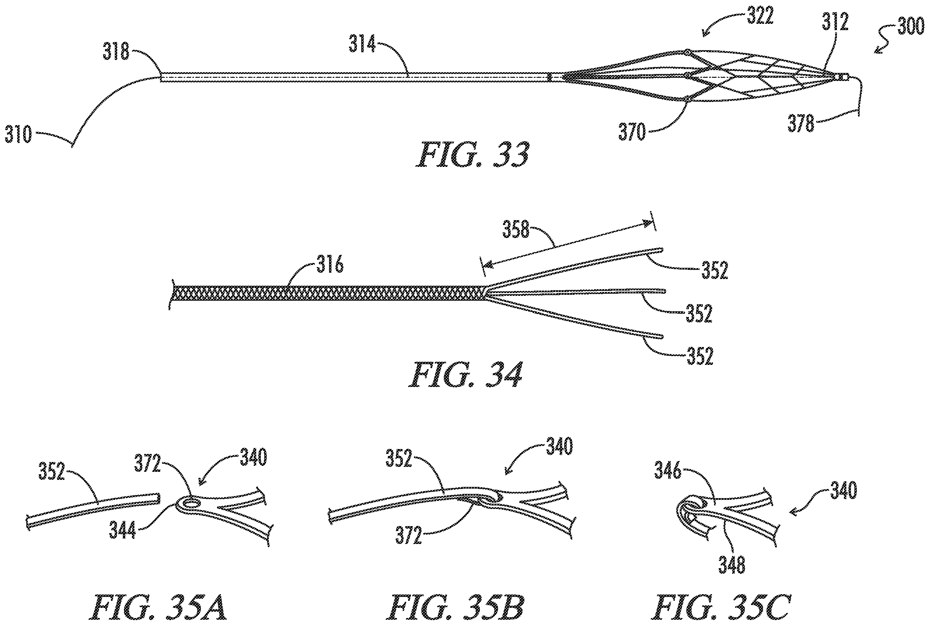

Optionally, each proximal crown **340** comprises a proximal tip **344** and further wherein each proximal strip **352** is configured to cover a proximal tip **344** when the distal basket **322** is in the distal collapsed state. See FIG. 35C, where the proximal strip **352** is folding back on itself to cover the proximal tip **344.** Optionally, each proximal crown **340** comprises an eyelet **370** and further wherein each proximal strip **352** passes through an eyelet **370.** Optionally, the distal end **356** of each proximal strip **352** comprises a loop **372** attaching the proximal strip **352** to an eyelet **370.** Optionally, each proximal crown **340** has an interior surface **348** facing the distal basket interior **324** and an exterior surface 350 opposite the interior surface **348** and further wherein each proximal strip **352** contacts an exterior surface **350** of a proximal crown **340** in the proximal collapsed state and the distal collapsed states, as best seen in FIGs. 35A-C. Without being bound to any particular theory, it is believed that threading the proximal strips **352** through the eyelets **370** as shown in FIGs. 35A-35C, helps protect the proximal crowns **340** (in particular, the proximal tips **344** of the proximal crowns **340**) from damaging the vessel wall **306** when the proximal crowns **340** move towards each other and the pull wire **308** when the distal basket **322** moves to the distal collapsed state and the proximal collapsed state. Optionally, the pull wire **308** extends through the distal basket interior **324** and further wherein the proximal crowns **340** are configured to move towards each other and towards the pull wire **308** when the distal basket **322** moves from the gaping state to the distal collapsed state. Optionally, the proximal crowns **340** are configured to remain a fixed distance from the distal end **328** of the distal basket **322** when the distal basket **322** moves from the relaxed state to the distal collapsed state. In other words, preferably, the distal basket length **330** does not change when the distal basket **322** moves from the distal basket relaxed state to the distal basket. Optionally, the coaxial sheath **316** is a braided catheter comprised of a plurality of braids and further wherein the proximal segments of the braids are wound/woven together to form the braided catheter and further wherein an unwound/unwoven distal segment of each braid forms a proximal strip **352**, as shown in FIG. 34. Optionally, at least one component of the system **300** (e.g., the proximal crown **340** or the distal tube **334**) comprises an x-ray marker **374** that is more visible under x-ray as compared to the other components when the distal basket **322** is located in a cranial blood vessel **304** inside the body of a human and the x-ray is taken from outside the human's body. Preferably, the x-ray marker **374** is a radiopaque material. Some examples of radiopaque materials can include, but are not limited to, gold, platinum, palladium, tantalum, tungsten alloy, polymer material loaded with radiopaque filler, and the like. Preferably, the non x-ray marker components are comprised of nitinol and the x-ray marker **374** is comprised of a material having a density greater than the nitinol. In some embodiments, as shown in FIGs. 30A, 30B, 31A, 31B, 32A-F, the proximal ends **354** of the proximal strips **352** are integral with the coaxial sheath **316.** In other embodiments, as shown in FIG. 33, the proximal ends **354** of the proximal strips **352** are attached to the coaxial sheath **316.** Optionally, the system **300** comprises between two and four proximal strips **352** and the proximal strips **352** are spaced substantially evenly apart (e.g., if there are two proximal strips **252**, the strips are located about 180 degrees relative to each other, as shown in FIG. 30D; if there are three proximal strips **252**, the strips are located about 120 degrees relative to each other, as shown in FIG. 30C; and if there are four proximal strips **252**, the strips are located about 1200 degrees relative to each other, as shown in FIG. 30E). Optionally, the proximal strips **352** have a length **358** of from about 5 mm to about 40 mm in the relaxed state. Optionally, the pull wire **308** extends through the basket interior **324** from the distal basket proximal end **326** to the distal basket distal end **328.** Optionally, the coaxial sheath interior has a size and shape, and further wherein the size and shape of the coaxial sheath interior are configured to prevent a segment **376** of the pull wire **308** located in the basket interior **322** and distal relative to the distal end **320** of the coaxial sheath **316** from moving through the coaxial sheath interior. In other words, optionally the pull wire **308** has a stop **376** that consists of a knot or other enlargement. Optionally, the distal end **328** of the distal basket **322** comprises a distal tube **334** having an open proximal end and an open distal end, the distal tube **334** comprised of a memory metal. Optionally, the distal tube **334** is attached to the pull wire **308** so that the distal tube **334** is not slideable along the pull wire **308.** This allows the entire distal basket **322** to be fixed to (i.e., not slideable along)the pull wire **308.** Optionally, wherein all proximal crowns **340** of the proximal cells **336** are attached to a proximal strip **352**, which is designed to minimize damage to the vessel wall **306.** Optionally, the distal basket **322** further comprises a lead wire **378** extending distally from the distal basket **322.** Optionally, the proximal strips **352** and the distal basket **322** have a different material composition. In other words, whereas the proximal strips **352** are designed to be soft, preferably, the distal basket **322** is comprised of a memory metal such as nitinol. Optionally, the proximal strips **352** are comprised of a polymer, which as used herein includes a co-polymer. Optionally, the polymer is selected from the group consisting of fluorinated ethylene propylene, polytetrafluoroethylene, and tetrafluoroethtylene. Optionally, the proximal strips **352** are comprised of a material selected from the group consisting of plastic, rubber, nylon, suture material, and braided catheter material.

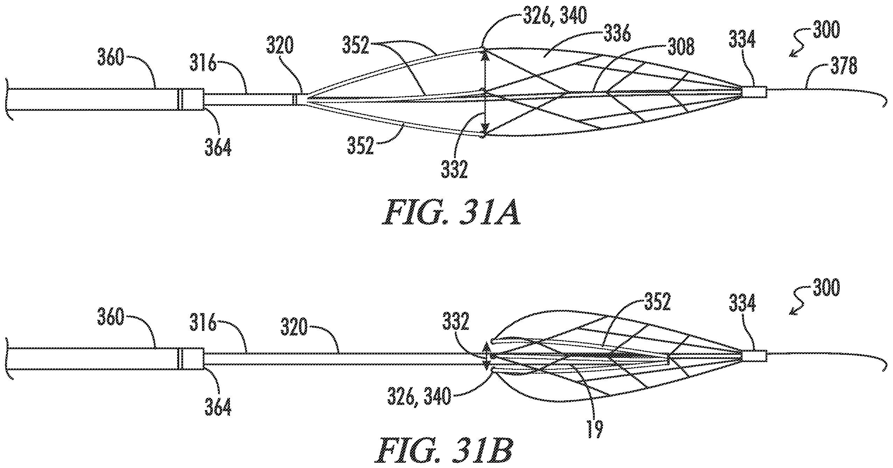

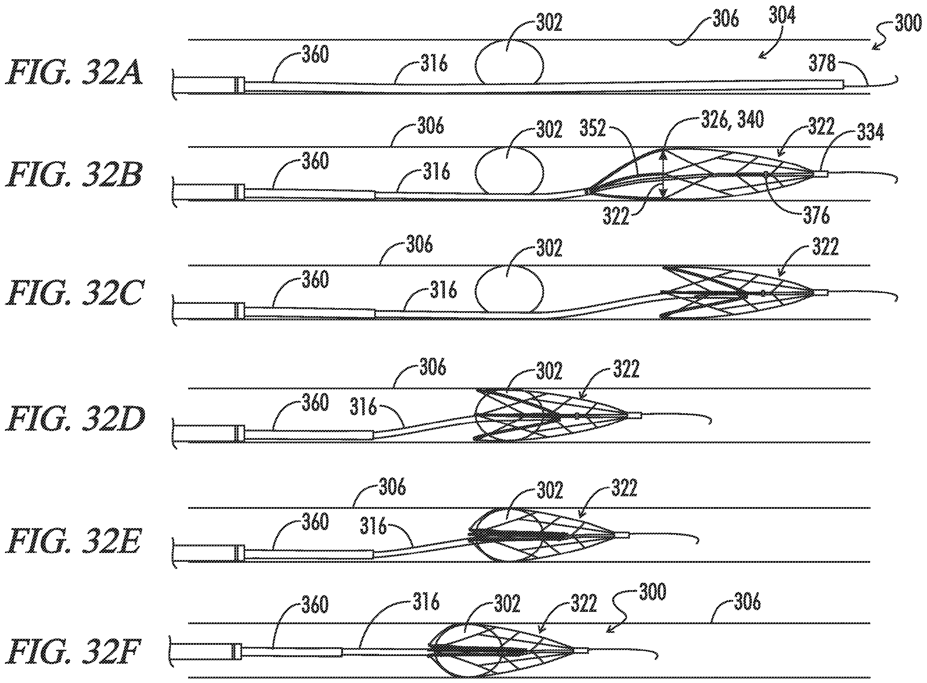

Optionally, as illustrated in FIGs. 32A-32F, the system **300** is used in an exemplary method of removing a clot **302** from a blood vessel **304** of an animal, the blood vessel **304** having an interior wall **306** forming the blood vessel **304**, the exemplary method comprising the steps of:
a) providing the system **300**, wherein the coaxial sheath **316** is located in the catheter interior **366** and the distal basket **322** is located in the catheter interior **366** in a collapsed state;
b) positioning the catheter **360** in the blood vessel **304** (see FIG. 32A);
c) deploying the distal basket **322** from the distal end **364** of the catheter **360** so that the proximal crowns **340** of the proximal cells **336** are distal to the clot **302**;
d) allowing the distal basket **322** to move to the relaxed state (see FIG. 32B; the coaxial sheath **316** is in the first position along the pull wire **308**);
e) moving the distal end **320** of the coaxial sheath **316** distally along the pull wire **308** to the fourth position (see FIG. 32C; note that the proximal crowns **340** have remained in the same location and that the distal basket height **332**, as measured at the proximal-most crown **340**, has not decreased yet; preferably, an x-ray marker **374** on the pull wire **308** allows the surgeon to locate the fourth position);
f) moving the distal basket **322** and the coaxial sheath **316** proximally and capturing the clot **302** in the distal basket interior **324** (see FIG. 32D);
g) moving the coaxial sheath **316** further distally along the pull wire (i.e., at or near the third position; preferably, an x-ray marker **374** on the pull wire **308** allows the surgeon to locate the third position) so that the distal basket height **332**, as measured at the proximal-most crown **340**, decreases and the proximal crowns **340** move toward each other and towards the pull wire **308** (see FIGs. 32D and 32E; it will be appreciated that the proximal crowns **340** collapse like a claw in FIGs. 31B, 32D and 32E due to tension exerted on the crowns **340** by the proximal strips **352**, similar to the mechanism described in FIGs. 3-10); and
h) moving the system **300** proximally out of the blood vessel **304.**

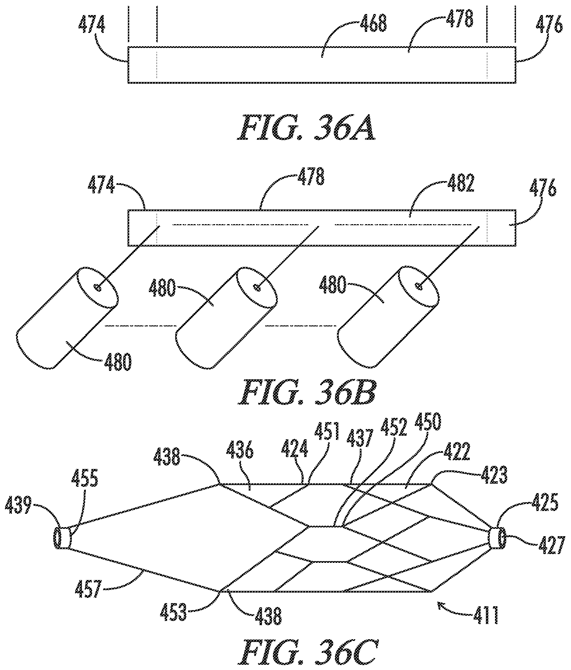

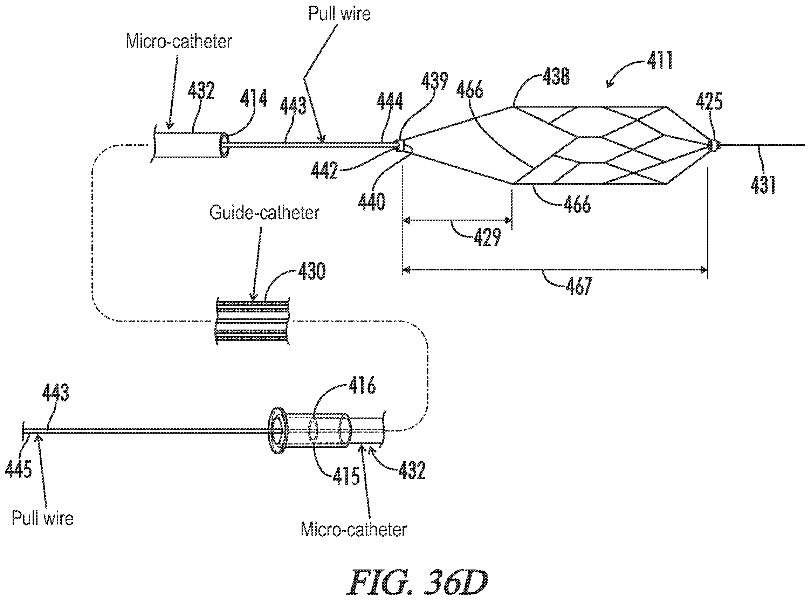

### The Embodiments of Figures 36-44

Figures 36-44 further illustrate other embodiments of a modular, easy-to-manufacture platform of systems for retrieving hard clots and other objects in animal lumens. In some embodiments, the system includes a proximal tube, a distal tube, and a plurality of memory metal strips between the proximal and distal tubes. The plurality of memory metal strips form a wide range of basket designs. Preferably, the proximal tube, memory metal strips, and distal tube are derived from a standard, off-the-shelf single tube of memory metal (e.g., nitinol), with the proximal tube and distal tube having the same inner diameter and outer diameter as the native tube from which they were derived and with the basket formed by cutting the middle portion of the native tube and expanding and shape-setting this cut portion. Preferably, the proximal tube and distal tube have an outer diameter that is from about 0.51 mm (0.02 inches) to about 0.76 mm (0.03 inches) (e.g., about 0.69 mm (0.027 inches)) so that the device fits inside a standard microcatheter and an inner diameter that is from about 0.25 mm (0.01 inches) to about 0.51 mm (0.02 inches). Preferably, there are no welded parts between the proximal tube and distal tube, which makes the system easy and cheap to reliably manufacture. The system also includes one or more catheters for deploying the system, and a first wire that is attached to the proximal tube and a second wire that is attached to the distal tube. Preferably, the system includes two catheters - a guide catheter and a microcatheter. The plurality of memory metal strips attached to the proximal hub include a plurality of proximal tether memory metal strips, which have a proximal end attached to the distal end of the proximal tube.

The present disclosure also provides a system for removing objects within an interior lumen of an animal. In some embodiments, the system includes
a pull wire having a proximal end, a distal end and a pull wire longitudinal axis extending from said proximal end to said distal end;
a distal basket attached to said pull wire, said distal basket comprising a proximal end, a distal end, a distal basket length extending from said distal basket proximal end to said distal end, a distal basket height perpendicular to said distal basket length and said pull wire longitudinal axis, a proximal tube located at said proximal end of the distal basket, said proximal tube comprising a hollow interior, a plurality of proximal tether memory metal strips, a row of proximal cells defined by a plurality of proximal cell memory metal strips, each proximal cell comprising a proximal crown located at the proximal end of the proximal cell and pointing generally in the proximal direction, each proximal tether memory metal strip having a proximal end attached to said proximal tube, a distal end attached to a crown of a proximal cell and a length extending from said proximal end to said distal end, a row of distal crowns located distal to said proximal cells pointing in the distal direction, and further wherein the number of distal crowns in said row is twice the number of proximal crowns attached to said proximal tether memory metal strips, and a distal tube located at said distal end of said distal basket,
said distal basket having
a relaxed state wherein said distal basket has a first height and
a collapsed state wherein said distal basket has a second height, said second height less than said first height, and
a catheter having an interior, a proximal end leading to said interior and a distal end leading to said interior, said catheter comprised of a biocompatible material and configured to envelope said distal body when said distal basket is in said collapsed state.

Optionally, said proximal tether memory metal strips rotate about said pull wire longitudinal axis such that a distal end of a proximal tether memory metal strip is located between about 90 and about 270 degrees relative to said proximal end of the same proximal tether memory metal strip. Optionally, said proximal tether memory metal strips and said proximal cell memory metal strips each have a thickness and further wherein said thickness of said proximal tether memory metal strips is between about 100 to about 175 percent of the thickness of the proximal cell memory metal strips. Optionally, the length of said proximal tether memory metal strips is about 10 mm to about 20 mm in the relaxed state (and the length of the remainder of the basket is about 10 to about 20 mm in the relaxed state so that the total basket length is between about 20 to about 40 mm in the relaxed state). Optionally, said distal end of said pull wire is attached to said proximal tube. Some or all of the proximal crowns of said proximal cells may be attached to a proximal tether memory metal strip. Optionally, said distal basket further comprises a row of strut memory metal strips, each strut memory metal strip having a proximal end attached to a distal crown of a proximal cell and a distal end attached to a proximal crown of a distal cell. Optionally, the distal basket comprises between two and four proximal tether memory metal strips. Optionally, said proximal tether memory metal strips are integral with said proximal tube. Optionally, said distal body further comprises a lead wire extending distally from said distal tube. Optionally, said distal tube, said proximal tube, and said basket are comprised of a nitinol having the same material composition. Optionally, said distal body further comprises an x-ray marker. Optionally, said proximal and said distal tubes are generally cylindrical in shape and each has an outer diameter and an inner diameter, the inner diameter forming the apertures of the proximal and distal tubes and further wherein the outer diameters of the proximal and distal tubes are substantially the same size and further wherein the inner diameters of the proximal and distal tubes are substantially the same size. Optionally, the outer diameters of the proximal and distal tubes are from about 0.28 mm (0.011 inches) to about 1.37 mm (0.054 inches), and further wherein the inner diameters of the proximal and distal tubes are from about 0.20 mm (0.008 inches) to about 1.30 mm (0.051 inches). Optionally, the pull wire is generally cylindrical and further wherein the diameter of the pull wire is between about 0.20 mm (0.008 inches) and about 1.30 mm (0.051 inches). Optionally, the first height is between about 2 millimeters and about 8 millimeters.

The present disclosure also provides an exemplary method of removing an object from an interior lumen of an animal, said lumen having an interior wall forming said lumen, the exemplary method comprising the steps of:
a) providing the system described above;
b) positioning the system in said lumen, said basket located in said catheter in said collapsed state;;
c) deploying said distal basket from said distal end of said catheter so that said proximal crowns of said proximal cells are distal to said obstruction;
d) allowing said distal basket to move to said relaxed state;
e) moving said distal basket over said obstruction; and
f) removing said distal basket and said obstruction from said lumen.

Optionally, said interior lumen is an intracranial artery and said obstruction is a blood clot.

In further embodiments, the system includes:
a pull wire having a proximal end, a distal end and a pull wire longitudinal axis extending from said proximal end to said distal end;
a proximal basket attached to said pull wire, said proximal basket comprising an interior, an exterior, a proximal end, a distal end, a proximal basket length extending from said proximal basket proximal end to said distal end, a proximal basket height perpendicular to said proximal basket length and said pull wire longitudinal axis, a proximal tube located at said proximal end of the proximal basket, said proximal tube comprising a hollow interior, a plurality of rows of cells, each cell defined by a plurality of memory metal strips, each cell comprising a proximal crown located at the proximal end of the proximal cell and pointing generally in the proximal direction and a distal crown located at the distal end of the proximal cell and pointing generally in the distal direction,
a distal basket attached to said pull wire, said distal basket comprising an interior, an exterior, a proximal end, a distal end, a distal basket length extending from said distal basket proximal end to said distal end, a distal basket height perpendicular to said distal basket length and said pull wire longitudinal axis, a distal tube located at said distal end of the distal basket, said distal tube comprising a distal tube aperture, a plurality of rows of cells, each cell defined by a plurality of memory metal strips, each cell comprising a proximal crown located at the proximal end of the proximal cell and pointing generally in the proximal direction and a distal crown located at the distal end of the proximal cell and pointing generally in the distal direction,
a plurality of tether memory metal strips, each tether memory metal strip having a proximal end attached to a distal crown of a cell located at the distal end of said proximal basket and a distal end attached to a proximal crown of a cell located at the proximal end of said distal basket,
said proximal basket having
a relaxed state wherein said proximal basket has a first height and
a collapsed state wherein said proximal basket has a second height, said second height less than said first height and said second width less than said first width,
said distal basket having
a relaxed state wherein said distal basket has a first height and a first width and
a collapsed state wherein said distal basket has a second height and a second width, said second height less than said first height, and
a catheter having an interior, a proximal end leading to said interior and a distal end leading to said interior, said catheter comprised of a biocompatible material and configured to envelope said distal and said proximal basket when said baskets are in said collapsed state.

Optionally, said tether memory metal strips rotate about said pull wire longitudinal axis such that a distal end of a tether memory metal strip is located between about 90 and about 270 degrees relative to said proximal end of the same proximal tether memory metal strip.

More particularly, with reference to FIGs. 36-44 the present disclosure provides a deployable system, generally designated by the numeral **410**, for removing an obstruction such as a blood clot **417** or other object from a blood vessel **488** or other interior lumen of an animal. In addition to a blood clot **417**, the obstruction may be, for example, extruded coils during aneurysm treatment, intravascular embolic material such as onyx or other obstructions requiring mechanical intravascular removal from small distal vessels. In the drawings, not all reference numbers are included in each drawing for the sake of clarity.

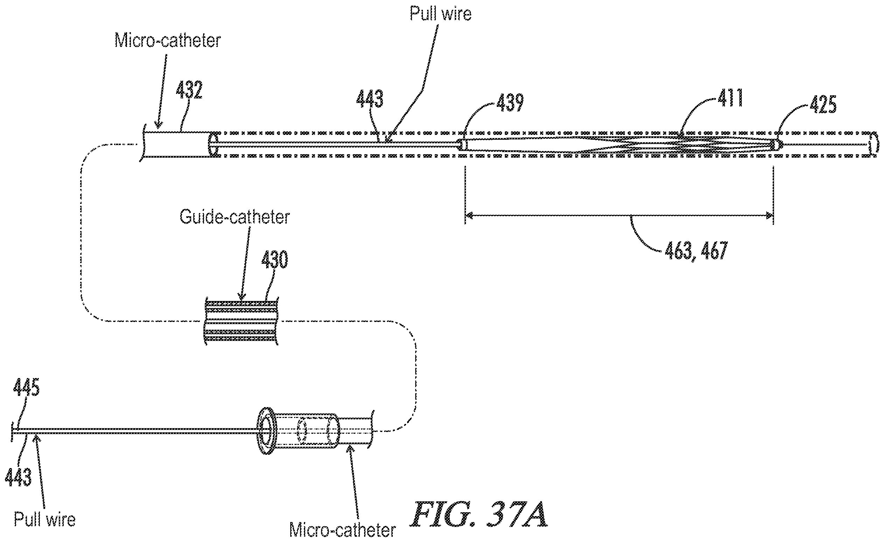

One example of a deployable basket system **410** is shown in FIGs. 37A-37B, 38A-E and 39A. As shown in FIGs. 31A-31E, 32G-32H and 35A, the system **410** includes a pull wire **443** having a proximal end **445**, a distal end **444** and a pull wire longitudinal axis **446** extending from said proximal end **445** to said distal end **444.** Optionally, the diameter of the pull wire **443** is between about 0.20 mm (0.008 inches) and about 1.30 mm (0.051 inches).

The system **410** further includes a distal basket **411** attached to said pull wire **443**, said distal basket **411** comprising a proximal end **469**, a distal end **465**, a distal basket length **467** extending from said distal basket proximal end **469** to said distal end **465**, a distal basket height **461** perpendicular to said distal basket length **467** and said pull wire longitudinal axis **446**, a proximal hub **439** located at said proximal end **469** of the distal basket **411** and comprising a hollow interior **441**, said distal end **444** of said pull wire **443** attached to said proximal hub **439**, a plurality of proximal tether memory metal strips **457**, a plurality of proximal cells **436** defined by a plurality of proximal cell memory metal strips **466**, each proximal cell **436** comprising a proximal crown **438** located at the proximal end of the proximal cell **436** and pointing generally in the proximal direction and a distal crown **424** located at the distal end of the proximal cell **436** and pointing generally in the distal direction, each proximal tether memory metal strip **457** having a proximal end **455** attached to said proximal hub **439** (preferably said proximal hub distal end **440**), a distal end **453** attached to a crown of a proximal cell **438** and a length **455** extending from said proximal end **455** to said distal end **453**, a plurality of distal cells **422** distal to the proximal cells **436**, and a distal hub **425** located at said distal end **465** of said distal basket, comprising a hollow interior **427** and attached to a proximal end of a leader wire **431.** Preferably, the proximal hub **439** and distal hub **425** are hollow tubes formed from the same tube of memory metal, as described below. In some embodiments, the basket **411** includes a first row of two crowns (i.e., the proximal crowns **438** of the proximal cells **436**) and then subsequent repeating rows of twice as many crowns as compared to the number of proximal crowns **438** (i.e., four crowns) along the basket length **467.**

The system further includes a guide catheter **430** and a microcatheter **432**, which is wider and shorter than the guide catheter **430**, so that the microcatheter **432** can fit inside the guide catheter **430.** The microcatheter **432** has a hollow interior **415**, a proximal end **416** leading to said interior **415** and a distal end **414** leading to said interior **415.** The microcatheter **432** is comprised of a biocompatible material. For purposes of FIGS. 36-44, the terms "guide catheter", "microcatheter" and "catheter" generally refers to any suitable tube through which the system **410** can be deployed. Preferably, the catheters are sterile and comprised of a biocompatible material (i.e., a material that does not irritate the human body during the course of a 45 minute operation that involves using the system **410** to remove a clot **417** from an intracranial blood vessel **488**). The catheter can be any suitable shape, including but not limited to generally cylindrical. For purposes of the present invention, when it is said that the catheter envelopes the system **410**, it will be understood that the catheter envelopes at least one component of the system **410** (preferably, the distal basket **411**, the lead wire **431**, which is a wire that extends distally from the pull wire **443**, and the pull wire **443**). In some embodiments, the microcatheter 32 is about 0.833 mm (2.5 French) in diameter. Optionally, the catheter is delivered to the region of the lumen that has the obstruction **417** as follows: a guide wire is delivered to the obstruction region past the obstruction **417**; the catheter is delivered over the guide wire; the guide wire is removed; and the system **410** is delivered with its pull wire **443** and lead wire **431** through the catheter. Optionally, the pull wire **443** is used to push the system **410** through the catheter as well as to retrieve the distal basket **411** after capturing the obstruction **417** as described below. The system **410** may utilize a plurality of catheters as described above, such as, for example, a wider catheter that travels to the brain and a very flexible, smaller diameter microcatheter that is delivered from the first catheter and travels through the small arteries of the brain.

FIG. 37A shows the distal basket **411** collapsed inside a microcatheter **432.** The distal basket 411 is in what's referred to as the collapsed state. In this state, the system **410** is able to be located inside the microcatheter **432** and the basket height **461** is collapsed. For purposes of FIGs. 36-44, the basket height **461** generally refers to the height at a particular location (e.g., at the proximal-most crown **438** of the distal basket **411** or the distal-most crown **500** of the proximal basket **433**), it being understood that the height of the distal basket **411** and proximal basket **433** may vary along the distal basket length **467** and the length of the proximal basket **433.**

As shown in FIGs. 36-44, the distance **463** between the proximal hub **439** and distal hub **425** (i.e., the basket length **467**) is generally longer in the collapsed state, as compared to the relaxed state.

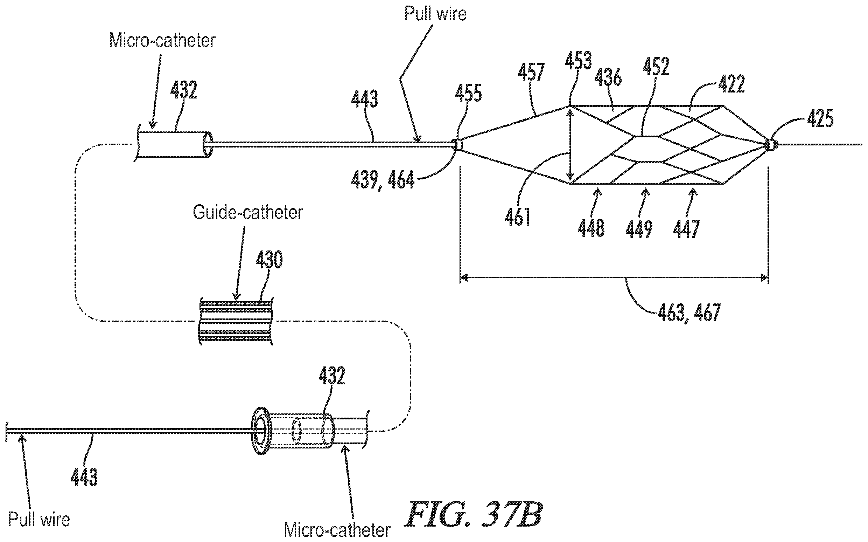

FIG. 37B shows the same basket system as FIG. 37A, except that the basket **411** has been deployed from the distal end **414** of the microcatheter **432** by pulling the microcatheter **432** proximally. As shown in FIG. 37B, the basket **411** is now in a relaxed state and the basket height **461** has increased. In the relaxed state exemplified, the basket length **467** and the distance **463** between the proximal and distal hubs **439** and **425** has decreased slightly as the basket **411** has relaxed. Optionally, the length of said proximal basket 467 is between about 20 and about 40 mm and the length **454** of said proximal tether memory metal strips **457** are between about 10 and about 20 mm in the relaxed state.

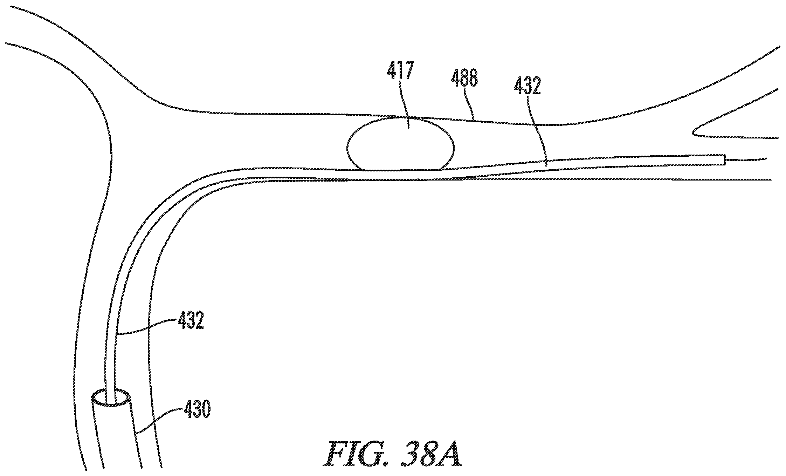

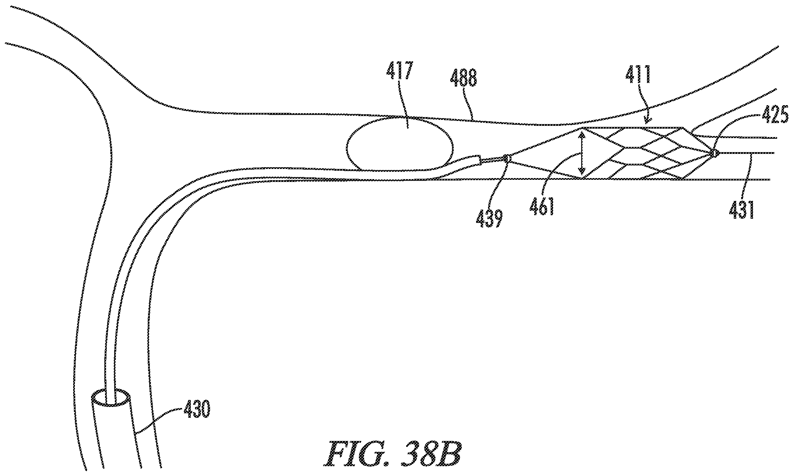

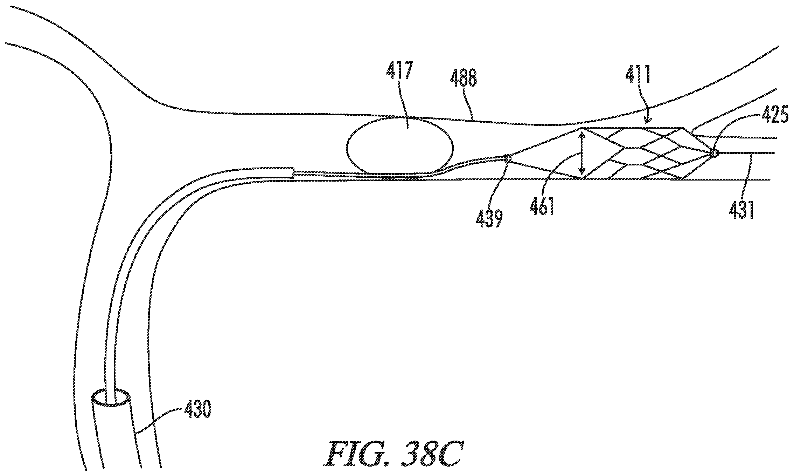

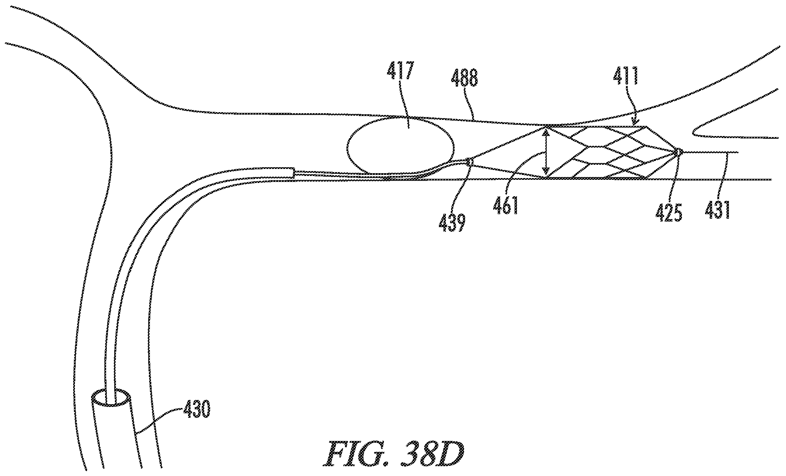

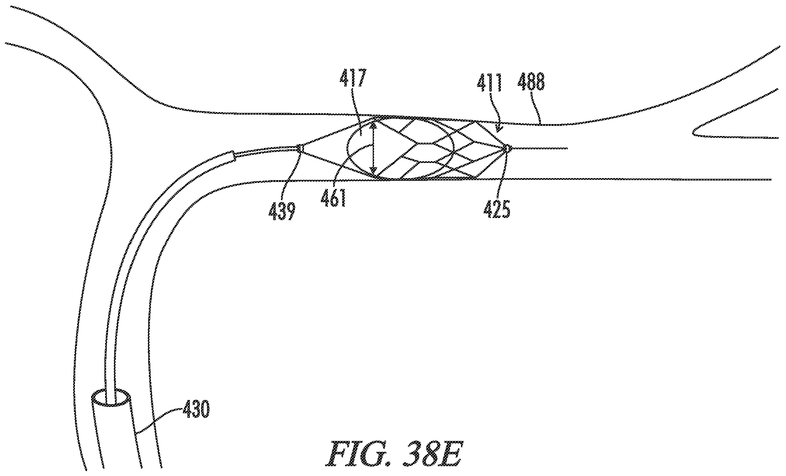

FIG. 38 illustrates use of the basket system shown in FIG. 37 in an intracranial artery **488.** As shown in FIG. 38A, first the guide catheter **430** is deployed proximal to the clot **417.** The microcatheter **432** is then advanced distally beyond the clot **417.** The basket **411** is collapsed inside the microcatheter **432.** Next, as shown in FIG. 38B, the microcatheter **432** is moved proximally to deploy the basket **411** so that the proximal tether memory metal strips **457** are distal to the clot **417.** The basket **411** is now in the relaxed state. Next, as shown in FIG. 38C, the user moves the basket **411** proximally over the clot **417.**

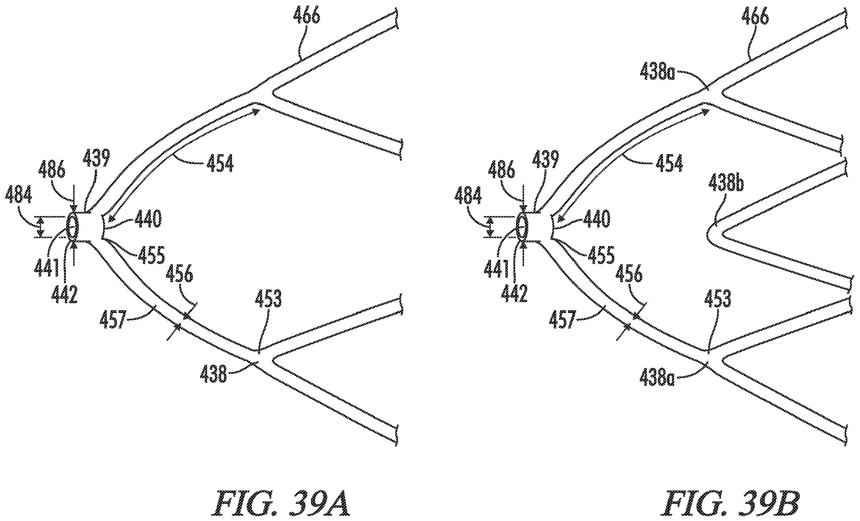

FIG. 39A shows a close-up view of the proximal end of the basket **411**, including the proximal tube interior **441**, the attachment of the proximal tether memory metal strips **457** at the distal end **455** of the proximal hub **439**, and the proximal crowns **438** of the proximal cells **436.** In FIG. 39A, all proximal crowns **438** of the proximal cells **436** are attached to a proximal tether memory metal strip **457.** FIG. 39B illustrates an alternative embodiment in which two proximal crowns **438a** of a proximal cell **436** (the top and bottom crowns **438a**) are attached to a proximal tether memory metal strip **457** and one proximal crown **438b** of a proximal cell **436** is not attached to a proximal tether memory metal strip **457.**

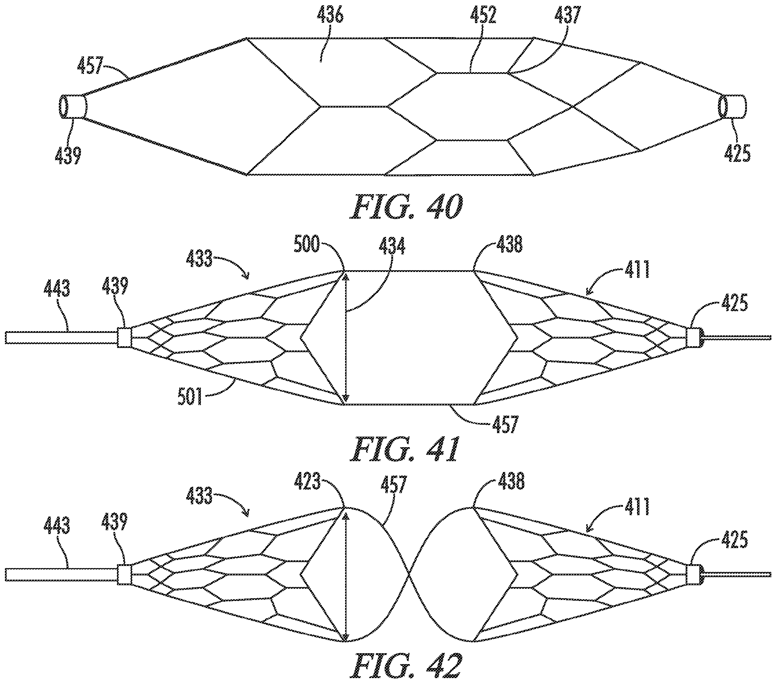

FIG. 40 illustrates a similar to basket system **410** to the above systems. In FIG. **40**, the proximal tether memory metal strips **457** are relatively thick (e.g., about 150% of the thickness of the proximal cell memory metal strips **466**).

It will be noted that the proximal end of the system **410** is shown at the bottom end of FIGs. 36-44 and the distal end of the system **410** is shown at the top end of FIGs. 36-44 because a principal use of the system **410** is to remove a blood clot **417** from a human intracranial artery **488**, in which case the system **410** generally will enter the artery **488** at its proximal end by the surgeon entering the patient's body near the groin and pushing the catheter **432** towards the brain. The diameter of human arteries **488** generally decrease from their proximal end to their distal end. However, when used in other types of lumens, the distal basket **411** may be located proximally relative to the catheter **432** as the term proximally and distally are used in that lumen.

FIG. 41 illustrates another embodiment of a basket system **411** with a proximal basket **433** and a distal basket **411.** In this embodiment, the system **411** includes a proximal hub **439** (similar to the prior embodiments). The difference is that the tether memory metal strips **457** actually join the proximal basket **433** and the distal basket **411.** More particularly, the proximal basket **433** is comprised of a plurality of proximal cells **436** attached to the proximal hub **439** and a plurality of distal cells **422** and the distal basket is comprised of a plurality of proximal cells **436** attached to the proximal hub 439 (preferably to the proximal end **499** of the distal hub **425**) and a plurality of distal cells 422 and the tether memory metal strips **457** join a distal crown **423** of a distal cell **422** of the distal basket **411** with a proximal crown **438** of a proximal cell **436** of the proximal basket **433.**

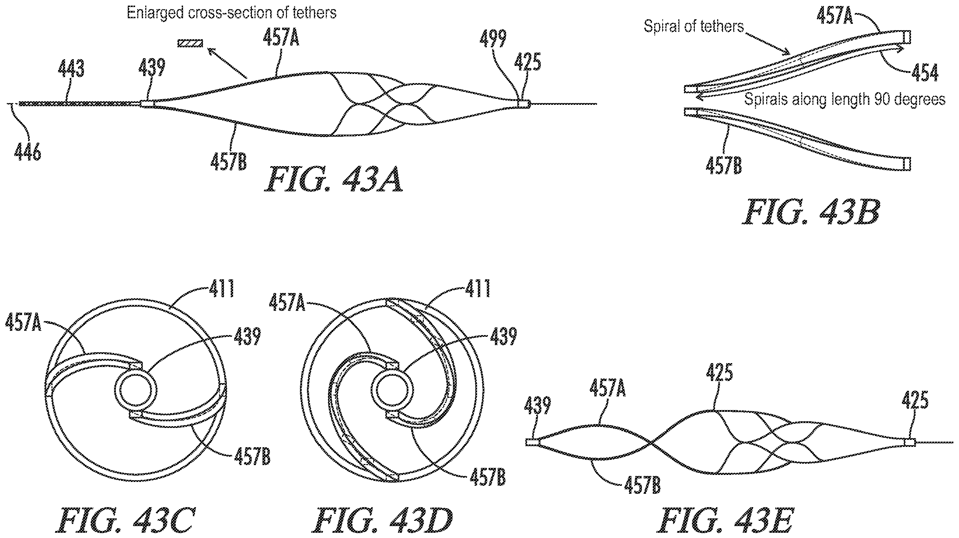

FIG. 42 illustrate an embodiment of the tether memory metal strips **457** rotating about said pull wire longitudinal axis **446** such that the distal end **453** of a proximal tether memory metal strip **457** is located between about 90 and about 270 degrees relative to said proximal end **455** of the same proximal tether memory metal strip **457.** In addition, the proximal tether memory metal strips **457** may rotate around their longitudinal axis **454** such that a distal end **453** of a proximal tether memory metal strip **457** rotates about 90 degrees around this tether longitudinal axis **454** from the distal end **453** to the proximal end **455** of the same proximal memory metal strip **457.** FIGs. 43B and 43C illustrates an exemplary embodiment, where the proximal end **455A** of the first proximal tether memory metal strip **457A** is located attached to the proximal tube **439** at the 12 o'clock position and the distal end **453A** of the same proximal tether memory metal strip **457A** is attached to a proximal-most crown **439** at the 9 o'clock position. In addition, the second proximal tether memory metal strip **457B** is located attached to the proximal tube **439** at the 6 o'clock position and the distal end **453B** of the same proximal tether memory metal strip **457B** is attached to the other proximal-most crown **439** at the 3 o'clock position. FIGs. 43D and 43E illustrate an exemplary embodiment of 180 degree rotation, where the proximal end **455A** of the first proximal tether memory metal strip **457A** is located attached to the proximal tube **439** at the 12 o'clock position and the distal end **453A** of the same proximal tether memory metal strip **457A** is attached to a proximal-most crown **439** at the 6 o'clock position. In addition, the second proximal tether memory metal strip **457B** is located attached to the proximal tube **439** at the 6 o'clock position and the distal end **453B** of the same proximal tether memory metal strip **457b** is attached to the other proximal-most crown **439** at the 12 o'clock position.

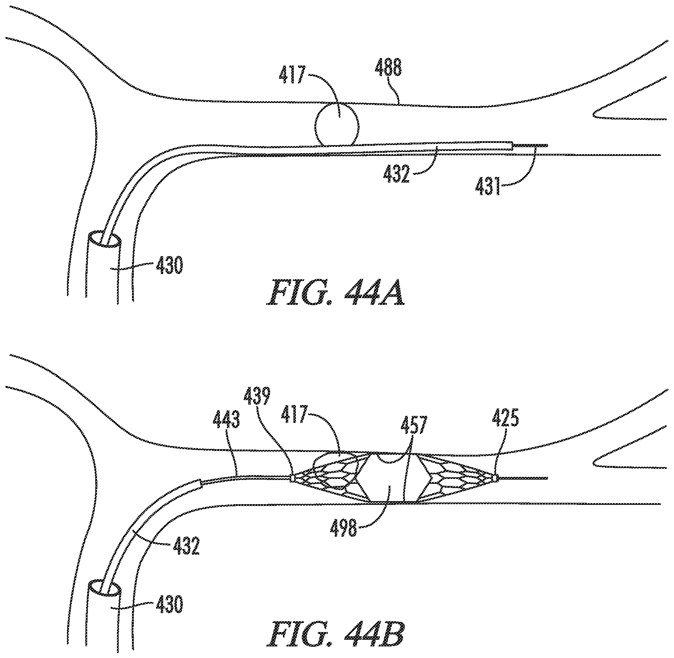

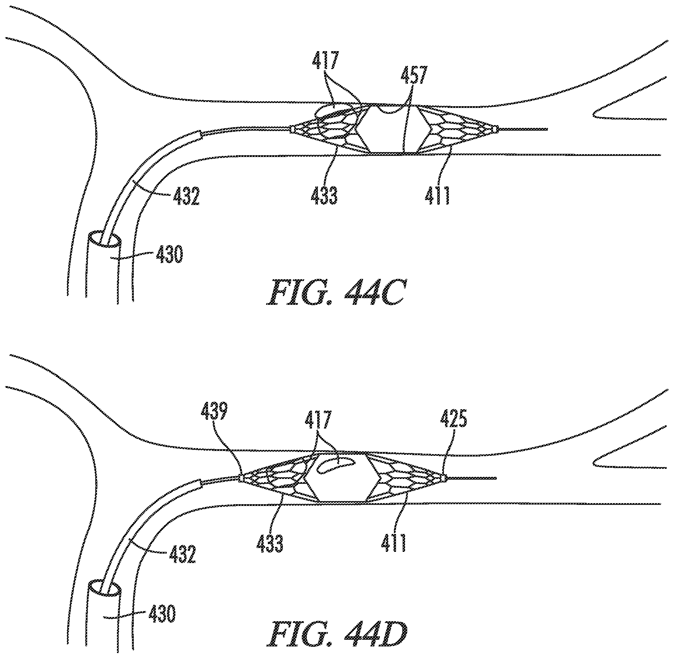

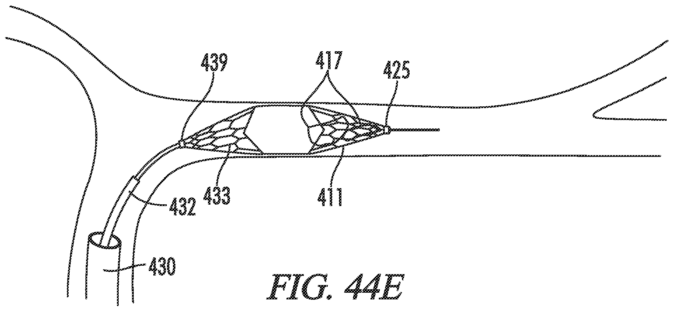

FIGs. 44A-44E illustrate a side, perspective view of stepwise deployment and use of a basket system **410** with a proximal basket **433** and a distal basket **411** in a blood vessel to retrieve a clot **417.** As shown, the distal basket **411** is deployed proximal to said clot **417** and said proximal basket **433** is deployed at said clot **417** so that said proximal basket **433** is at level of the clot. After allowing some time for clot debris to penetrate the proximal basket **433**, the basket system **433** is moved proximally toward said microcatheter **432.** See FIGs. 44B and 44C. As shown in FIG. 44D, the clot **417** falls moves medially into the void or space **498** between the proximal basket **433** and distal basket **411.** The system **410** continues to move proximally. The clot **477** is then located inside the distal basket **411.** See FIG. 44E. The proximal basket **433** optionally has a length in the relaxed state of preferably from about 10 to about 20 mm, as measured from the proximal-most crown to the distal-most crown.

The proximal basket **433** is used to deploy the system **411** across the obstruction **417** and is the initial site where the clot **417** enters through the struts **452.** As the basket system **411** is pulled/dragged proximally, the site of the proximal tether memory metal strip **457** gives a relative "open" area **498** for the clot **417** to fall into in the lumen of the vessel **488.** The distal basket **411** captures the clot **417** that has entered into the system **410** either through the basket cell openings or at the level of proximal tether memory metal strips **457** and prevents embolization into distal vessels **480.** Preferably, the proximal basket **433** has two distal crowns **500** at the distal end of the proximal basket **433** that are attached to the proximal end **455** of the proximal tether memory metal strips **457** and then one or more rows of proximal cells **501**, with four cells in each row.

In some examples, the basket system **410** is prepared by a process that includes one or more of the following steps, as illustrated in FIG. 36:
a) providing a single tube **468** comprised of a memory metal such as nitinol, the single tube **468** having an exterior, a substantially hollow interior, a wall **482** separating the exterior from the substantially hollow interior, an open proximal end **474**, an open distal end **476**, a middle portion **478** between the open proximal end **474** and the open distal end **476** (see FIG. 36A);
b) cutting the wall of the middle portion **478** with a laser **480** (see FIG. 36B);
c) removing the pieces of the middle portion cut by the laser **480** to form a basket system **410** comprising a proximal tube **439** comprising a hollow interior **441** extending through said proximal tube **439**, said proximal tube having a proximal end **442** and a distal end **440**, a distal tube **425** comprising a hollow interior **441** extending through said distal tube **425**, and a middle portion **478** located between said proximal tube **439** and said distal tube **425** and comprising a plurality of proximal tether memory metal strips **457**, each proximal tether memory metal strip **457** having a proximal end **455** attached to the distal end **440** of the proximal tube **439** and a distal end **453**;
d) altering the shape of the middle portion **478** using a mandrel and allowing the middle portion **478** to expand relative to the distal tube **476** and proximal tube **474** to form a distal basket **411** that includes a plurality of cells **422** and **436**;
e) quenching the middle portion **478** at room temperature;
f) removing the mandrel from the middle portion **478**;
g) mechanically or chemically electropolishing the middle portion **478** to remove oxides (see FIG. 36C);
h) inserting a pull wire **443** to said proximal tube **439**; and
i) attaching a leader wire **431** to said distal hub **425** (see FIG. 36D).

In some examples, the middle portion **478** is expanded by heating the mandrel and the middle portion **478** by, for example, placing the mandrel and the middle portion **478** in a fluidized sand bath at about 500°C for about 3 to about 7 minutes. As the middle portion **478** is heated, the heating causes the crystalline structure of the memory metal tube **468** to realign. Preferably, the mandrel is tapered (e.g., substantially conical or bullet in shape) so that the portion of the distal basket **411** formed from the middle portion **478** tapers from the proximal-most crown **438** to the distal end **466.** Preferably, the proximal and distal ends of the tube **474** and **476** are not shape set by the mandrel and are not cut by the laser **480** so that the proximal and distal ends **474** and **476** do not change in shape and only slightly expand in size under heating and return to the size of the native tube **468** after the heat is removed. Preferably, the laser cuts are programmed via a computer. To ensure that the laser cuts only one surface of the tube wall at the time (and not the surface directly opposite the desired cutting surface), the laser 480 is preferably focused between the inner and outer diameter of the desired cutting surface and a coolant is passed through the memory metal tube **468** so that the laser **480** cools before reaching the surface directly opposite the desired cutting surface.

The portions of the wall not cut by the laser **480** create the proximal and distal tubes **474** and **476** as well as the other components of the distal basket **411**, and memory metal strips **457** and **466,** as described.

Preferably, the memory metal selected for the native tube **468** has a heat of transformation below average human body temperature (37°C) so that the distal basket **411** has sufficient spring and flexibility after deployment from the catheter **432** in the human blood vessel 88.

In some embodiments, the native tube **468** (and hence the distal and proximal tubes **474** and **476**) have an outer diameter of less than about 1.33 mm (4 French), e.g., a diameter of about 0.33 mm to about 1.33 mm (about 1 to about 4 French). In some embodiments, the diameter of the pull wire **443** is between about 0.20 mm (0.008 inches) and about 1.30 mm (0.051 inches), as noted above, and in such embodiments, the diameter of the pull wire **443** may be approximately equal to the inner diameter **472** of the native nitinol tube **468.**

Without being bound by any particular theory, it is believed that manufacturing the distal basket **411** from a single memory metal tube **468** provides ease of manufacturing and safety from mechanical failure and provides tensile strength necessary for the system **410** to remove hard thrombus **417** and other obstructions.

Optionally, after step e, the basket **411** further comprises a row **448** of proximal cells **436**, each proximal cell **436** defined by a plurality of memory metal strips **466** and comprising a proximal crown **438** located at a proximal end of the cell **436** and pointing in the proximal direction and a distal crown **424** located at a distal end of the cell and pointing in the distal direction and further wherein each of said proximal crowns **438** of said proximal cells **436** is attached to a distal end **453** of a proximal tether memory metal strip **457.** Optionally, after step e, the basket **410** further comprises a row **447** of distal cells **422** located distal to said proximal cells **436** and connected to said distal crowns **424** of said proximal cells **436**, each distal cell **422** defined by a plurality of memory metal strips **466** and comprising a proximal crown **437** located at a proximal end of the cell **422** and pointing in the proximal direction and a distal crown **423** located at a distal end of the cell **422** and pointing in the distal direction, and further wherein the number of distal cells **422** is twice the number of proximal cells **436.** Optionally, after step e, the basket system **410** further comprises a row **449** of strut memory metal strips **452**, each strut memory metal strip **452** having a proximal end **451** attached to a distal crown **424** of a proximal cell **436** and a distal end **450** attached to a proximal crown **437** of a distal cell **422.** Optionally, the basket **411** comprises no welded components and said proximal tether memory metal strips **457** are integral with said proximal cell crowns **438.**

Optionally, after step e, the basket system **411** comprises between two and four proximal tether memory metal strips **457.** Optionally, prior to cutting the memory metal tube **468**, the memory metal tub **468** has an outer diameter 486 that is from about 0.28 mm (0.011 inches) to about 1.37 mm (0.054 inches) and an inner diameter 484 that is from about 0.20 mm (0.008 inches) to about 1.30 mm (0.051 inches). Optionally, after step e), the proximal tube **439** and distal tube **425** have an outer diameter that is from about 0.51 mm (0.02 inches) to about 0.76 mm (0.03 inches) and an inner diameter that is from about 0.25 mm (0.01 inches) to about 0.51 mm (0.02 inches). Optionally, the exemplary method further includes placing said basket **411** inside a catheter **432** comprised of a biocompatible material. Optionally, the exemplary method further includes the steps of placing the basket **411** inside a lumen **488** of an animal and using the basket to retrieve an object **417** located inside said lumen **488.**

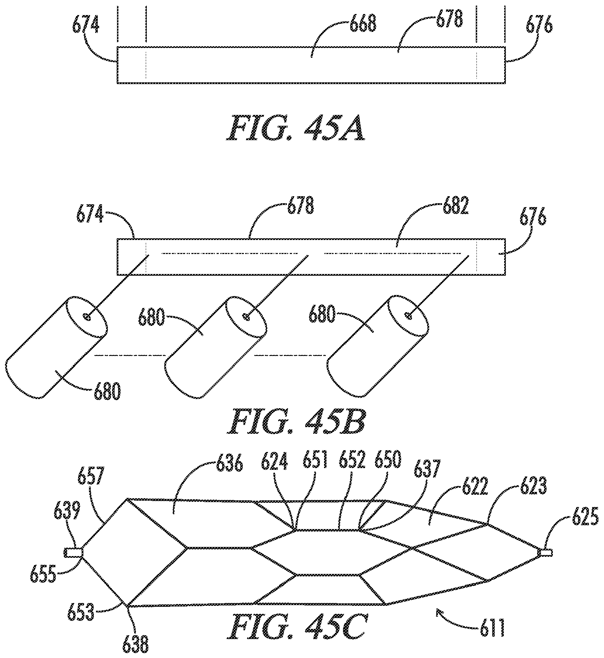

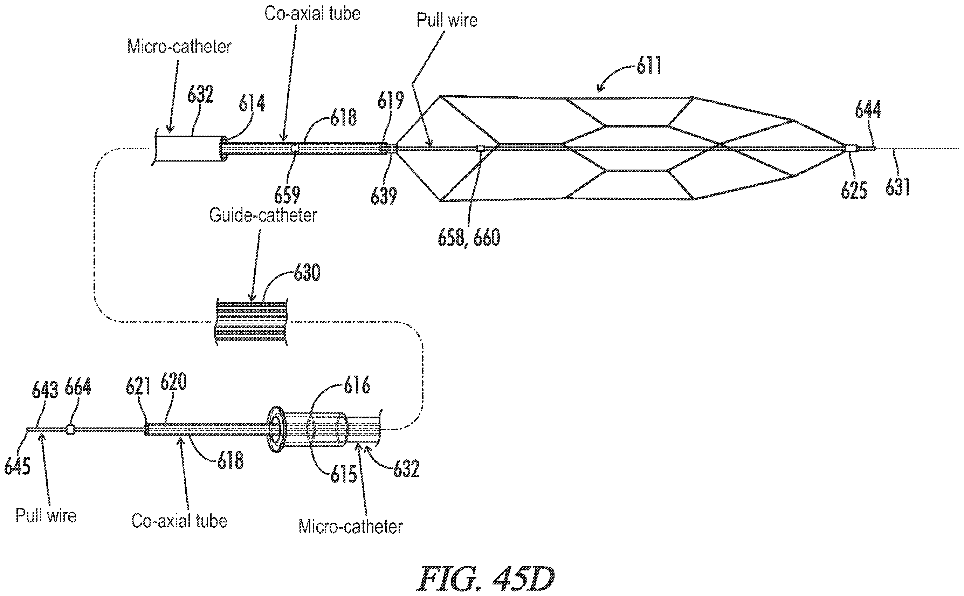

### The Embodiments of FIGs. 45-62

FIGs. 45-62 illustrate additional embodiments of a modular, easy-to-manufacture platform of systems for retrieving hard clots and other objects in animal lumens. In some embodiments, the system includes a proximal tube, a distal tube, and a plurality of memory metal strips between the proximal and distal tubes. The plurality of memory metal strips form a wide range of basket designs. Preferably, the proximal tube, memory metal strips, and distal tube are derived from a standard, off-the-shelf single tube of memory metal (e.g., nitinol), with the proximal tube and distal tube having the same inner diameter and outer diameter as the native tube from which they were derived and with the basket formed by cutting the middle portion of the native tube and expanding and shape-setting this cut portion. Preferably, the proximal tube and distal tube have an outer diameter that is from about 0.51 mm (0.02 inches) to about 0.76 mm (0.03 inches) (e.g., about 0.69 mm (0.027 inches)) so that the device fits inside a standard microcatheter and an inner diameter that is from about 0.25 mm (0.01 inches) to about 0.51 mm (0.02 inches). Preferably, there are no welded parts between the proximal tube and distal tube, which makes the system easy and cheap to reliably manufacture. The system also includes one or more catheters for deploying the system, a pull wire that passes through the hollow interior of the proximal tube, and a coaxial tube. Preferably, the system includes two catheters - a guide catheter and a microcatheter. The coaxial tube envelopes the pull wire, is slideable along at least a segment of the pull wire, and is attached to the proximal hub. The coaxial tube allows a user to move the proximal hub toward and away from the distal hub while keeping the distal hub stationary. Movement of the proximal hub toward and away from the distal hub causes conformational changes in the basket, including (depending on the basket design and the location of the proximal tube), collapsing the basket, expanding the basket, strengthening the basket, and moving the basket around the clot. The plurality of memory metal strips attached to the proximal hub include a plurality of proximal tether memory metal strips, which have a proximal end attached to the distal end of the proximal tube. The length and thickness of the proximal tether memory metal strips vary in the different embodiments described herein, which allows the surgical user to select from the various embodiments in the platform based on the features needed for the particular operation (e.g., vessel anatomy and hardness of the clot).

In some embodiments, the disclosure provides a system for removing objects within an interior lumen of an animal that includes
a pull wire having a proximal end, a distal end and a pull wire longitudinal axis extending from said proximal end to said distal end;
a distal basket attached to said pull wire, said distal basket comprising a proximal end, a distal end, a distal basket length extending from said distal basket proximal end to said distal end, a distal basket height perpendicular to said distal basket length and said pull wire longitudinal axis, a proximal hub located at said proximal end of the distal basket, said proximal hub comprising a hollow interior, said pull wire passing through said proximal hub hollow interior, said proximal hub slideable along at least a segment of the pull wire, a plurality of proximal tether memory metal strips, a plurality of proximal cells defined by a plurality of proximal cell memory metal strips, each proximal cell comprising a proximal crown located at the proximal end of the proximal cell and pointing generally in the proximal direction and a distal crown located at the distal end of the proximal cell and pointing generally in the distal direction, each proximal tether memory metal strip having a proximal end attached to said proximal hub, a distal end attached to a crown of a proximal cell and a length extending from said proximal end to said distal end, a plurality of distal cells distal to the proximal cells, and a distal hub located at said distal end of said distal basket and comprising a hollow interior,
said distal basket having
a relaxed state in which said proximal hub is located a first distance proximal to said proximal crowns and wherein said distal basket has a first height, as measured at the proximal-most crown,
a gaping state in which said proximal hub is located a second distance from said proximal crowns and wherein has a second height, as measured at the proximal-most crown, said second height greater than said first height, said second distance less than said first distance,
a proximal collapsed state in which said proximal hub is located a third distance proximal to said proximal crowns and wherein said distal basket has a third height and a third width, as measured at the proximal-most crown, said third distance greater than said first distance, said third height less than said first height,
a catheter having a hollow interior, a proximal end leading to said interior and a distal end leading to said interior, said catheter comprised of a biocompatible material and configured to envelope said distal basket when said distal basket is in said proximal collapsed state; wherein said distal basket is configured to move from said relaxed state to said gaping state by moving said proximal hub distally relative to said distal hub; and
wherein said distal basket is configured to move from said expanded state to said proximal collapsed state by moving said proximal hub proximally relative to said distal hub.

Optionally, the distal basket further comprises a distal collapsed state in which said proximal hub is located distal to said proximal crowns and wherein said distal basket has a fourth height, as measured at the proximal-most crown, said fourth height less than said first height, wherein said catheter is configured to envelope said distal basket when said distal basket is in said distal collapsed state, and further wherein said distal basket is configured to move from said gaping state to said distal collapsed state by moving said proximal hub distally relative to said distal hub. Optionally, the system further includes a coaxial tube, said coaxial tube configured to be received in said catheter, said coaxial tube having a proximal end, a distal end attached to said proximal hub, and a hollow interior, said pull wire passing through said coaxial tube hollow interior, said coaxial tube slideable along at least a segment of said pull wire. In some embodiments, said proximal tether memory metal strips and said proximal cell memory metal strips each have a thickness and further wherein said thickness of said proximal tether memory metal strips is between about 25 to about 75 percent of the thickness of the proximal cell memory metal strips. In such embodiments, the length of the proximal tether memory metal strips is between about 3 mm to about 10 mm in the relaxed state. In some embodiments with thin proximal tether memory metal strips, the combined length of two of said proximal tether memory metal strips is within about 2 mm of said second height. In other embodiments with thin proximal tether memory metal strips, the combined length of two of said proximal tether memory metal strips is within about 2 mm of said second height multiplied by a factor of two.

In other embodiments, the proximal tether memory metal strips are as thick or thicker than the memory metal strips forming the proximal cells and in such embodiments, the length of the proximal tether memory metal strips may be between about 10 mm and about 20 mm in the relaxed state.

Optionally, said pull wire extends from said distal basket proximal end to said distal basket distal end. Optionally, said pull wire is not in contact with said distal hub. Optionally, in said gaping state, said proximal hub is located parallel to said proximal crown. Optionally, said pull wire and said proximal hub are offset from the center of the distal basket height, as measured at the proximal-most crown. Optionally, all proximal crowns of said proximal cells are attached to a proximal tether memory metal strip. Optionally, said distal basket further comprises a plurality of strut memory metal strips and plurality of distal cells defined by a plurality of distal memory metal strips, said distal cells comprising a proximal crown located at a proximal end of said distal cells and a distal crown located at a distal end of said distal cells, said strut memory metal strips having a proximal end attached to a distal crown of a proximal cell and a distal end attached to a proximal crown of a distal cell. Optionally, the distal basket comprises between two and four proximal tether memory metal strips. Optionally, said proximal memory metal strips are integral with said proximal hub. Optionally, said proximal hub is a tube, wherein said interior of said proximal hub has a size and shape, and further wherein said size and shape of said proximal hub interior are configured to prevent a segment of said pull wire distal relative to said proximal hub from moving through proximal hub interior. Optionally, said distal hub is a tube. Optionally, said distal hub is attached to said pull wire such that said distal hub is not slideable along said pull wire. Optionally, said distal basket further comprises a lead wire extending distally from said distal hub. Optionally, said distal hub, said proximal hub, and said basket are comprised of a nitinol having the same material composition. Optionally, said distal basket further comprises an x-ray marker that is more visible under x-ray as compared to the other components when the distal basket is located in a cranial blood vessel inside the body of a human and the x-ray is taken from outside the human's body. Preferably, the x-ray marker is a radiopaque material. Some examples of radiopaque materials can include, but are not limited to, gold, platinum, palladium, tantalum, tungsten alloy, polymer material loaded with radiopaque filler, and the like. Preferably, the components are comprised of nitinol and the x-ray marker is comprised of a material having a density greater than the nitinol. Optionally, said proximal and said distal hubs are generally cylindrical in shape and each has an outer diameter and an inner diameter, the inner diameter forming apertures of the proximal and distal hubs and further wherein the outer diameters of the proximal and distal hubs are substantially the same size and further wherein the inner diameters of the proximal and distal hubs are substantially the same size. Optionally, the outer diameters of the proximal and distal hubs are from about 0.28 mm (0.011 inches) to about 1.37 mm (0.054 inches), and further wherein the inner diameters of the proximal and distal hubs are from about 0.20 mm (0.008 inches) to about 1.30 mm (0.051 inches). Optionally, the proximal tube and distal tube have an outer diameter that is from about 0.51 mm (0.02 inches) to about 0.76 mm (0.03 inches) and an inner diameter that is from about 0.25 mm (0.01 inches) to about 0.51 mm (0.02 inches). Optionally, the pull wire is generally cylindrical and further wherein the diameter of the pull wire is between about 0.20 mm (0.008 inches) and about 1.30 mm (0.051 inches). Optionally, the first height is between about 2 millimeters and about 8 millimeters. Optionally, said proximal tether memory metal strips rotate about said pull wire longitudinal axis such that a distal end of a proximal tether memory metal strip is located between about 90 and about 270 degrees relative to said proximal end of the same proximal tether memory metal strip.

The present disclosure also provides an exemplary method of removing an object from an interior lumen of an animal, said lumen having an interior wall forming said lumen. In some examples, the exemplary method includes:
a) providing the system described above;
b) positioning the system in said lumen, said basket located in said catheter in a collapsed state;
c) deploying said distal basket from said distal end of said catheter so that said proximal crowns of said proximal cells are distal to said obstruction;
d) allowing said distal basket to move to said relaxed state;
e) moving said proximal hub distally relative to said distal hub so that said distal basket height, as measured at the proximal-most crown, increase;
f) moving said distal basket over said obstruction; and
g) removing said distal basket and said obstruction from said lumen.

Optionally, the interior lumen is an intracranial artery and said obstruction is a blood clot. Optionally, the exemplary method further comprises using said blood clot to move said proximal hub distally relative to said distal hub and allow said distal basket to move to said gaping state. Optionally, the exemplary method further comprises using a coaxial tube to push said proximal hub distally relative to said distal hub and allow said distal basket to move to said gaping state. Optionally, the exemplary method further includes, after step e, moving said proximal hub relative to said distal hub so that said distal basket height, as measured at the proximal-most crown, decrease. Optionally, after step e, said pull wire and said proximal hub are offset with respect to the center of said distal basket height, as measured at the proximal-most crown, as measured at the proximal-most crown, and the center of said lumen.

The present disclosure also provides a system for removing objects within an interior lumen of an animal, the system comprising:
a pull wire having a proximal end, a distal end and a pull wire longitudinal axis extending from said proximal end to said distal end;
a proximal basket attached to said pull wire, said proximal basket comprising a proximal end, a distal end, a proximal basket length extending from said proximal basket proximal end to said distal end, a proximal basket height perpendicular to said proximal basket length and said pull wire longitudinal axis, a proximal tube located at said proximal end of the proximal basket, said proximal tube comprising a hollow interior, said pull wire passing through said hollow interior and said proximal tube slideable along at least a segment of said pull wire, a plurality of rows of cells, each cell defined by a plurality of memory metal strips, each cell comprising a proximal crown located at the proximal end of the proximal cell and pointing generally in the proximal direction and a distal crown located at the distal end of the proximal cell and pointing generally in the distal direction,
a distal basket attached to said pull wire, said distal basket comprising a proximal end, a distal end, a distal basket length extending from said distal basket proximal end to said distal end, a distal basket height perpendicular to said distal basket length and said pull wire longitudinal axis, a distal tube located at said distal end of the distal basket, said distal tube comprising a hollow interior, a plurality of rows of cells, each cell defined by a plurality of memory metal strips, each cell comprising a proximal crown located at the proximal end of the proximal cell and pointing generally in the proximal direction and a distal crown located at the distal end of the proximal cell and pointing generally in the distal direction,
a plurality of tether memory metal strips, each tether memory metal strip having a proximal end attached to a distal crown of a cell located at the distal end of said proximal basket and a distal end attached to a proximal crown of a cell located at the proximal end of said distal basket,
   said proximal basket having
a relaxed state wherein said proximal basket has a first height as measured at the distal-most crown, and said proximal hub is located a first distance proximal to said distal hub;
   a collapsed state wherein said proximal basket has a second height, as measured at the distal-most crown, said second height less than said first height;
a gaping state wherein said proximal basket has a third height, as measured at the distal-most crown, and said proximal hub is located a second distance proximal to said distal hub, said third height greater than said first height and said second distance less than said first distance, said proximal basket configured to move from said expanded state to said gaping state by pushing said proximal tube distally relative to said distal tube;
   said distal basket having
a relaxed state wherein said distal basket has a first height and
a collapsed state wherein said distal basket has a second height, said second height less than said first height, and
a catheter having an interior, a proximal end leading to said interior and a distal end leading to said interior, said catheter comprised of a biocompatible material and configured to envelope said distal and said proximal basket when said baskets are in said collapsed state.

Optionally, said proximal tether memory metal strips rotate about said pull wire longitudinal axis such that a distal end of a proximal tether memory metal strip is located between about 90 and about 270 degrees relative to said proximal end of the same proximal tether memory metal strip.

In some arrangements, the system does not include a proximal hub and the system includes soft cords in place of or in addition to the proximal memory metal strips. For example, in one arrangement, the system includes:
a pull wire having a proximal end, a distal end and a pull wire longitudinal axis extending from said proximal end to said distal end;
a coaxial tube having a proximal end, a distal end and a hollow interior, said pull wire passing through said coaxial tube hollow interior, said coaxial tube slideable along at least a segment of said pull wire;
a distal basket attached to said pull wire and said coaxial tube, said distal basket comprising a proximal end, a distal end, a distal basket length extending from said distal basket proximal end to said distal end, a distal basket height perpendicular to said distal basket length and said pull wire longitudinal axis, a plurality of cords, a plurality of proximal cells defined by a plurality of proximal cell memory metal strips, each proximal cell comprising a proximal crown located at the proximal end of the proximal cell and pointing generally in the proximal direction and a distal crown located at the distal end of the proximal cell and pointing generally in the distal direction, each cord having a proximal end attached to said coaxial tube, a distal end attached to a crown of a proximal cell and a length extending from said proximal end to said distal end, a plurality of distal cells distal to the proximal cells, and a distal hub located at said distal end of said distal basket and comprising a hollow interior,
   said distal basket having
a relaxed state in which said coaxial tube is located a first distance proximal to said proximal crowns and wherein said distal basket, as measured at the proximal-most crown, has a first height,
a proximal collapsed state in which said coaxial tube is located a second distance proximal to said proximal crowns and wherein said distal basket, as measured at the proximal-most crown, has a second height, said second distance greater than said first distance, said second height less than said first height,
a catheter having a hollow interior, a proximal end leading to said interior and a distal end leading to said interior, said catheter comprised of a biocompatible material and configured to envelope said coaxial tube and said distal basket when said distal basket is in said proximal collapsed state;
wherein said distal basket is configured to move from said relaxed state to said proximal collapsed state by moving said coaxial tube proximally relative to said distal hub.

Optionally, the distal basket further comprises a distal collapsed state in which said coaxial tube is located distal to said proximal crowns and wherein said distal basket, as measured at the proximal-most crown, has a third height, said third height less than said first height, wherein said catheter is configured to envelope said distal basket when said distal basket is in said distal collapsed state, and further wherein said distal basket is configured to move from said relaxed state to said distal collapsed state by moving said coaxial tub distally relative to said distal hub. Optionally said cord is comprised of a material selected from the group consisting of plastic, rubber, nylon, suture material, and braided catheter material. Optionally, said cords are integral with said coaxial sheath. Optionally, said cords are glued to said coaxial sheath. Optionally, said cords are shrink wrapped to said coaxial sheath. Optionally, said cords have a thickness of from about 0.025 mm to about 0.25 mm (about 0.001 to about 0.1 inches) (more preferably about 0.1 mm to about 0.46 mm (about 0.004 to about 0.018 inches)) and have a length of from about 3 mm to about 20 mm in said relaxed state. Optionally, said pull wire extends from said distal basket proximal end to said distal basket distal end and said pull wire is attached to said distal hub. Optionally, all proximal crowns of said proximal cells are attached to a cord. Optionally, the basket comprises four proximal cells, each proximal cell having a proximal crown, and not all (e.g., only two) of the proximal crowns are attached to a cord. Optionally, said distal basket further comprises a plurality of strut memory metal strips and plurality of distal cells defined by a plurality of distal memory metal strips, said distal cells comprising a proximal crown located at a proximal end of said distal cells and a distal crown located at a distal end of said distal cells, said strut memory metal strips having a proximal end attached to a distal crown of a proximal cell and a distal end attached to a proximal crown of a distal cell. Optionally, the distal basket comprises between two and four cords. Optionally, said distal hub is attached to said pull wire such that said distal hub is not slideable along said pull wire. Optionally, said distal basket further comprises a lead wire extending distally from said distal hub. Optionally, said distal hub and said basket are comprised of a nitinol having the same material composition. Optionally, said distal basket and/or said coaxial tube further comprises an x-ray marker that is more visible under x-ray as compared to the other components when the distal basket is located in a cranial blood vessel inside the body of a human and the x-ray is taken from outside the human's body. Preferably, the x-ray marker is a radiopaque material. Some examples of radiopaque materials can include, but are not limited to, gold, platinum, palladium, tantalum, tungsten alloy, polymer material loaded with radiopaque filler, and the like. Preferably, the components are comprised of nitinol and the x-ray marker is comprised of a material having a density greater than the nitinol. Optionally, said distal hub is generally cylindrical in shape and has an outer diameter and an inner diameter, the inner diameter forming the aperture of the distal hub and further wherein the outer diameter of the distal hub from about 0.28 mm (0.011 inches) to about 1.37 mm (0.054 inches), and further wherein the inner diameter of the distal hub is from about 0.20 mm (0.008 inches) to about 1.30 mm (0.051 inches). Optionally, the distal tube has an outer diameter that is from about 0.51 mm (0.02 inches) to about 0.76 mm (0.03 inches) and an inner diameter that is from about 0.25 mm (0.01 inches) to about 0.51 mm (0.02 inches). Optionally, the pull wire is generally cylindrical and further wherein the diameter of the pull wire is between about 0.20 mm (0.008 inches) and about 1.30 mm (0.051 inches). Optionally, the first height of the distal basket, as measured at the proximal-most crown, is between about 2 millimeters and about 8 millimeters. Optionally, said cords are soft.

The present disclosure provides an exemplary method of removing an object from an interior lumen of an animal, said lumen having an interior wall forming said lumen, the exemplary method comprising the steps of:
a) providing the system described above;
b) positioning the system in said lumen, said basket located in said catheter in a collapsed state;
c) deploying said distal basket from said distal end of said catheter so that said proximal crowns of said proximal cells are distal to said obstruction;
d) allowing said distal basket to move to said relaxed state;
e) moving said coaxial tube distally relative to said distal hub so that said coaxial tube moves distally to the proximal-most crown;
f) moving said distal basket, said pull wire and said coaxial tube proximally so that said distal basket moves over said obstruction;
g) moving said coaxial sheath distally relative to said distal hub so that said distal basket height, as measured at the proximal-most crown, decreases and said coaxial tube is closer to said distal hub as compared to the proximal-most crown; and
i) removing said distal basket and said obstruction from said lumen.

In other examples, the exemplary method includes
a) providing the system described above;
b) positioning the system in said lumen, said basket located in said catheter in a collapsed state;
c) deploying said distal basket from said distal end of said catheter so that said proximal crowns of said proximal cells are distal to said obstruction;
d) allowing said distal basket to move to said relaxed state;
e) moving said coaxial tube distally relative to said distal hub so that said coaxial tube moves distally to the proximal-most crown;
f) moving said distal basket, said pull wire and said coaxial tube proximally so that said distal basket moves over said obstruction;
g) moving said coaxial sheath proximally relative to said distal hub so that said distal basket height, as measured at the proximal-most crown, decreases;
h) moving said catheter distally relative to said distal hub so that said catheter re-sheaths said coaxial sheath and partially re-sheaths said cords, thereby decreasing said distal basket height, as measured at the proximal-most crown;
i) removing said distal basket and said obstruction from said lumen.

Optionally, said interior lumen is an intracranial artery and said obstruction is a blood clot.

In other arrangements that do not include a proximal hub, the system includes
a pull wire having a proximal end, a distal end and a pull wire longitudinal axis extending from said proximal end to said distal end;
a coaxial tube having a proximal end, a distal end and a hollow interior, said pull wire passing through said coaxial tube hollow interior, said coaxial tube slideable along at least a segment of said pull wire;
a distal basket attached to said pull wire and said coaxial tube, said distal basket comprising a proximal end, a distal end, a distal basket length extending from said distal basket proximal end to said distal end, a distal basket height perpendicular to said distal basket length and said pull wire longitudinal axis, a plurality of proximal tether memory metal strips, a plurality of cords, a plurality of proximal cells defined by a plurality of proximal cell memory metal strips, each proximal cell comprising a proximal crown located at the proximal end of the proximal cell and pointing generally in the proximal direction and a distal crown located at the distal end of the proximal cell and pointing generally in the distal direction, each proximal tether memory metal strip having a proximal end attached to said coaxial tube and a distal end, each cord having a proximal end attached to a distal end of a proximal tether memory metal strip and a distal end attached to a crown of a proximal cell and a length extending from said proximal end to said distal end, and a plurality of distal cells distal to the proximal cells, and a distal hub located at said distal end of said distal basket and comprising a hollow interior,
said distal basket having
a relaxed state in which said distal basket, as measured at the proximal-most crown, has a first height,
a collapsed state in which said distal basket, as measured at the proximal-most crown, has a second height, said second height less than said first height,
a catheter having a hollow interior, a proximal end leading to said interior and a distal end leading to said interior, said catheter comprised of a biocompatible material and configured to envelope said coaxial tube and said distal basket when said distal basket is in said collapsed state.

Optionally, said cord is comprised of a material selected from the group consisting of plastic, rubber, nylon, suture material, and braided catheter material. Optionally, said proximal tether memory metal strips are integral with said coaxial sheath. Optionally, said cords are glued to said proximal tether memory metal strips. Optionally, said cords are shrink wrapped to said proximal tether memory metal strips. Optionally, said cords have a thickness of from about 0.1 mm to about 0.25 mm (about 0.004 to about 0.1 inches) (more preferably about 0.1 mm to about 0.46 mm (about 0.004 inches to about 0.018 inches)) and further wherein said cords have a length of from about 3 mm to about 20 mm in said relaxed state. Optionally, said pull wire extends from said distal basket proximal end to said distal basket distal end and said pull wire is attached to said distal hub. Optionally, all proximal crowns of said proximal cells are attached to a cord. Optionally, the basket comprises four proximal cells, each proximal cell having a proximal crown, and not all (e.g., only two) of the proximal crowns are attached to a cord. Optionally, said distal basket further comprises a plurality of strut memory metal strips and plurality of distal cells defined by a plurality of distal memory metal strips, said distal cells comprising a proximal crown located at a proximal end of said distal cells and a distal crown located at a distal end of said distal cells, said strut memory metal strips having a proximal end attached to a distal crown of a proximal cell and a distal end attached to a proximal crown of a distal cell. Optionally, the distal basket comprises between two and four cords. Optionally, said distal hub is attached to said pull wire such that said distal hub is not slideable along said pull wire. Optionally, said distal basket further comprises a lead wire extending distally from said distal hub. Optionally, said distal hub and said basket are comprised of a nitinol having the same material composition. Optionally, said distal basket and/or said coaxial tube further comprises an x-ray marker that is more visible under x-ray as compared to the other components when the distal basket is located in a cranial blood vessel inside the body of a human and the x-ray is taken from outside the human's body. Preferably, the x-ray marker is a radiopaque material. Some examples of radiopaque materials can include, but are not limited to, gold, platinum, palladium, tantalum, tungsten alloy, polymer material loaded with radiopaque filler, and the like. Preferably, the components are comprised of nitinol and the x-ray marker is comprised of a material having a density greater than the nitinol. Optionally, said distal hub is generally cylindrical in shape and has an outer diameter and an inner diameter, the inner diameter forming the aperture of the distal hub and further wherein the outer diameter of the distal hub from about 0.28 mm 0.011 inches) to about 1.37 mm (0.054 inches), and further wherein the inner diameter of the distal hub is from about 0.20 mm (0.008 inches) to about 1.30 mm (0.051 inches). Optionally, the distal tube has an outer diameter that is from about 0.51 mm (0.02 inches) to about 0.76 mm (0.03 inches) and an inner diameter that is from about 0.25 mm (0.01 inches) to about 0.51 mm (0.02 inches). Optionally the pull wire is generally cylindrical and further wherein the diameter of the pull wire is between about 0.20 mm (0.008 inches) and about 1.30 mm (0.051 inches). Optionally, the first height of the distal basket, as measured at the proximal-most crown, is between about 2 millimeters and about 8 millimeters. Optionally, the cords are soft.

The above system may be used in an exemplary method of removing an object from an interior lumen of an animal, said lumen having an interior wall forming said lumen that includes
a) providing the above system;
b) positioning the system in said lumen, said basket located in said catheter in a collapsed state;
c) deploying said distal basket from said distal end of said catheter so that said proximal crowns of said proximal cells are distal to said obstruction, said coaxial sheath is proximal to said obstruction, said proximal tether memory metal strips are proximal to said obstruction, and said cords are adjacent to said obstruction;
d) allowing said distal basket to move to said relaxed state;
e) moving said coaxial tube distally relative to said distal hub so that said proximal tether memory metal strips move distally relative to the proximal-most crown and said obstruction is sandwiched between said proximal tether memory metal strips and said proximal crowns of said proximal cells;
f) removing said distal basket and said obstruction from said lumen.

Optionally said interior lumen is an intracranial artery and said obstruction is a blood clot.

With reference to FIGs. 45-62 the present disclosure provides a deployable system, generally designated by the numeral **610**, for removing an obstruction such as a blood clot **617** or other object from a blood vessel **688** or other interior lumen of an animal. In addition to a blood clot **617**, the obstruction may be, for example, extruded coils during aneurysm treatment, intravascular embolic material such as onyx or other obstructions requiring mechanical intravascular removal from small distal vessels. In the drawings, not all reference numbers are included in each drawing for the sake of clarity.

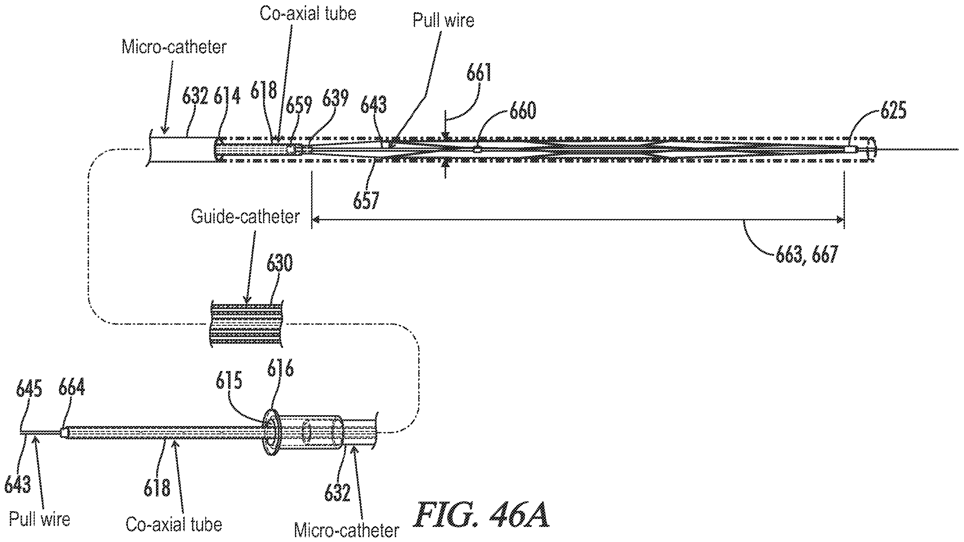

One example of a deployable basket system **610** is shown in FIGs. 46A-46E, 47G-47H and 50A. As shown in FIGs. 46A-46E, 47G-47H and 50A, the system **610** includes a pull wire **643** having a proximal end **645**, a distal end **644** and a pull wire longitudinal axis **646** extending from said proximal end **645** to said distal end **644.** Optionally, the diameter of the pull wire **643** is between about 0.20 mm (0.008 inches) and about 1.30 mm (0.051 inches).

The system **610** further includes a distal basket **611** attached to said pull wire **643**, said distal basket **611** comprising a proximal end **669**, a distal end **665**, a distal basket length **667** extending from said distal basket proximal end **669** to said distal end **665**, a distal basket height **661** perpendicular to said distal basket length **667** and said pull wire longitudinal axis **646**, a proximal hub **639** located at said proximal end **669** of the distal basket **611**, said proximal hub **639** comprising a hollow interior **641**, said pull wire **643** passing through said proximal hub hollow interior **641**, said proximal hub **639** slideable along at least a segment of the pull wire **643**, a plurality of proximal tether memory metal strips **657**, a plurality of proximal cells **636** defined by a plurality of proximal cell memory metal strips **666**, each proximal cell **636** comprising a proximal crown **638** located at the proximal end of the proximal cell **636** and pointing generally in the proximal direction and a distal crown **624** located at the distal end of the proximal cell **636** and pointing generally in the distal direction, each proximal tether memory metal strip **657** having a proximal end **655** attached to said proximal hub **639**, a distal end **663** attached to a crown of a proximal cell **638** and a length **655** extending from said proximal end **655** to said distal end **653**, a plurality of distal cells **622** distal to the proximal cells **636**, and a distal hub **625** located at said distal end **665** of said distal basket and comprising a hollow interior **627.** Preferably, the proximal hub **639** and distal hub **625** are hollow tubes formed from the same tube of memory metal, as described below. In some embodiments, the basket **611** includes a first row of two, three, or four crowns (i.e., the proximal crowns **638** of the proximal cells **638**) and then subsequent repeating rows of twice as many crowns as compared to the number of proximal crowns **638** (i.e., four, six, or eight crowns) along the basket length **667.**

The system further includes a guide catheter **630** and a microcatheter **632**, which is wider and shorter than the guide catheter **630**, so that the microcatheter **632** can fit inside the guide catheter **630.** The microcatheter **632** has a hollow interior **615**, a proximal end **616** leading to said interior **615** and a distal end **614** leading to said interior **615.** The microcatheter **632** is comprised of a biocompatible material. As used herein, the terms "guide catheter", "microcatheter" and "catheter" generally refers to any suitable tube through which the system **610** can be deployed. Preferably, the catheters are sterile and comprised of a biocompatible material (i.e., a material that does not irritate the human body during the course of a 45 minute operation that involves using the system **610** to remove a clot **617** from an intracranial blood vessel **688**). The catheter can be any suitable shape, including but not limited to generally cylindrical. For purposes of the present invention, when it is said that the catheter envelopes the system **610**, it will be understood that the catheter envelopes at least one component of the system **610** (preferably, the distal basket **611**, the lead wire **631**, which is a wire that extends distally from the pull wire **643**, and the pull wire **643**). In some embodiments, the microcatheter **632** is about 0.833 mm (2.5 French) in diameter. Optionally, the catheter is delivered to the region of the lumen that has the obstruction **617** as follows: a guide wire is delivered to the obstruction region past the obstruction **617**; the catheter is delivered over the guide wire; the guide wire is removed; and the system **610** is delivered with its pull wire **643** and lead wire **631** through the catheter. Optionally, the pull wire **643** is used to push the system **610** through the catheter as well as to retrieve the distal basket **611** after capturing the obstruction **617** as described below. The system **610** may utilize a plurality of catheters as described above, such as, for example, a wider catheter that travels to the brain and a very flexible, smaller diameter microcatheter that is delivered from the first catheter and travels through the small arteries of the brain.

Preferably, a coaxial tube **618**, which has a hollow interior **620** and is slideable along at least a portion of the pull wire **643** is attached to the proximal hub **639.**

FIG. 46A shows the distal basket **611** collapsed inside a microcatheter **632.** The distal basket **611** is in what's referred to as the proximal collapsed state. In this state, the system **610** is able to be located inside the microcatheter **632** and the basket height **661** is collapsed. For purposes of the present invention, the basket height **661** generally refers to the height at a particular location (e.g., at the proximal-most crown **638** of the distal basket **611** or the distal-most crown **623** of the proximal basket **633**), it being understood that the height of the distal basket **611** and proximal basket **633** may vary along the distal basket length **667** and the length of the proximal basket **633.**

In FIG. 46A, the proximal hub **639** is located a maximum distance from the distal hub **625**. The distance from the proximal hub **639** to the distal hub **625** changes by exerting force on the proximal hub **639**, as described herein, and the distance is shown in the drawings using the numeral **663.** This distance is also generally equal to the length of the basket **667**, as shown.

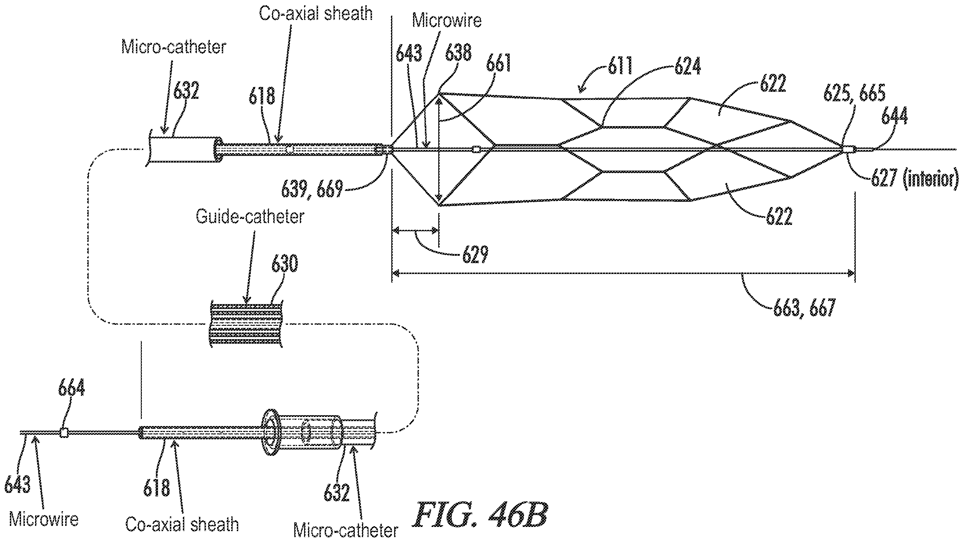

FIG. 46B shows the same basket system as FIG. 46A, except that the basket **611** has been deployed from the distal end **614** of the microcatheter **632** by pulling the microcatheter **632** proximally. As shown in FIG. 46B, the basket **611** is now in a relaxed state and the basket height **661** has increased. In the relaxed state exemplified, the proximal tube **639** is located a short distance **629** proximal to the proximal-most crown **638.** In addition, the basket length **667** and the distance **663** between the proximal and distal hubs **639** and **625** has decreased as the basket **611** has relaxed. In addition, the user has moved the coaxial tube **618** proximally relative to the pull wire **643** as shown by the line in the lower part of FIG. 46B, which indicates that the distance between the proximal stop **664** and the coaxial tube proximal end **621** has increased from FIG. 46A to FIG. 46B. The present invention may utilize a variety of stops, such as a proximal stop **664**, which is any barrier that prevents the coaxial tube **618** from moving proximally beyond the proximal stop **664**. In some forms, the proximal stop **664** is merely an enlargement or x-ray marker **658** in the pull wire **643** that is taller and/or wider than the open coaxial tube interior **620** (i.e., the inner diameter of the coaxial tube **618**). Instead of stops or in addition to stops, the pull wire **643** may be etched to provide guidance to the surgeon on the distance to push and pull the coaxial tube **618.**

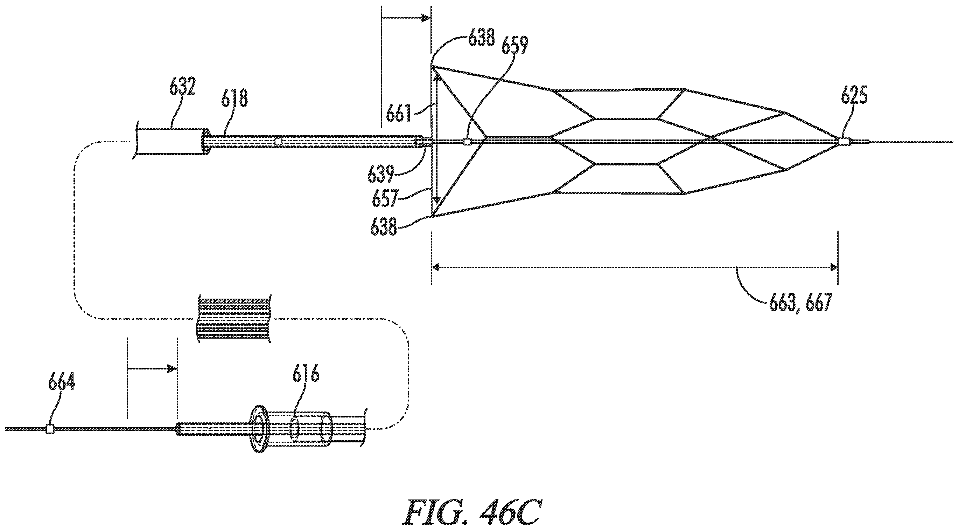

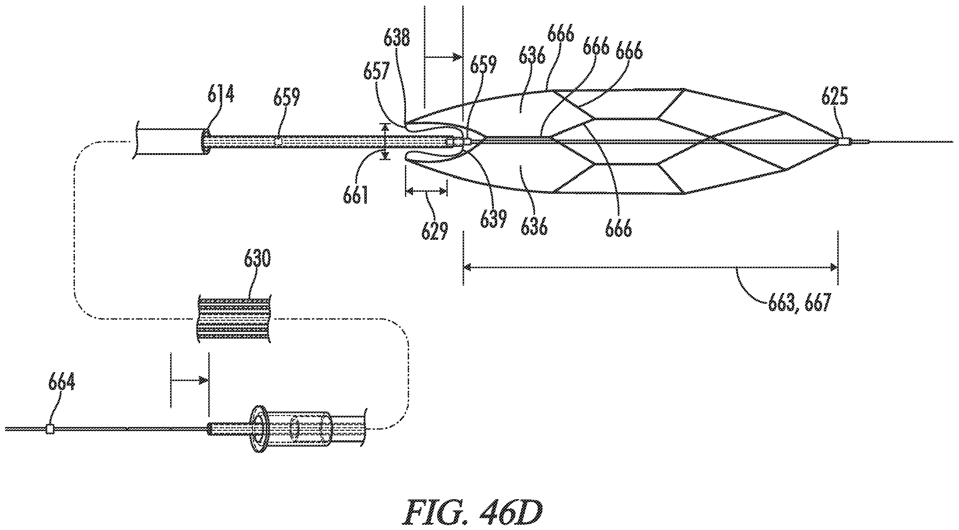

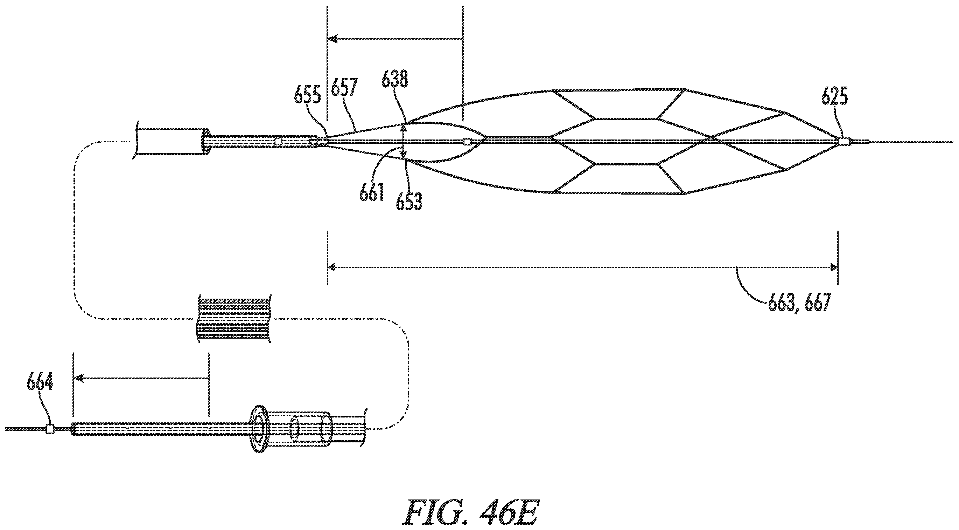

FIG. 46C exemplifies what is referred to as the gaping state of the basket **611.** To move the basket **611** from the relaxed state to the gaping state, a user merely pushes the proximal hub **639** distally towards the stationary distal hub **625.** This causes the proximal tether memory metal strips **657** to increase the height **661** of the distal basket **611** at the proximal-most crown **638.** The proximal tether memory metal strips **657** of the embodiment shown in FIGs. 46, 47 and 50 are relatively short. The proximal tether memory metal strips **657** are relatively thin compared to the memory metal strips **666** that make up the proximal cells **636**, which makes the proximal tether memory metal strips **657** easy to bend. Preferably, in the gaping state of short, relatively thin proximal tether memory metal strips **657**, the proximal memory metal strips **657** are substantially perpendicular (e.g., about 75 to about 105 degrees) relative to the longitudinal axis of the pull wire **646.**

FIG. 46D exemplifies what is referred to as the distal collapsed state. To move the basket **611** from the gaping state to the distal collapsed state, a user merely pushes the proximal hub **639** distally towards the stationary distal hub **625.** This causes the proximal tether memory metal strips **657** to reduce the height **661** of the distal basket at the proximal-most crown **638**, which in certain embodiments, allows the user to recapture the system **610** in the microcatheter **632.** This is particularly helpful if the system **610** was deployed at the wrong location. Preferably, the pull wire **643** includes a distal stop **660**, which prevents the proximal hub **39** from moving too far distally and breaking.

FIG. 46E also exemplifies the proximal collapsed state. To move the basket **611** from the relaxed state to the proximal collapsed state, a user merely pulls the proximal hub **639** away from the stationary distal hub **625.** This causes the proximal tether memory metal strips **657** to reduce the height **661** of the distal basket at the proximal-most crown **638**, which in certain embodiments, allows the user to recapture the system **610** in the microcatheter **632.** This is particularly helpful if the system **610** was deployed at the wrong location. Preferably, the pull wire **643** includes a middle stop **655**, which prevents the proximal hub **639** from moving too far proximally.

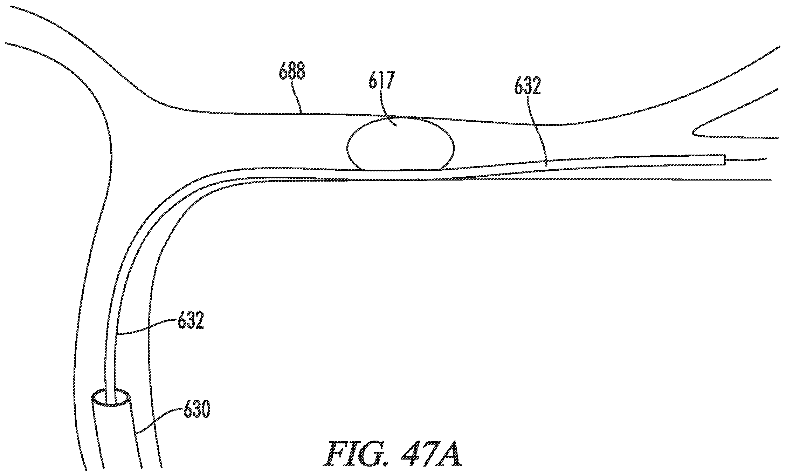

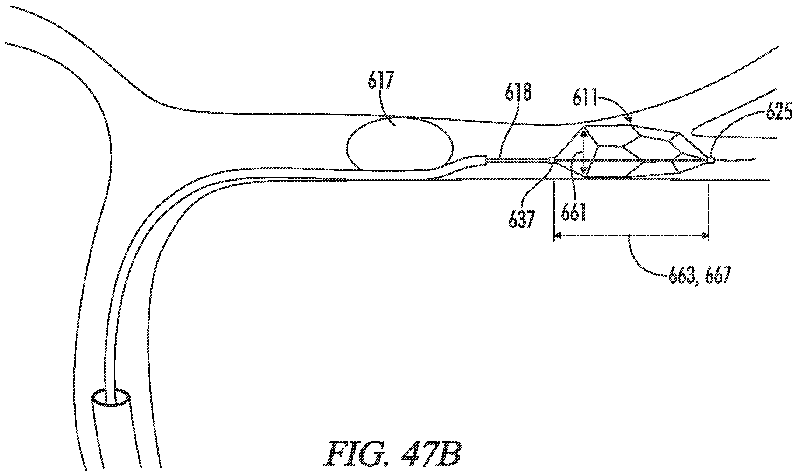

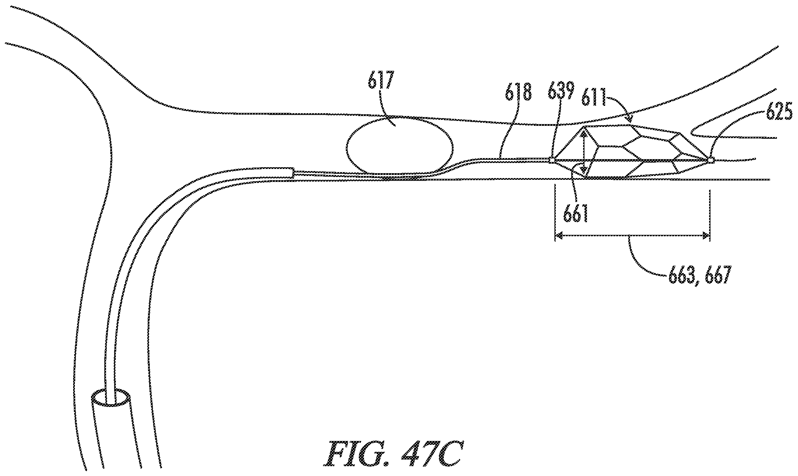

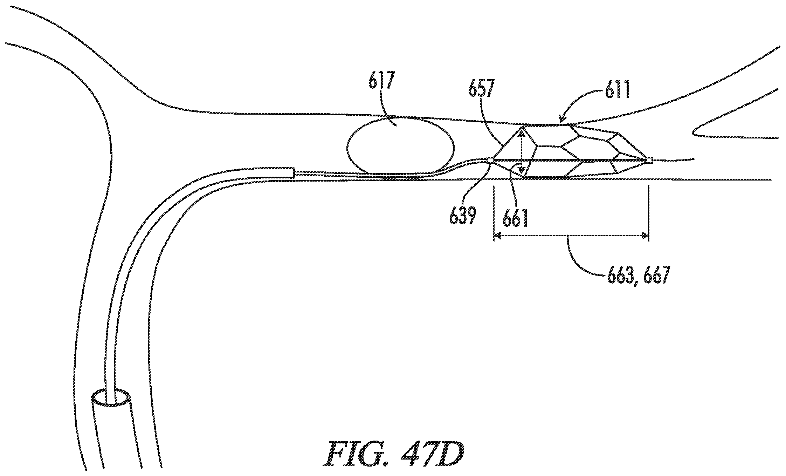

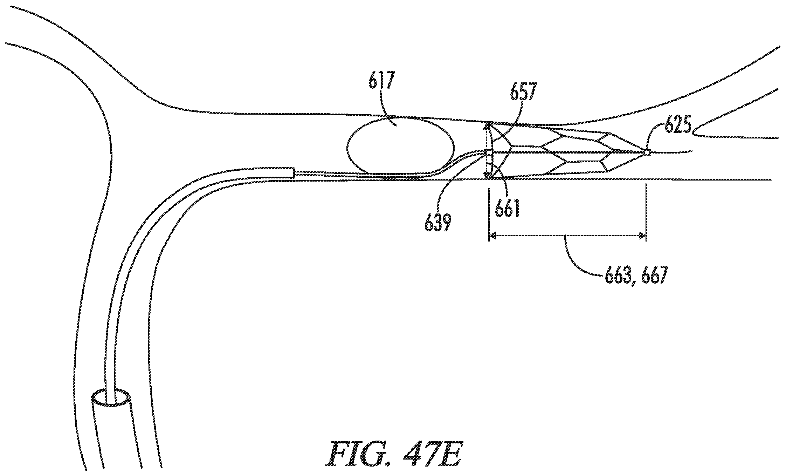

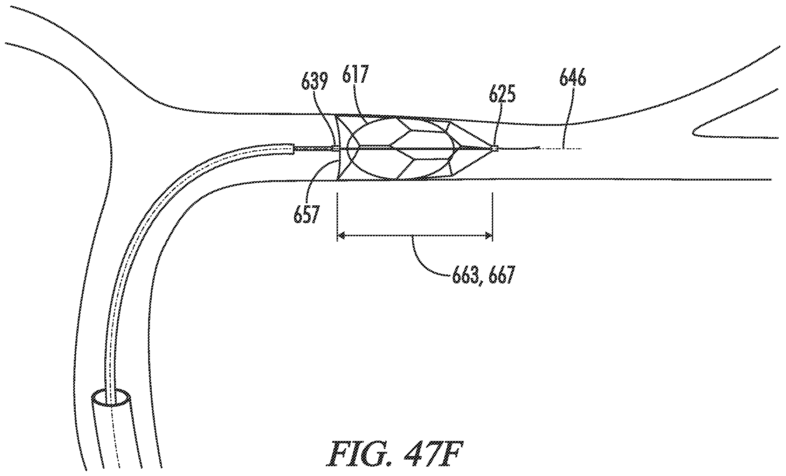

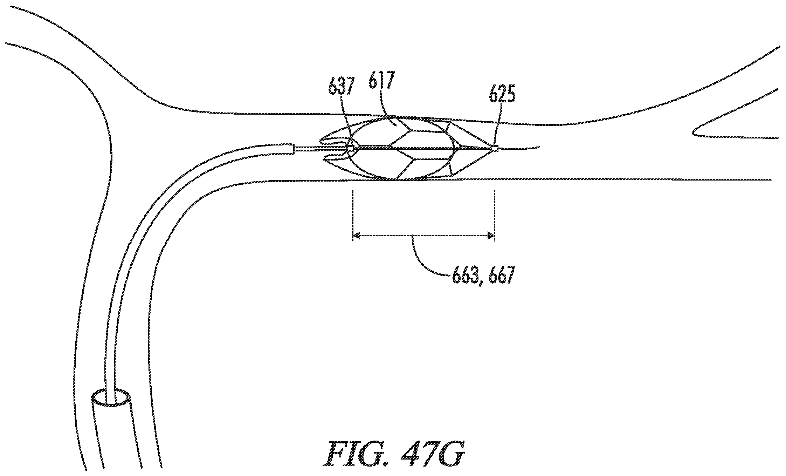

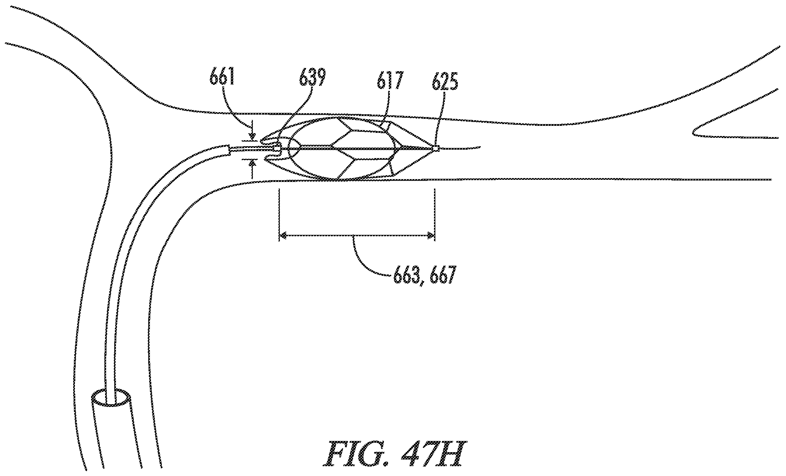

FIG. 47 illustrates use of the basket system shown in FIG. 46 in an intracranial artery **688.** As shown in FIG. 47A, first the guide catheter **630** is deployed proximal to the clot **617.** The microcatheter **632** is then advanced distally beyond the clot **617.** The basket **611** is collapsed inside the microcatheter **632.** Next, as shown in FIG. 47B, the microcatheter **632** is moved proximally to deploy the basket **611** distal to the clot **617.** The basket **611** is now in the relaxed state. Next, as shown in FIG. 47C, the user continues to move the microcatheter **632** proximally. Then, as shown in FIG. 47D, the basket **611** is moved closer to the clot **617** by a user pulling the pull wire **643** and coaxial tube **618** proximally at the same time. Then, as shown in FIG. 47E, the user uses the coaxial tube **618** to move the proximal hub **639** toward the distal hub **625** so that the basket **611** is in the gaping state. The gaping state is particularly important, as it believed to allow the basket **611** to capture the clot **617** without having the clot **617** collapse the basket **611.** Then, as shown in FIG. 47F, the basket **611** is moved proximally over the clot **617.** Then, as shown in FIG. 47G, the coaxial tube **618** is moved further proximally to close the proximal end **669** around the clot **617.** The system **611** is moved proximally by moving the pull wire **643** and the coaxial tube **618** proximally simultaneously.

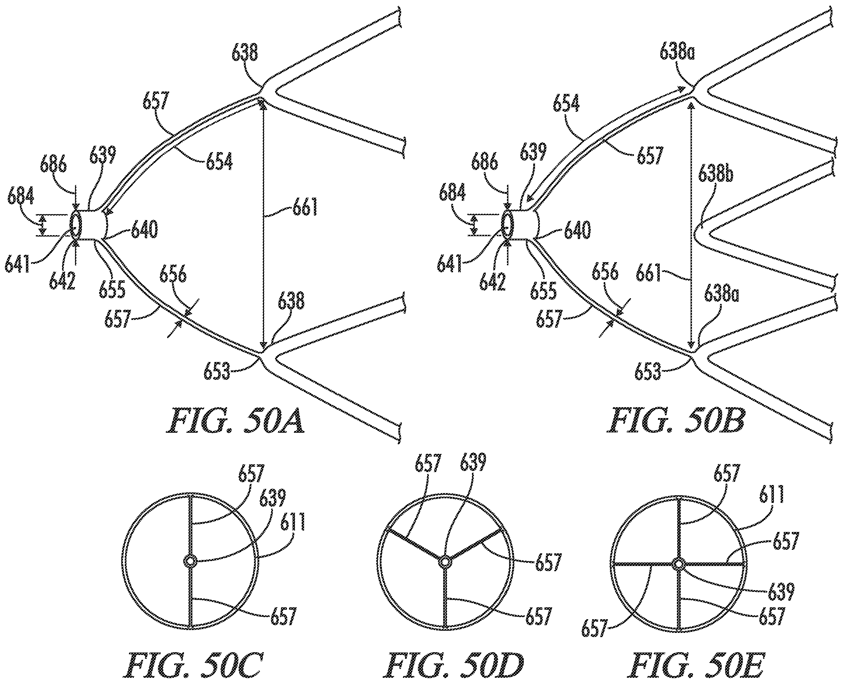

FIG. 50A shows a close-up view of the proximal end of the basket **611**, including the proximal tube interior **641**, the attachment of the proximal tether memory metal strips **657** at the distal end **655** of the proximal hub **639**, and the proximal crowns **638** of the proximal cells **636.** In FIG. 50A, all proximal crowns **638** of the proximal cells **636** are attached to a proximal tether memory metal strip **657.** FIG. 50B illustrates an alternative embodiment in which two proximal crowns **638a** of a proximal cell **636** (the top and bottom crowns **638a**) are attached to a proximal tether memory metal strip **657** and one proximal crown **638b** of a proximal cell **636** is not attached to a proximal tether memory metal strip **657.** FIGs. 50C - 50E illustrate that the basket system may include, for example, between 2 and 4 proximal tether memory metal strips **657.**

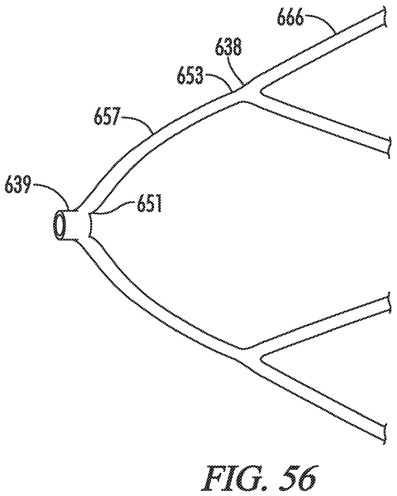

FIG. 56 illustrates a side, perspective view of a basket system **610** with relatively thick and short proximal tether memory metal strips **657** (i.e., the proximal tether memory metal strips **657** are slightly thicker than the memory metal strips **666** making up the proximal cells **636.**

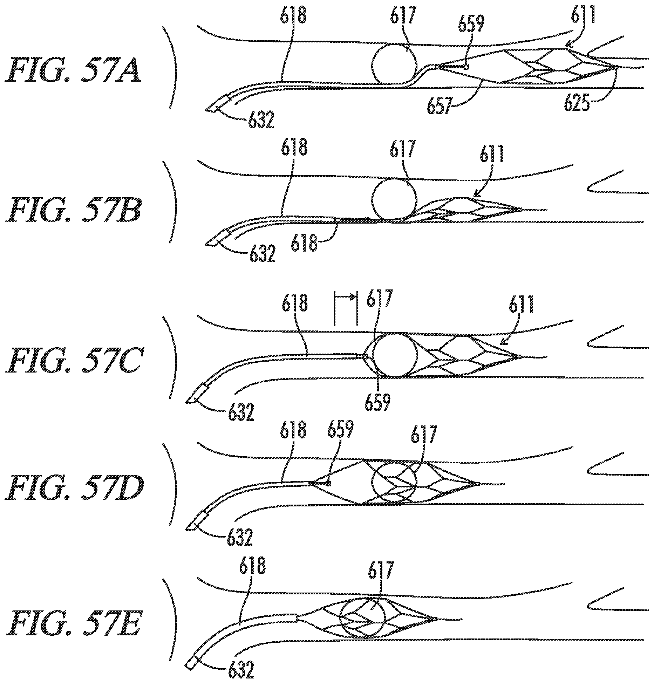

In, FIG. 57 the proximal tether memory metal strips **657** are thicker than the memory metal strips **666** forming the proximal cells **636** of the distal basket **611.** In these embodiments with thicker proximal tether memory metal strips **657**, the proximal tether memory metal strips **657** resist deforming when the proximal hub **635** is translated distally toward the stationary distal hub **629** and instead the proximal tether memory metal strips **657** are bowed out laterally, dissecting through or around the clot **617** and centering, buttressing and strengthening the opening of the basket **611.** In particular, as illustrated in FIG. 57A, the basket **611** is deployed distal to the clot **617.** The basket **611** is move distally so that the clot **617** partially collapses the proximal tether memory metal strips **657.** *See* FIG. 57B. The proximal hub **614C** is moved distally to slice the proximal tether memory metal strips **657** through the clot **617.** *See* FIG. 57C. The basket **611** is moved proximally to ensnare the clot **617.** *See* FIG.57. The tether proximal memory metal strips **657** are partially withdrawn into the microcatheter **632** and the system is removed from the body. *See* FIG. 57E.

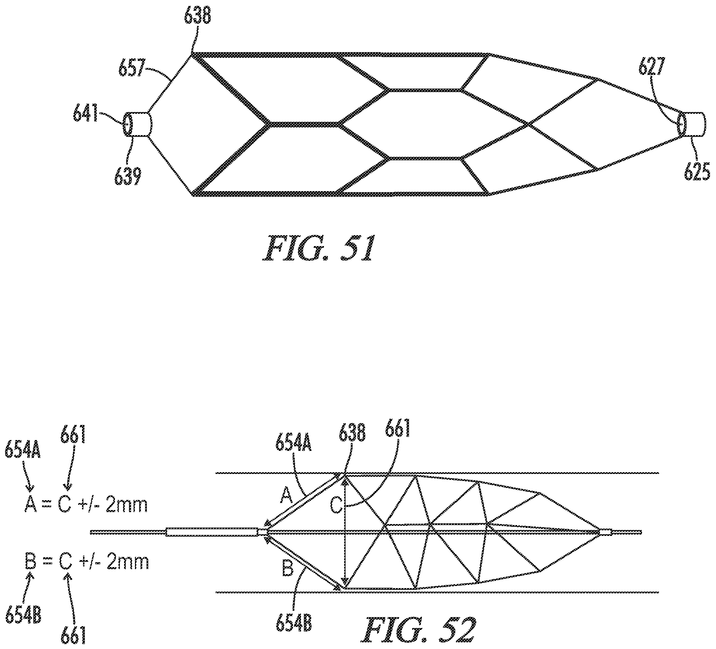

FIG. 51 illustrates a similar to basket system **610** to FIGs. 46, 47 and 50. In FIG. 51, the proximal tether memory metal strips **657** are relatively thin and short and the proximal memory metal strips making up the remainder of the basket are thickest at the proximal-most crown **38** and decrease gradually along the distal basket length **667.**

FIG. 52 illustrates a similar to basket system **610** to FIGs. 46, 47, 50, and 51. Again, the proximal tether memory metal strips **657** are relatively thin and short. In this embodiment, the length **654A** of the first proximal memory metal strip **657A** and the length **654B** of the second proximal memory metal strip **657B** are equal to the height **661** of the basket **611** in the relaxed state, as measured at the proximal-most crown **638**, plus or minus two mm. Thus, if for example, the height of the vessel **688** is 4 mm and the length of the proximal tether memory metal strips is 3 mm, the height **661** of the basket **611** as measured at the proximal-most crown **638** could be 4 mm. This is believed to allow the basket **611** in the gaping state to fill the vessel **688.**

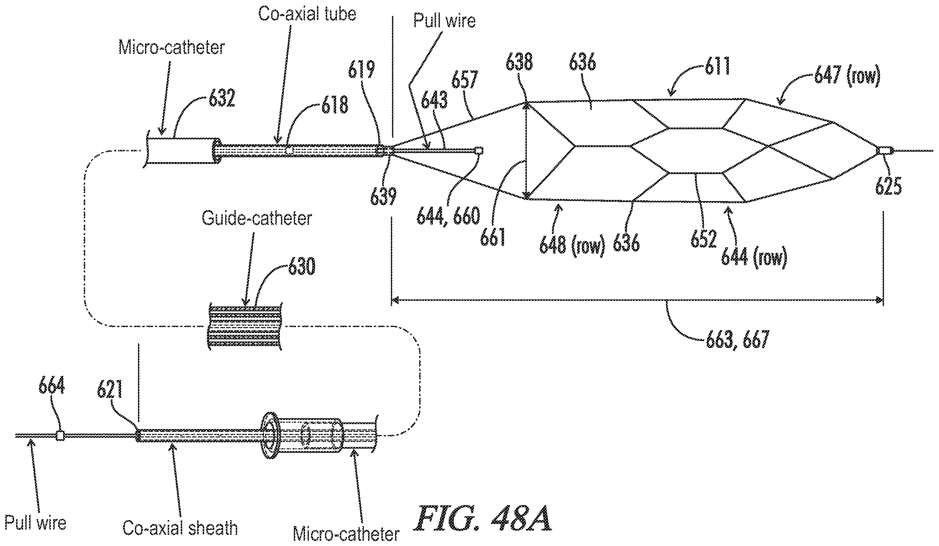

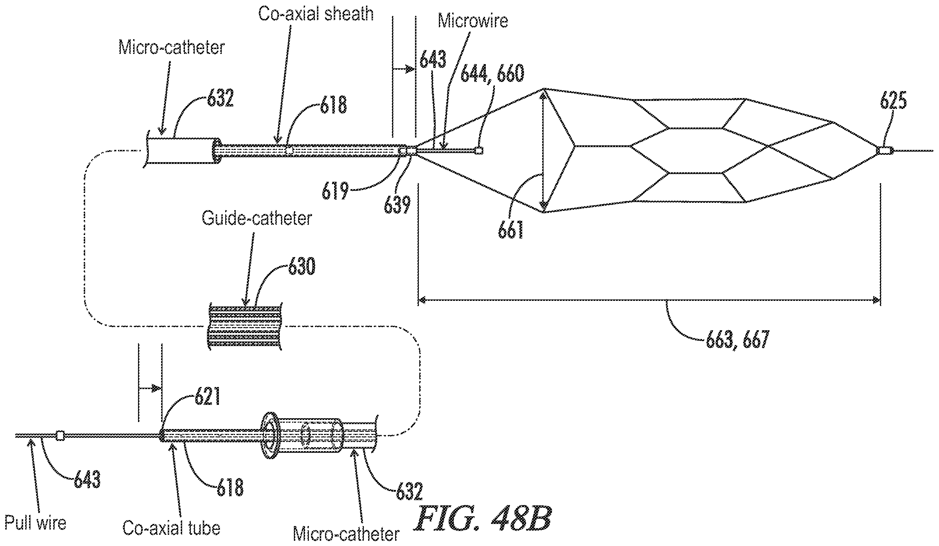

FIG. 48 illustrates another embodiment of the basket system **610.** In this embodiment, the pull wire **643** does not extend through the entire basket **611** but rather ends at distal stop **660.** As compared to the embodiment of FIGs. 46, 47 and 50, the proximal tether memory metal strips **657** of the embodiment of FIG. 48 are about the same thickness as the thickness **656** of the proximal cell memory metal strips **666**, which makes the basket **611** relatively rigid and the proximal tether memory metal strips **657** relatively inflexible, which may be desired for certain applications. As shown, moving the basket **611** from the relaxed state (see FIG. 48A) to the gaping state by moving the coaxial tube **618** proximally does not greatly enhance the basket height **661** in this embodiment due to the rigidity.

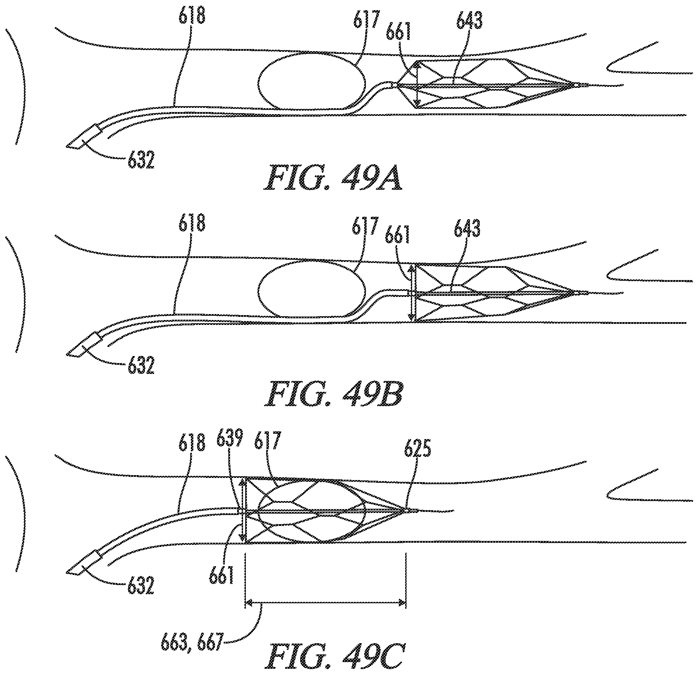

FIGs. 49A-49C illustrate stepwise deployment and use of a basket system **610** with three relatively thin and short proximal tether memory metal strips **657**; the system **610** is deployed in a blood vessel **688** to retrieve a clot **617.**

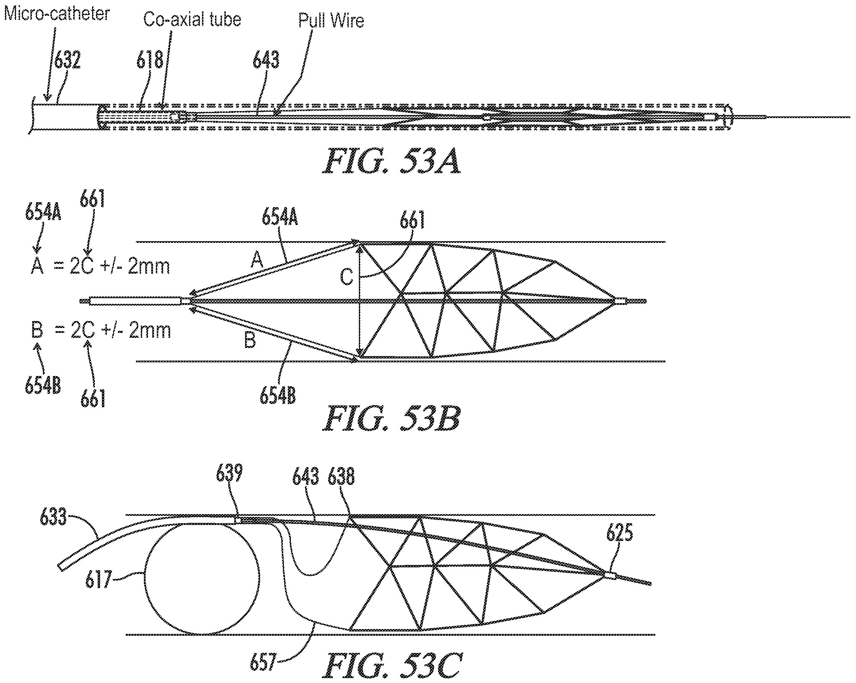

FIG. 53 illustrates another embodiment of the basket system **610.** In this embodiment, the proximal tether memory metal strips **657** are relatively thin (like the embodiment of FIGs. 46, 47 and 50) but longer than the FIG. 46, 47, and 50 prior embodiment. This length allows the basket **611** to open asymmetrically around the clot **617** (see FIG. 53C), which is helpful if the microcatheter **632** and pull wire **643** are pushed against the vessel **688** wall by the clot **617.** As shown in FIG. 53B, the length **654A** of the first proximal tether memory metal strip **657A** also may be two times the height **661** of the basket **611**, as measured at the proximal-most crown **638** plus or minus 2 mm and the length **654B** of the second proximal tether memory metal strip **657B** may be two times the height **661** of the basket **611** plus or minus 2mm. Thus, for example, if the vessel **688** has a height of 4 mm and the length **654A** and **654B** of the proximal tether memory metal strips **657A** and **657B** are 7 mm each, the height **661** of the distal basket **611** as measured at the proximal-most crown may be set to for example 4 mm in the relaxed state.

It will be noted that the proximal end of the system **610** is shown at the bottom end of FIGs. 45-62 and the distal end of the system **610** is shown at the top end of FIGs. 45-62 because a principal use of the system **610** is to remove a blood clot **617** from a human intracranial artery **688**, in which case the system **610** generally will enter the artery **688** at its proximal end by the surgeon entering the patient's body near the groin and pushing the catheter **632** towards the brain. The diameter of human arteries **688** generally decrease from their proximal end to their distal end. However, when used in other types of lumens, the distal basket **611** may be located proximally relative to the catheter **632** as the term proximally and distally are used in that lumen.

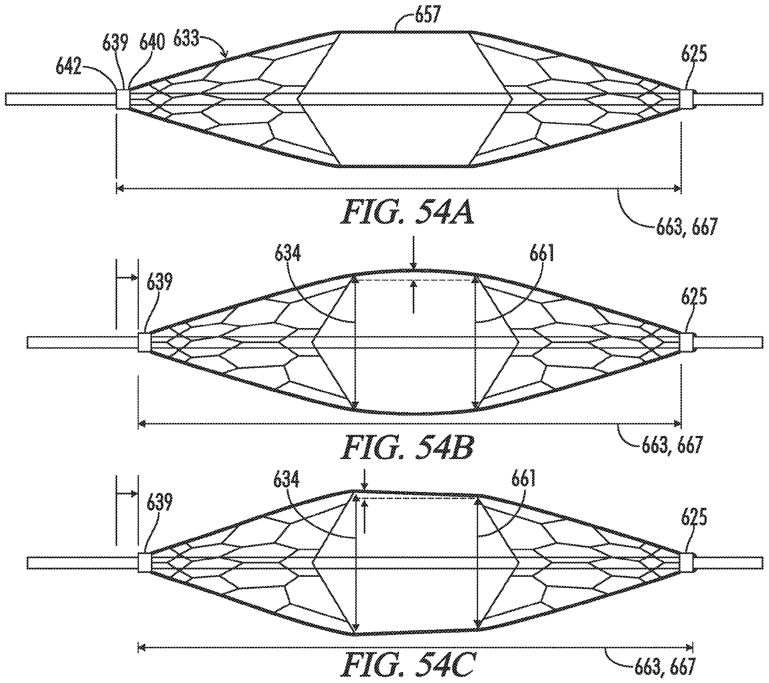

FIG. 54 illustrates another embodiment of a basket system **611.** In this embodiment, the system **611** includes a proximal hub **639** that is slideable towards a distal hub **625** (similar to the prior embodiments). The difference is that the tether memory metal strips **657** actually join the proximal basket **633** and the distal basket **611.** More particularly, the proximal basket **633** is comprised of a plurality of proximal cells **636** attached to the proximal hub **639** and a plurality of distal cells **622** and the distal basket is comprised of a plurality of proximal cells **636** attached to the proximal hub **639** and a plurality of distal cells **622** and the tether memory metal strips **657** join a distal crown **623** of a distal cell **622** of the distal basket **611** with a proximal crown **638** of a proximal cell **636** of the proximal basket **633.** As shown, in FIG. 54B, movement of the proximal hub **639** toward the distal hub **625** increases the height **634** of the proximal basket **633** as measured at the distal-most crown **623** of the distal basket **611.**

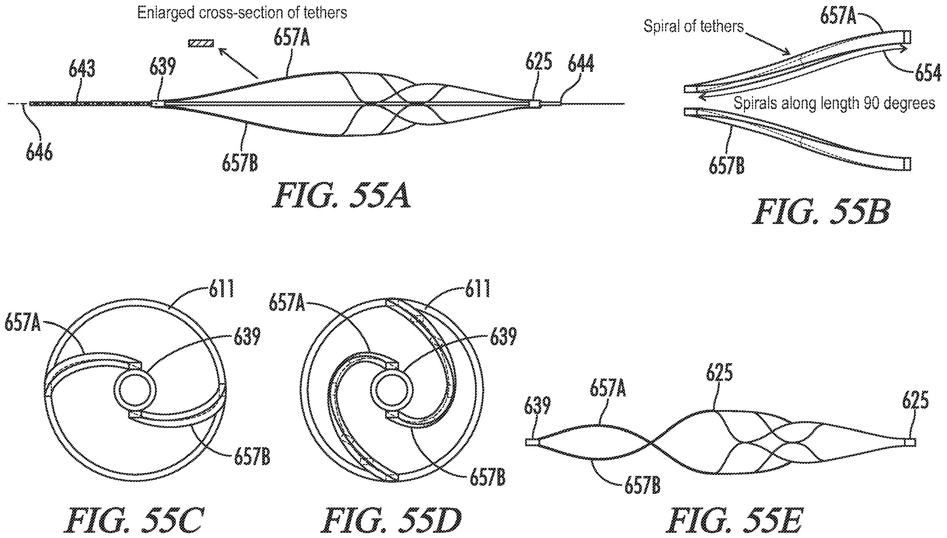

FIG. 55A and 55B illustrate an embodiment of the proximal tether memory metal strips **657** rotating about said pull wire longitudinal axis **646** such that the distal end **653** of a proximal tether memory metal strip **657** is located between about 90 and about 270 degrees relative to said proximal end **655** of the same proximal tether memory metal strip **657.** In addition, the proximal tether memory metal strips **657** may rotate around their longitudinal axis **654** such that a distal end **653** of a proximal tether memory metal strip **657** rotates about 90 and about 270 degrees around this tether longitudinal axis **654** from the distal end **653** to the proximal end **655** of the same proximal memory metal strip **657.** FIG. 55C illustrates an exemplary embodiment, where the proximal end **655A** of the first proximal tether memory metal strip **657A** is located attached to the proximal tube **639** at the 12 o'clock position and the distal end **653A** of the same proximal tether memory metal strip **657A** is attached to a proximal-most crown **639** at the 9 o'clock position. In addition, the second proximal tether memory metal strip **657B** is located attached to the proximal tube **639** at the 6 o'clock position and the distal end **653B** of the same proximal tether memory metal strip **657b** is attached to the other proximal-most crown **639** at the 3 o'clock position. FIGs. 55D and 55E illustrate a similar embodiment with the proximal tether memory metal strips **657A** and **657B** rotating 180 degrees. FIG. 55D illustrates an exemplary embodiment, where the proximal end **655A** of the first proximal tether memory metal strip **657A** is located attached to the proximal tube **639** at the 12 o'clock position and the distal end **653A** of the same proximal tether memory metal strip **657A** is attached to a proximal-most crown **639** at the 6 o'clock position. In addition, the second proximal tether memory metal strip **657B** is located attached to the proximal tube **639** at the 6 o'clock position and the distal end **653B** of the same proximal tether memory metal strip **657b** is attached to the other proximal-most crown **639** at the 12 o'clock position.

In some examples, the basket system **610** is prepared by a process that includes one or more of the following steps, as illustrated in FIG. 45:
a) providing a single tube **668** comprised of a memory metal such as nitinol, the single tube **668** having an exterior, a substantially hollow interior, a wall **682** separating the exterior from the substantially hollow interior, an open proximal end **674**, an open distal end **676**, a middle portion **678** between the open proximal end **674** and the open distal end **676** (see FIG. 45A);
b) cutting the wall of the middle portion **678** with a laser **680** (see FIG. 45B);
c) removing the pieces of the middle portion cut by the laser **680** to form a basket system **610** comprising a proximal tube **639** comprising a hollow interior **641** extending through said proximal tube **639**, said proximal tube having a proximal end **642** and a distal end **640**, a distal tube **625** comprising a hollow interior **641** extending through said distal tube **625**, and a middle portion **678** located between said proximal tube **639** and said distal tube **625** and comprising a plurality of proximal tether memory metal strips **657**, each proximal tether memory metal strip **657** having a proximal end **655** attached to the distal end **640** of the proximal tube **639** and a distal end **653**;
d) altering the shape of the middle portion **678** using a mandrel and allowing the middle portion **678** to expand relative to the distal tube **676** and proximal tube **674** to form a basket that includes cells **623** and **636**;
e) quenching the middle portion **678** at room temperature;
f) removing the mandrel from the middle portion **678**;
g) mechanically or chemically electropolishing the middle portion **678** to remove oxides (see FIG. 45C);
h) inserting a pull wire **643** through said proximal tube interior **641** so that said proximal tube **639** is slideable along at least a portion of said pull wire **643**, said pull wire **643** having a proximal end **645** and a distal end **644**; and
i) attaching said pull wire **643** to said distal tube **625** so that the distal tube **625** is not slideable along the pull wire **643** but instead the distal tube **625** moves with the pull wire **643** (see FIG. 45D).

In other examples, steps h) and i) above replaced with the steps of inserting a pull wire comprising a proximal end, a distal end, a stop located adjacent to said distal end, through said proximal tube interior, said stop having a width and/or height that is greater than said proximal tube interior, said stop located distal relative to said proximal tube interior, so that said proximal tube is slideable distally until the proximal hub reaches said stop, said pull wire not contacting said distal tube; and attaching a leader wire to said distal tube.

In some examples, the middle portion **678** is expanded by heating the mandrel and the middle portion **678** by, for example, placing the mandrel and the middle portion **678** in a fluidized sand bath at about 500°C for about 3 to about 7 minutes. As the middle portion **678** is heated, the heating causes the crystalline structure of the memory metal tube **668** to realign. Preferably, the mandrel is tapered (e.g., substantially conical or bullet in shape) so that the portion of the distal basket **611** formed from the middle portion **678** tapers from the proximal-most crown **638** to the distal end **666.** Preferably, the proximal and distal ends of the tube **674** and **676** are not shape set by the mandrel and are not cut by the laser **680** so that the proximal and distal ends **674** and **676** do not change in shape and only slightly expand in size under heating and return to the size of the native tube **668** after the heat is removed. Preferably, the laser cuts are programmed via a computer. To ensure that the laser cuts only one surface of the tube wall at the time (and not the surface directly opposite the desired cutting surface), the laser **680** is preferably focused between the inner and outer diameter of the desired cutting surface and a coolant is passed through the memory metal tube **668** so that the laser **680** cools before reaching the surface directly opposite the desired cutting surface.

The portions of the wall not cut by the laser **680** create the proximal and distal tubes **674** and **676** as well as the other components of the distal basket **611**, and memory metal strips **657** and **666**, as described.

Preferably, the memory metal selected for the native tube **668** has a heat of transformation below average human body temperature (37°C) so that the distal basket **611** has sufficient spring and flexibility after deployment from the catheter **632** in the human blood vessel **688.**

In some embodiments, the native tube **668** (and hence the distal and proximal tubes **674** and **676**) have an outer diameter of less than about 1.33 mm (4 French), e.g., a diameter of about 0.33 mm to about 1.33 mm (about 1 to about 4 French). In some embodiments, the diameter of the pull wire **643** is between about 0.20 mm (0.008 inches) and about 1.30 mm (0.051 inches), as noted above, and in such embodiments, the diameter of the pull wire **43** may be approximately equal to the inner diameter **672** of the native nitinol tube **668.**

Without being bound by any particular theory, it is believed that manufacturing the distal basket **611** from a single memory metal tube **668** provides ease of manufacturing and safety from mechanical failure and provides tensile strength necessary for the system **610** to remove hard thrombus **617** and other obstructions.

In some examples, the exemplary method further includes providing a coaxial tube **618**, said coaxial tube **618** comprising a hollow interior **620** receiving said pull wire **643**, a proximal end **621**, and a distal end **619**, and attaching said distal end **619** of said coaxial tube **643** to said proximal tube **625**. In some examples, the exemplary method of attaching said distal end **619** of said coaxial tube **618** to said proximal tube **625** comprises welding said distal end **619** of said coaxial tube **618** to said proximal tube **625.** In other examples, the exemplary method of attaching said distal end **619** of said coaxial tube **618** to said proximal tube **625** comprises shrink wrapping said distal end **619** of said coaxial tube **618** to said proximal tube **625.** In other examples, the exemplary method of attaching said distal end **619** of said coaxial tube **618** to said proximal tube **625** comprises gluing said distal end **619** of said coaxial tube **618** to said proximal tube **625.**

Optionally, after step e, the basket **611** further comprises a row **648** of proximal cells **636**, each proximal cell **636** defined by a plurality of memory metal strips **666** and comprising a proximal crown **638** located at a proximal end of the cell **636** and pointing in the proximal direction and a distal crown **624** located at a distal end of the cell and pointing in the distal direction and further wherein each of said proximal crowns **638** of said proximal cells **636** is attached to a distal end **653** of a proximal tether memory metal strip **657**. Optionally, after step e, the basket **610** further comprises a row **647** of distal cells **622** located distal to said proximal cells **636** and connected to said distal crowns **624** of said proximal cells 636, each distal cell **622** defined by a plurality of memory metal strips **666** and comprising a proximal crown **637** located at a proximal end of the cell **622** and pointing in the proximal direction and a distal crown **623** located at a distal end of the cell **622** and pointing in the distal direction, and further wherein the number of distal cells **622** is twice the number of proximal cells **636.** Optionally, after step e, the basket system **610** further comprises a row **649** of strut memory metal strips **652**, each strut memory metal strip **652** having a proximal end **651** attached to a distal crown **624** of a proximal cell **636** and a distal end **650** attached to a proximal crown **637** of a distal cell **622.** Optionally, the basket **611** comprises no welded components and said proximal tether memory metal strips **657** are integral with said proximal cell crowns **638.**

Optionally, after step e, the basket system **611** comprises between two and four proximal tether memory metal strips **657.** Optionally, prior to cutting the memory metal tube **668**, the memory metal tub **668** has an outer diameter **686** that is from about 0.28 mm (0.011 inches) to about 1.37 mm (0.054 inches) and an inner diameter **684** that is from about 0.20 mm (0.008 inches) to about 1.30 mm (0.051 inches). Optionally, after step e), the proximal tube **639** and distal tube **625** have an outer diameter that is from about 0.51 mm (0.02 inches) to about 0.76 mm (0.03 inches) and an inner diameter that is from about 0.25 mm (0.01 inches) to about 0.51 mm (0.02 inches). Optionally, the exemplary method further includes placing said basket **611** inside a catheter **632** comprised of a biocompatible material. Optionally, the exemplary method further includes the steps of placing the basket **611** inside a lumen **688** of an animal and using the basket to retrieve an object **617** located inside said lumen **688.**

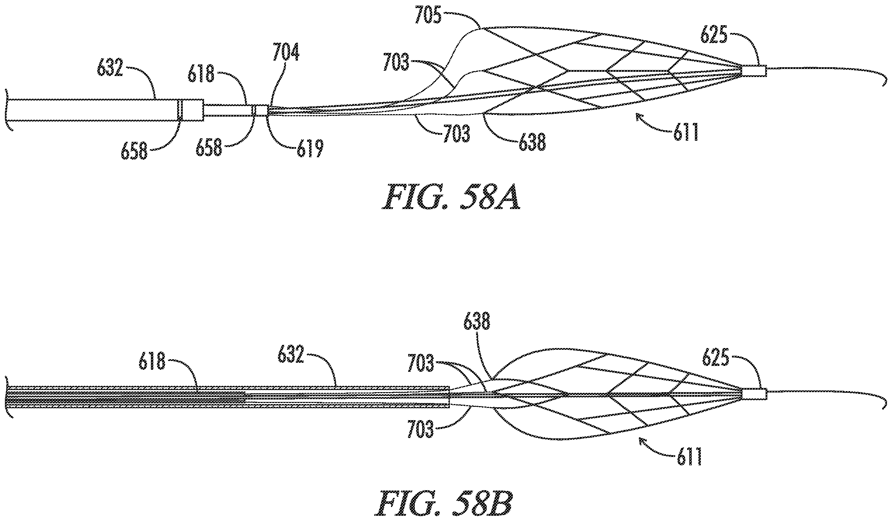

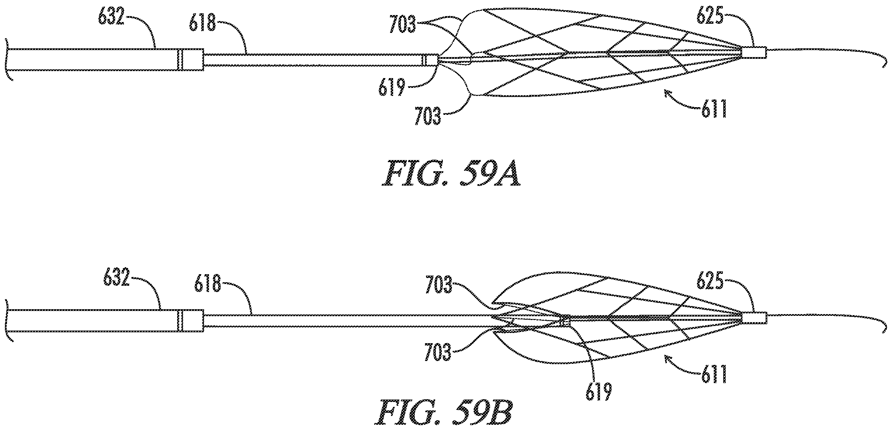

In other arrangements, as shown in FIGs. 58-60, the basket system **610** does not include a proximal hub **639** and the system **610** includes a plurality of cords **703** (e.g., 2-4 cords **703**) instead of or in addition to said proximal tether memory metal strips **657.** For example, FIG. 15-17 shows a first set of arrangements, where soft cords made of rubber, nylon, suture material, braided catheter material, platinum coils, and ultrathin nitinol for example, are used. The cords **703** have a proximal end **704** attached to the distal end **619** of the coaxial tube **618** and a distal end **705** attached to a proximal crown **638** of a proximal cell **636.** FIG. 58 illustrates one arrangements in which the cords **703** are relatively long. FIG. 59 illustrates another arrangements in which the cords **703** are relatively short.

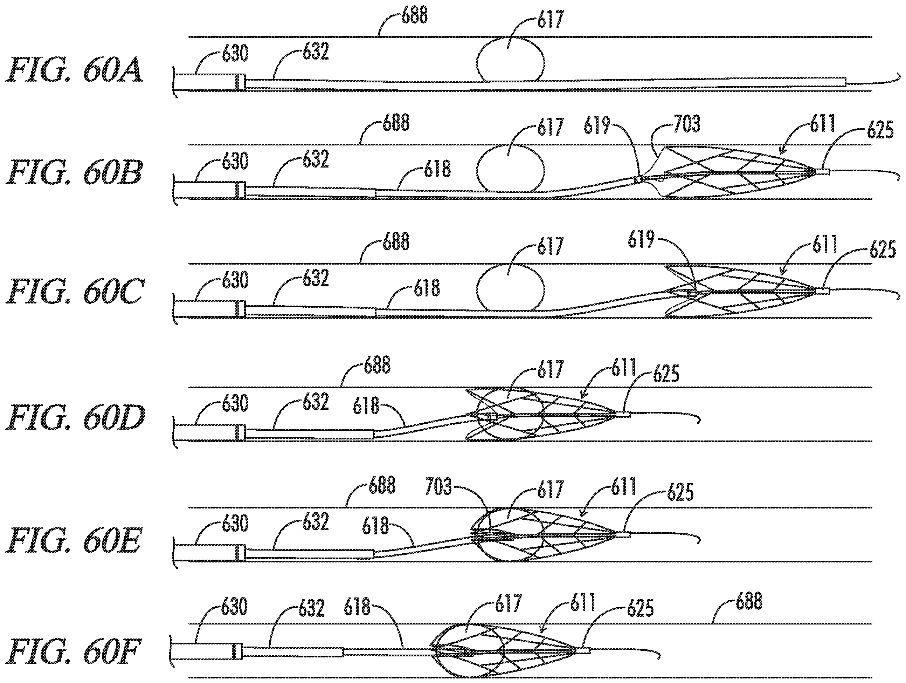

The system **610** may be used in an exemplary method that includes
a) providing the system **610**;
b) positioning the system **610** in said lumen **688**, said basket **611** located in said catheter **632** in a collapsed state (see FIG. 60A);
c) deploying said distal basket **611** from said distal end **614** of said catheter **632** so that said proximal crowns **638** of said proximal cells **636** are distal to said obstruction **617**;
d) allowing said distal basket **611** to move to said relaxed state (see FIG. 60B);
e) moving said coaxial tube **618** distally relative to said distal hub **625** so that said coaxial tube **618** moves distally to the proximal-most crown **638** (see FIG. 60C);
f) moving said distal basket **611**, said pull wire **643** and said coaxial tube **618** proximally simultaneously so that said distal basket **611** moves over said obstruction **617** (see FIG. 60D);
g) moving said coaxial sheath **618** distally relative to said distal hub **625** so that said distal basket height **661**, as measured at the proximal-most crown **638**, decreases and said coaxial tube **618** is closer to said distal hub **625** as compared to the proximal-most crown **638** (see FIG. 60E); and
h) removing said distal basket **611** and said obstruction **617** from said lumen **688** (see FIG. 60F).

In other examples, steps g-h above are replaced with the steps below:
g) moving said coaxial sheath **618** proximally relative to said distal hub **625** so that said distal basket height **661**, as measured at the proximal-most crown **661**, decreases;
h) moving said catheter **632** distally relative to said distal hub **625** so that said catheter **632** re-sheaths said coaxial sheath **618** and partially re-sheaths said cords, thereby decreasing said distal basket height **66**1, as measured at the proximal-most crown **638**;
i) removing said distal basket **611** and said obstruction **617** from said lumen **688.**

As shown, an advantage of this embodiment is that the cords **703** move distally to the proximal-most crowns **638** so they do not obstruct entry way of the clot **617** into the distal basket **611.**

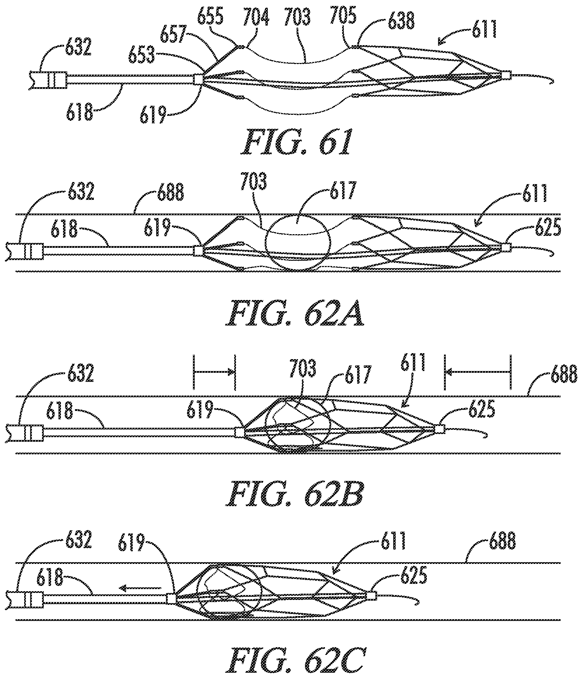

In other embodiments, as shown in FIGs. 61 and 62, the system **610** includes cords **703** and proximal tether memory metal strips **657.** In such embodiments, the proximal tether memory metal strips **657** have a proximal end **655** attached to the distal end **619** of the coaxial tube **618.** The cords have a proximal end attached to the distal end **653** of the proximal memory metal strips **657** and a distal end attached to a proximal crown **638** of a proximal cell **636.**

The system **610** may be used in an exemplary method of removing an object from an interior lumen **688** of an animal, said lumen **688** having an interior wall forming said lumen **688** that includes:
a) providing the system **610**;
b) positioning the system **610** in said lumen **688**, said basket **611** located in said catheter **632** in a collapsed state;
c) deploying said distal basket **611** from said distal end **614** of said catheter **632** so that said proximal crowns **638** of said proximal cells **636** are distal to said obstruction **617**, said coaxial sheath **618** is proximal to said obstruction **617**, said proximal tether memory metal strips **657** are proximal to said obstruction **617**, and said cords are adjacent to said obstruction **617**;
d) allowing said distal basket **611** to move to said relaxed state (see FIG. 62A);
e) moving said coaxial tube **618** distally relative to said distal hub **625** and moving said basket **611** proximally so that said proximal tether memory metal strips **657** move distally relative to the proximal-most crown **638** and said obstruction **617** is sandwiched between said proximal tether memory metal strips **657** and said proximal crowns **638** of said proximal cells **636** (see FIG. 62B); and
f) removing said distal basket **611** and said obstruction **617** from said lumen **688.**

Having now described the invention in accordance with the requirements of the patent statutes, those skilled in the art will understand how to make changes and modifications to the disclosed embodiments to meet their specific requirements or conditions. Changes and modifications may be made without departing from the scope of the invention, as defined and limited solely by the following claims. In particular, although the system has been exemplified for use in retrieving blood clots, the system may be used to retrieve other objects from animal lumens. In addition, the steps of any exemplary method described herein may be performed in any suitable order and steps may be performed simultaneously if needed.

Terms of degree such as "substantially", "about" and "approximately" as used herein mean a reasonable amount of deviation of the modified term such that the end result is not significantly changed. For example, these terms can be construed as including a deviation of at least ± 5% of the modified term if this deviation would not negate the meaning of the word it modifies.

## Claims

1. A system (200) for removing objects (270A, 270B, 270C) from an interior lumen (266) of an animal, the system (200) comprising:
a pull wire (202) having a proximal end (204) and a distal end (206);
a distal body (216) attached to the pull wire (202), the distal body (216) comprising an interior (222), a proximal end (218), a distal end (220), a distal body length (226) extending from the proximal end (218) to the distal end (220), a proximal hub (228) forming the proximal end (218) of the distal body (216), a plurality of proximal strips (252), a basket (246) comprised of a plurality of cells (248) formed by a plurality of basket strips (291), the basket (246) comprising a basket interior (292), each proximal strip (252) having a distal end (256) attached to a cell (248) and a proximal end (254), the proximal ends (254) of the proximal strips (252) converging at the proximal hub (228), the distal body (216) having a relaxed state wherein the distal body (216) has a first height (224) and a first width, and a collapsed state wherein the distal body has a second height and a second width, the second height less than the first height (224), the second width less than the first width; and
a catheter (208) having an interior (210), a proximal end (212) leading to the interior (210) and a distal end (214) leading to the interior (210), the catheter (208) comprised of a biocompatible material and configured to envelope the distal body (216) when the distal body (216) is in the collapsed state,
wherein, in the relaxed state, the basket (246) comprises a first pair of distal crowns (258A, 258B) not attached to another cell (248) of the basket (246) and pointing generally in the distal direction, the distal crowns (258A, 258B) in the first pair of distal crowns located approximately the same distance from the proximal hub (228) and between 150 degrees and 180 degrees relative to each other, and further wherein the basket (246) further comprises a second pair of distal crowns (258C, 258D) not attached to another cell (248) of the basket (246) and pointing generally in the distal direction, the second pair of distal crowns (258C, 258D) located distally relative to the first pair of distal crowns (258A, 258B), each of the distal crowns (258C, 258D) in the second pair of distal crowns located between 60 degrees and 90 degrees relative to a distal crown (258A, 258B) in the first pair of distal crowns, the distal crowns (258C, 258D) in the second pair of distal crowns located approximately the same distance from the proximal hub (228) and further wherein each of the distal crowns (258A, 258B, 258C, 258D) in the first and second pair of distal crowns comprises an x-ray marker (244), the x-ray marker (244) more visible under x-ray as compared to the basket strips (291) when the distal body (216) is located in a cranial blood vessel (266) inside the body of a human and the x-ray is taken from outside the human's body and further wherein each distal crown (258A, 258B, 258C, 258D) in the first and second pair of distal crowns forms part of a cell (250A, 250B, 250C, 250D) that further comprises a non free-floating proximal crown (260) pointing generally in the proximal direction,
wherein at least some of the basket strips (291) are located at a distal end of the basket (246), wherein each of the basket strips (291) located at the distal end of the basket (246) have a distal end, and
**characterised in that** the system comprises a single distal hub (236) comprising a proximal end (238), the proximal end (238) of the single distal hub (236) forming the distal end of the basket (246), wherein each of the distal ends of the basket strips (291) located at the distal end of the basket (246) converge at the single distal hub (236) and further wherein the distal body (216), in the relaxed state, comprises a tapered region in which the distal body height (224) and width decrease as the basket strips (291) located at the distal end of the basket (246) approach the single distal hub (236),
wherein the basket (246) comprises a plurality of cells (248) distal to the second pair of distal crowns (258C, 258D),
and further wherein each of the first and second pair of distal crowns (258A, 258B, 258C, 258D) is proximal to the single distal hub (236).

2. The system (200) of claim 1, wherein, the x-ray markers (244) are comprised of a material different than the material forming the basket strips (291).

3. The system (200) of claim 1, wherein, in the relaxed state, the basket interior (292) is hollow.

4. The system (200) of claim 1, wherein, in the relaxed state, the distal body (216) does not have another x-ray marker that is located approximately the same distance from the proximal hub (228) as the first pair of x-ray markers (244) and the distal body (216) does not have another x-ray marker that is located approximately the same distance from the proximal hub (228) as the second pair of x-ray markers (244).

5. The system (200) of claim 1, wherein each distal crown (258A, 258B, 258C, 258D) in the first and second pair of distal crowns forms part of an enlarged cell (262A, 262B, 262C, 262D) and further wherein the surface area of the enlarged cells (262A, 262B, 262C, 262D) in the relaxed state is greater than the surface area of the other cells (248) of the basket (246) and further wherein the enlarged cells (262A, 262B, 262C, 262D) are configured to allow a thrombus to pass therethrough and into the basket interior (292).

6. The system (200) of claim 1 wherein, in the relaxed state, the distal body (216) does not have another free distal-pointing crown that is located approximately the same distance from the proximal hub (228) as the first pair of distal crowns (258A, 258B) and the distal body (216) does not have another free distal-pointing crown that is located approximately the same distance from the proximal hub (228) as the second pair of distal crowns (258C, 258D).

7. The system (200) of claim 1 wherein the basket strips (291) are comprised of a memory metal.

8. The system (200) of claim 1, wherein each of the distal crowns (258A, 258B) in the first pair of distal crowns are configured to contact each other when an exterior, external compressive force is exerted on a distal crown (258A, 258B) of the first pair of distal crowns when the distal body (216) is in the relaxed state, and further wherein each of the distal crowns (258C, 258D) in the second pair of distal crowns are configured to contact each other when an exterior, external compressive force is exerted on a distal crown (258C, 258D) of the second pair of distal crowns when the distal body (216) is in the relaxed state.

9. The system (200) of claim 1 wherein the proximal hub (228) is located approximately in the center of the first height (224) and first width in the relaxed state.

10. The system (200) of claim 1, wherein the proximal end (254) of a first proximal strip (252) is located at least about 65 degrees relative to the distal end (256) of the first proximal strip (252), wherein the proximal end (254) of a second proximal strip (252) is located at least about 65 degrees relative to the distal end (256) of the second proximal strip (252), and further wherein the first and second proximal strips (252) intersect adjacent and distal to the proximal hub (228).

11. The system (200) of claim 1, wherein the basket (246) includes no free proximal crowns pointing generally in the proximal direction.

12. The system (200) of claim 1, wherein the proximal hub (228) is in the form of a proximal tube (228), wherein the basket (246), the proximal tube (228) and the proximal strips (252) are comprised of a memory metal, wherein the proximal tube (228) comprises a proximal end (230) and a distal end (232), and further wherein the proximal strips (252) are integral with the distal end (232) of the proximal tube (228).

13. The system (200) of claim 1, wherein the pull wire (202) is comprised of a biocompatible metallic material.

14. The system (200) of claim 1, wherein the catheter (208) is comprised of a polymeric material.

15. The system (200) of claim 1, wherein the distal body (216) further comprises a lead wire (286) extending distally from the single distal hub (236).

## Patentansprüche

1. System (200) zur Entfernung von Objekten (270A, 270B, 270C) aus einem Innenlumen (266) eines Tieres, wobei das System (200) Folgendes umfasst:
einen Zugdraht (202), der ein proximales Ende (204) und ein distales Ende (206) aufweist;
einen an dem Zugdraht (202) befestigten distalen Körper (216), wobei der distale Körper (216) ein Inneres (222), ein proximales Ende (218), ein distales Ende (220), eine distale Körperlänge (226), die sich vom proximalen Ende (218) zum distalen Ende (220) erstreckt, eine proximale Nabe (228), die das proximale Ende (218) des distalen Körpers (216) bildet, eine Vielzahl von proximalen Streifen (252), einen Korb (246), der aus einer Vielzahl von Zellen (248) besteht, die aus einer Vielzahl von Korbstreifen (291) gebildet sind, wobei der Korb (246) ein Korbinneres (292) aufweist, jeder proximale Streifen (252) ein an einer Zelle (248) befestigtes distales Ende (256) und ein proximales Ende (254) aufweist, die proximalen Enden (254) der proximalen Streifen (252) an der proximalen Nabe (228) zusammenlaufen, der distale Körper (216) einen entspannten Zustand, worin der distale Körper (216) eine erste Höhe (224) und eine erste Breite aufweist, und einen eingefallenen Zustand, worin der distale Körper eine zweite Höhe und eine zweite Breite aufweist, aufweist, wobei die zweite Höhe geringer ist als die erste Höhe (224), die zweite Breite geringer ist als die erste Breite; und
einen Katheter (208), der ein Inneres (210), ein zu dem Inneren (210) führendes proximales Ende (212) und ein zu dem Inneren (210) führendes distales Ende (214) aufweist, wobei der Katheter (208) aus einem biokompatiblen Material besteht und konfiguriert ist, den distalen Körper (216) zu umhüllen, wenn sich der distale Körper (216) im eingefallenen Zustand befindet,
wobei, im entspannten Zustand, der Korb (246) ein erstes Paar distaler Kronen (258A, 258B) umfasst, die nicht an einer anderen Zelle (248) des Korbes (246) befestigt sind und im Allgemeinen in die distale Richtung zeigen, wobei die distalen Kronen (258A, 258B) in dem ersten Paar distaler Kronen etwa in demselben Abstand von der proximalen Nabe (228) und zwischen 150 Grad und 180 Grad relativ zueinander angeordnet sind, und wobei weiter der Korb (246) weiter ein zweites Paar distaler Kronen (258C, 258D) umfasst, die nicht an einer anderen Zelle (248) des Korbes (246) befestigt sind und im Allgemeinen in die distale Richtung zeigen, wobei das zweite Paar distaler Kronen (258C, 258D) distal relativ zu dem ersten Paar distaler Kronen (258A, 258B) angeordnet ist, wobei jede der distalen Kronen (258C, 258D) in dem zweiten Paar distaler Kronen zwischen 60 Grad und 90 Grad relativ zu einer distalen Krone (258A, 258B) in dem ersten Paar distaler Kronen angeordnet ist, wobei die distalen Kronen (258C, 258D) in dem zweiten Paar distaler Kronen etwa in demselben Abstand von der proximalen Nabe (228) angeordnet sind und wobei weiter jede der distalen Kronen (258A, 258B, 258C, 258D) in dem ersten und zweiten Paar distaler Kronen einen Röntgenmarker (244) umfasst, wobei der Röntgenmarker (244) im Vergleich zu den Korbstreifen (291) unter Röntgenstrahlen sichtbarer ist, wenn der distale Körper (216) in einem kranialen Blutgefäß (266) im Inneren des Körpers eines Menschen angeordnet ist und die Röntgenaufnahme von außerhalb des Körpers des Menschen gemacht wird, und wobei weiter jede distale Krone (258A, 258B, 258C, 258D) in dem ersten und zweiten Paar distaler Kronen einen Teil einer Zelle (250A, 250B, 250C, 250D) bildet, die weiter eine nicht frei schwebende proximale Krone (260) umfasst, die im Allgemeinen in die proximale Richtung zeigt,
wobei mindestens einige der Korbstreifen (291) an einem distalen Ende des Korbes (246) angeordnet sind, wobei jeder der Korbstreifen (291), die am distalen Ende des Korbes (246) angeordnet sind, ein distales Ende aufweist, und
**dadurch gekennzeichnet, dass** das System eine einzelne distale Nabe (236), umfassend ein proximales Ende (238), umfasst, wobei das proximale Ende (238) der einzelnen distalen Nabe (236) das distale Endes des Korbes (246) bildet, wobei jedes der distalen Enden der Korbstreifen (291), die am distalen Ende des Korbes (246) angeordnet sind, an der einzelnen distalen Nabe (236) zusammenläuft, und wobei weiter der distale Körper (216) im entspannten Zustand eine konische Region umfasst, in der die distale Körperhöhe (224) und Breite abnehmen, wenn sich die Korbstreifen (291), die am distalen Ende des Korbes (246) angeordnet sind, der einzelnen distalen Nabe (236) nähern,
wobei der Korb (246) eine Vielzahl von Zellen (248) distal zu dem zweiten Paar distaler Kronen (258C, 258D) umfasst,
und wobei weiter jedes des ersten und zweiten Paares distaler Kronen (258A, 258B, 258C, 258D) zu der einzelnen distalen Nabe (236) proximal ist.

2. System (200) nach Anspruch 1, wobei die Röntgenmarker (244) aus einem Material bestehen, das sich von dem Material, das die Korbstreifen (291) bildet, unterscheidet.

3. System (200) nach Anspruch 1, wobei im entspannten Zustand das Korbinnere (292) hohl ist.

4. System (200) nach Anspruch 1, wobei der distale Körper (216) im entspannten Zustand keinen anderen Röntgenmarker aufweist, der etwa in demselben Abstand von der proximalen Nabe (228) angeordnet ist wie das erste Paar Röntgenmarker (244), und der distale Körper (216) keinen anderen Röntgenmarker aufweist, der etwa in demselben Abstand von der proximalen Nabe (228) angeordnet ist wie das zweite Paar Röntgenmarker (244).

5. System (200) nach Anspruch 1, wobei jede distale Krone (258A, 258B, 258C, 258D) in dem ersten und zweiten Paar distaler Kronen einen Teil einer vergrößerten Zelle (262A, 262B, 262C, 262D) bildet, und wobei weiter die Oberfläche der vergrößerten Zellen (262A, 262B, 262C, 262D) im entspannten Zustand größer ist als die Oberfläche der anderen Zellen (248) des Korbes (246), und wobei weiter die vergrößerten Zellen (262A, 262B, 262C, 262D) konfiguriert sind, einem Thrombus zu erlauben, dort hindurch und in das Korbinnere (292) zu gelangen.

6. System (200) nach Anspruch 1, wobei der distale Körper (216) im entspannten Zustand keine andere freie nach distal zeigende Krone aufweist, die etwa in demselben Abstand von der proximalen Nabe (228) angeordnet ist wie das erste Paar distaler Kronen (258A, 258B), und der distale Körper (216) keine andere freie nach distal zeigende Krone aufweist, die etwa in demselben Abstand von der proximalen Nabe (228) angeordnet ist wie das zweite Paar distaler Kronen (258C, 258D).

7. System (200) nach Anspruch 1, wobei die Korbstreifen (291) aus einem Memory-Metall bestehen.

8. System (200) nach Anspruch 1, wobei jede der distalen Kronen (258A, 258B) in dem ersten Paar distaler Kronen konfiguriert ist, mit jeder anderen in Kontakt zu treten, wenn eine äußere externe Druckkraft auf eine distale Krone (258A, 258B) des ersten Paares distaler Kronen ausgeübt wird, wenn sich der distale Körper (216) im entspannten Zustand befindet, und wobei weiter jede der distalen Kronen (258C, 258D) in dem zweiten Paar distaler Kronen konfiguriert ist, mit jeder anderen in Kontakt zu treten, wenn eine äußere externe Druckkraft auf eine distale Krone (258C, 258D) des zweiten Paares distaler Kronen ausgeübt wird, wenn sich der distale Körper (216) im entspannten Zustand befindet.

9. System (200) nach Anspruch 1, wobei die proximale Nabe (228) etwa in der Mitte der ersten Höhe (224) und ersten Breite im entspannten Zustand angeordnet ist.

10. System (200) nach Anspruch 1, wobei das proximale Ende (254) eines ersten proximalen Streifens (252) mindestens etwa 65 Grad relativ zu dem distalen Ende (256) des ersten proximalen Streifens (252) angeordnet ist, wobei das proximale Ende (254) eines zweiten proximalen Streifens (252) mindestens etwa 65 Grad relativ zu dem distalen Ende (256) des zweiten proximalen Streifens (252) angeordnet ist, und wobei sich weiter der erste und zweite proximale Streifen (252) angrenzend an und distal zu der proximalen Nabe (228) schneiden.

11. System (200) nach Anspruch 1, wobei der Korb (246) keine im Allgemeinen in die proximale Richtung zeigenden freien proximalen Kronen einschließt.

12. System (200) nach Anspruch 1, wobei die proximale Nabe (228) in der Form einer proximalen Röhre (228) ist, wobei der Korb (246), die proximale Röhre (228) und die proximalen Streifen (252) aus einem Memory-Metall bestehen, wobei die proximale Röhre (228) ein proximales Ende (230) und ein distales Ende (232) umfasst, und wobei weiter die proximalen Streifen (252) einstückig mit dem distalen Ende (232) der proximalen Röhre (228) sind.

13. System (200) nach Anspruch 1, wobei der Zugdraht (202) aus einem biokompatiblen Metallmaterial besteht.

14. System (200) nach Anspruch 1, wobei der Katheter (208) aus einem Polymermaterial besteht.

15. System (200) nach Anspruch 1, wobei der distale Körper (216) weiter einen Zuleitungsdraht (286) umfasst, der sich distal von der einzelnen distalen Nabe (236) erstreckt.

## Revendications

1. Système (200) pour retirer des objets (270A, 270B, 270C) d'une lumière intérieure (266) d'un animal, le système (200) comprenant :
un fil de traction (202) ayant une extrémité proximale (204) et une extrémité distale (206) ;
un corps distal (216) attaché au fil de traction (202), le corps distal (216) comprenant un intérieur (222), une extrémité proximale (218), une extrémité distale (220), une longueur de corps distal (226) s'étendant de l'extrémité proximale (218) à l'extrémité distale (220), un raccord proximal (228) formant l'extrémité proximale (218) du corps distal (216), une pluralité de bandes proximales (252), un panier (246) composé d'une pluralité de cellules (248) formées par une pluralité de bandes de panier (291), le panier (246) comprenant un intérieur de panier (292), chaque bande proximale (252) ayant une extrémité distale (256) attachée à une cellule (248) et une extrémité proximale (254), les extrémités proximales (254) des bandes proximales (252) convergeant au niveau du raccord proximal (228), le corps distal (216) ayant un état relâché dans lequel le corps distal (216) a une première hauteur (224) et une première largeur, et un état affaissé dans lequel le corps distal a une seconde hauteur et une seconde largeur, la seconde hauteur étant inférieure à la première hauteur (224), la seconde largeur étant inférieure à la première largeur ; et
un cathéter (208) ayant un intérieur (210), une extrémité proximale (212) menant à l'intérieur (210) et une extrémité distale (214) menant à l'intérieur (210), le cathéter (208) étant composé d'un matériau biocompatible et configuré pour envelopper le corps distal (216) quand le corps distal (216) est dans l'état affaissé,
dans lequel, dans l'état relâché, le panier (246) comprend une première paire de couronnes distales (258A, 258B) non attachées à une autre cellule (248) du panier (246) et pointant généralement dans la direction distale, les couronnes distales (258A, 258B) de la première paire de couronnes distales se situant approximativement à la même distance du raccord proximal (228) et entre 150 degrés et 180 degrés l'une par rapport à l'autre, et en outre dans lequel le panier (246) comprend en outre une seconde paire de couronnes distales (258C, 258D) non attachées à une autre cellule (248) du panier (246) et pointant généralement dans la direction distale, la seconde paire de couronnes distales (258C, 258D) se situant de manière distale par rapport à la première paire de couronnes distales (258A, 258B), chacune des couronnes distales (258C, 258D) de la seconde paire de couronnes distales se situant entre 60 degrés et 90 degrés par rapport à une couronne distale (258A, 258B) de la première paire de couronnes distales, les couronnes distales (258C, 258D) de la seconde paire de couronnes distales se situant approximativement à la même distance du raccord proximal (228) et en outre dans lequel les couronnes distales (258A, 258B, 258C, 258D) des première et seconde paires de couronnes distales comprennent un marqueur à rayons X (244), le marqueur à rayons X (244) étant plus visible sous rayons X par rapport aux bandes de panier (291) quand le corps distal (216) se situe dans un vaisseau sanguin crânien (266) à l'intérieur du corps d'un humain et le rayon X est pris depuis l'extérieur du corps de l'humain et en outre dans lequel chaque couronne distale (258A, 258B, 258C, 258D) des première et seconde paires de couronnes distales forme une partie d'une cellule (250A, 250B, 250C, 250D) qui comprend en outre une couronne proximale ne flottant pas librement (260) pointant généralement dans la direction proximale,
dans lequel au moins certaines des bandes de panier (291) se situent à une extrémité distale du panier (246), dans lequel chacune des bandes de panier (291) situées à l'extrémité distale du panier (246) a une extrémité distale, et
**caractérisé en ce que** le système comprend un raccord distal unique (236) comprenant une extrémité proximale (238), l'extrémité proximale (238) du raccord distal unique (236) formant l'extrémité distale du panier (246), dans lequel chacune des extrémités distales des bandes de panier (291) situées à l'extrémité distale du panier (246) converge au niveau du raccord distal unique (236) et en outre dans lequel le corps distal (216), dans l'état relâché, comprend une région conique dans laquelle la hauteur et la largeur de corps distal (224) diminuent quand les bandes de panier (291) situées à l'extrémité distale du panier (246) s'approchent du raccord distal unique (236),
dans lequel le panier (246) comprend une pluralité de cellules (248) distales de la seconde paire de couronnes distales (258C, 258D),
et en outre dans lequel chacune des première et seconde paires de couronnes distales (258A, 258B, 258C, 258D) est proximale au raccord distal unique (236).

2. Système (200) selon la revendication 1, dans lequel, les marqueurs à rayons X (244) sont composés d'un matériau différent du matériau formant les bandes de panier (291).

3. Système (200) selon la revendication 1, dans lequel, dans l'état relâché, l'intérieur de panier (292) est creux.

4. Système (200) selon la revendication 1, dans lequel, dans l'état relâché, le corps distal (216) n'a pas d'autre marqueur à rayons X qui se situe approximativement à la même distance du raccord proximal (228) que la première paire de marqueurs à rayons X (244) et le corps distal (216) n'a pas d'autre marqueur à rayons X qui se situe approximativement à la même distance du raccord proximal (228) que la seconde paire de marqueurs à rayons X (244).

5. Système (200) selon la revendication 1, dans lequel chaque couronne distale (258A, 258B, 258C, 258D) des première et seconde paires de couronnes distales forme une partie d'une cellule agrandie (262A, 262B, 262C, 262D) et en outre dans lequel la superficie des cellules agrandies (262A, 262B, 26C, 262D) dans l'état relâché est supérieure à la superficie des autres cellules agrandies (248) du panier (246) et en outre dans lequel les cellules agrandies (262A, 262B, 262C, 262D) sont configurées pour permettre à un thrombus de passer à travers celles-ci et dans l'intérieur de panier (292).

6. Système (200) selon la revendication 1, dans lequel, dans l'état relâché, le corps distal (216) n'a pas d'autre couronne pointant de manière distale libre qui se situe approximativement à la même distance du raccord proximal (228) que la première paire de couronnes distales (258A, 258B) et le corps distal (216) n'a pas d'autre couronne pointant de manière distale libre qui se situe approximativement à la même distance du raccord proximal (228) que la seconde paire de couronnes distales (258C, 258D).

7. Système (200) selon la revendication 1 dans lequel les bandes de panier (291) se composent d'un métal à mémoire.

8. Système (200) selon la revendication 1, dans lequel les couronnes distales (258A, 258B) de la première paire de couronnes distales sont configurées pour être en contact entre elles quand une force de compression externe, extérieure est exercée sur une couronne distale (258A, 258B) de la première paire de couronnes distales quand le corps distal (216) est dans l'état relâché, et en outre dans lequel les couronnes distales (258C, 258D) de la seconde paire de couronnes distales sont configurées pour être en contact entre elles quand une force de compression externe, extérieure est exercée sur une couronne distale (258C, 258D) de la seconde paire de couronnes distales quand le corps distal (216) est dans l'état relâché.

9. Système (200) selon la revendication 1 dans lequel le raccord proximal (228) se situe approximativement au centre de la première hauteur (224) et de la première largeur dans l'état relâché.

10. Système (200) selon la revendication 1, dans lequel l'extrémité proximale (254) d'une première bande proximale (252) se situe au moins à environ 65 degrés par rapport à l'extrémité distale (256) de la première bande proximale (252), dans lequel l'extrémité proximale (254) d'une seconde bande proximale (252) se situe à au moins environ 65 degrés par rapport à l'extrémité distale (256) de la seconde bande proximale (252), et en outre dans lequel les première et seconde bandes proximales (252) se croisent de manière adjacente et distale au raccord proximal (228).

11. Système (200) selon la revendication 1, dans lequel le panier (246) n'inclut aucune couronne proximale libre pointant généralement dans la direction proximale.

12. Système (200) selon la revendication 1, dans lequel le raccord proximal (228) se présente sous la forme d'un tube proximal (228), dans lequel le panier (246), le tube proximal (228) et les bandes proximales (252) se composent d'un métal à mémoire, dans lequel le tube proximal (228) comprend une extrémité proximale (230) et une extrémité distale (232), et en outre dans lequel les bandes proximales (252) font corps avec l'extrémité distale (232) du tube proximal (228).

13. Système (200) selon la revendication 1, dans lequel le fil de traction (202) se compose d'un matériau métallique biocompatible.

14. Système (200) selon la revendication 1, dans lequel le cathéter (208) se compose d'un matériau polymère.

15. Système (200) selon la revendication 1, dans lequel le corps distal (216) comprend en outre un fil conducteur (286) s'étendant de manière distale depuis le raccord distal unique (236).
